# EUROPEAN PATENT APPLICATION

(11) **EP 3 835 418 A1**
(43) Date of publication of application: **16.06.2021**
(21) Application number: 19846625.2
(22) Date of filing: 09.08.2019
(51) Int. Cl.: C12N 9/22, C12N 15/52, A61K 48/00, A61P 35/00, A61K 38/00

(54) **NOVEL CRISPR-ASSOCIATED PROTEIN AND USE THEREOF**

(30) Priority: 09.08.2018 KR 20180093336
(71) Applicant: G+Flas Life Sciences, Seoul 08790 (KR); Seoul National University R & DB Foundation, Seoul 08826 (KR)
(72) Inventor: CHOE, Sunghwa, Seoul 08790 (KR); KIM, Han Seong, Seoul 08826 (KR); KIM, Dong Wook, Seoul 08826 (KR); PARK, Jongjin, Seoul 08790 (KR); YOON, Jiyoung, Seoul 08790 (KR)
(74) Representative: Dehns
(86) International application number: PCT/KR2019/010110
(87) International publication number: WO 2020/032711

(57) **Abstract**

The present invention relates to a novel CRISPR-associated protein and a use thereof. A protein represented by an amino acid sequence of SEQ ID NO: 1 or SEQ ID NO: 3, according to the present invention, exhibits the activity of endonucleases, which recognize and cleave an intracellular nucleic acid sequence linked to a guide RNA. Therefore, a novel CRISPR-associated protein of the invention can be used as a different nuclease for genome editing, in a CRISPR-Cas system.

## Description

### Technical Field

The present invention relates to a novel CRISPR-associated protein and a use thereof.

### Background Art

Genome editing is a technique by which the genetic information of a living organism is freely edited. Advances in the field of life sciences and development in genome sequencing technology have made it possible to understand a wide range of genetic information. For example, understanding of genes for reproduction of animals and plants, diseases and growth, genetic mutations that cause various human genetic diseases, and production of biofuels has already been achieved; however, further technological advances must be made to directly utilize this understanding for the purpose of improving living organisms and treating human diseases.

Genome editing techniques can be used to change the genetic information of animals, including humans, plants, and microorganisms, and thus their application range can be dramatically expanded. Genetic scissors, which are molecular tools designed and made to precisely cut desired genetic information, play a key role in genome editing techniques. Similar to the next-generation sequencing techniques that took the field of gene sequencing to the next level, use of the gene scissors is becoming a key technique in increasing the speed and range of utilization of genetic information and creating new industrial fields.

The genetic scissors having been developed so far may be divided into three generations according to the order of their appearance. The first generation of genetic scissors is zinc finger nuclease (ZFN); the second generation of genetic scissors is transcription activator-like effector nuclease (TALEN); and the most recently studied, clustered regularly interspaced short palindromic repeat (CRISPR)/CRISPR-associated protein 9 (Cas9) is the third generation of genetic scissors.

The CRISPRs are loci containing multiple short direct repeats that are found in the genomes of approximately 40% of sequenced bacteria and 90% of sequenced archaea. The Cas9 protein forms an active endonuclease when complexed with two RNAs termed CRISPR RNA (crRNA) and trans-activating crRNA (tracrRNA), thereby slicing foreign genetic elements in invading phages or plasmids to protect the host cells. The crRNA is transcribed from the CRISPR element of the host genome that has previously been occupied by foreign invaders.

RNA-guided nucleases derived from this CRISPR-Cas system provide a tool capable of genome editing. In particular, studies have been actively conducted which are related to techniques capable of editing genomes of cells and organs using a single-guide RNA (sgRNA) and a Cas protein. Recently, Cpf1 protein (derived from Prevotella and Francisella 1) was reported as another nuclease protein in the CRISPR-Cas system (B. Zetsche, et al., 2015), which results in a wider range of options in genome editing.

### Disclosure of Invention

### Technical Problem

As a result of making continuous efforts to develop a protein that is more effective in genome editing than the known nucleases, the present inventors have found a novel CRISPR-associated protein that recognizes and cleaves a target nucleic acid sequence, and thus have completed the present invention.

Accordingly, an object of the present invention is to provide a novel CRISPR-associated protein that recognizes and cleaves a target nucleic acid sequence.

### Solution to Problem

To achieve the above-mentioned object, the present invention provides a Cas12a protein having the amino acid sequence of SEQ ID NO: 1.

In addition, the present invention provides a Cas12a protein having the amino acid sequence of SEQ ID NO: 1, of which lysine (Lys) at position 925 is substituted with another amino acid.

In addition, the present invention provides a Cas12a protein having the amino acid sequence of SEQ ID NO: 3.

In addition, the present invention provides a Cas12a protein having the amino acid sequence of SEQ ID NO: 3, of which lysine (Lys) at position 930 is substituted with another amino acid.

In addition, the present invention provides a Cas12a protein having the amino acid sequence of SEQ ID NO: 1, of which aspartic acid (Asp) at position 877 is substituted with another amino acid.

In addition, the present invention provides a Cas12a protein having the amino acid sequence of SEQ ID NO: 3, of which aspartic acid (Asp) at position 873 is substituted with another amino acid.

In addition, the present invention provides a pharmaceutical composition for treating cancer, comprising as active ingredients: mgCas12a; and crRNA that targets a nucleic acid sequence specifically present in cancer cells.

### Advantageous Effects of Invention

The protein represented by the amino acid sequence of SEQ ID NO: 1 or SEQ ID NO: 3, according to the present invention, has endonuclease activity that recognizes and cleaves an intracellular nucleic acid sequence bound to a guide RNA. Therefore, the novel CRISPR-associated protein of the present invention can be used as another nuclease, which performs genome editing, in the CRISPR-Cas system.

### Brief Description of Drawings

FIG. 1 illustrates a schematic diagram of a process of discovering Cas12a from metagenome.
FIG. 2A illustrates a phylogenetic tree of the discovered Cas12a.
FIG. 2B illustrates structures of novel Cas12a's and AsCas12a.
FIGS. 3 to 8 illustrate amino acid sequences of existing Cas12a's and the mgCas12a's of the present invention, which have been aligned using the ESPript program.
FIGS. 9A and 9B illustrate tables obtained by comparing and summarizing the sequence information of the Cas12a's and the mgCas12a's of the present invention.
FIGS. 10 to 12 illustrate results obtained by identifying activity, depending on pH, of the mgCas12a's according to the present invention. On the other hand, crRNA #1 in FIG. 10 has the nucleotide sequence of SEQ ID NO: 25, and crRNA #2 in FIG. 11 has the nucleotide sequence of SEQ ID NO: 26.
FIG. 13 illustrates a diagram in which a target nucleic acid sequence and positions where crRNAs bind are indicated.
FIG. 14 illustrates results obtained by identifying gene editing efficiency achieved by respective proteins (mock, mgCas12a-1, and mgCas12a-2) in a case where crRNA for each of the genes CCR5 and DNMT1 is used.
FIG. 15 illustrates results obtained by identifying gene editing efficiency achieved by respective proteins (FnCpf1, mgCas12a-1, and mgCas12a-2) in a case where two crRNAs for the respective genes FucT14-1 and FucT14-2 are used.
FIGS. 16A and 16B illustrates results obtained by identifying DNA cleavage activity of FnCas12a, WT mgCas12a-1, or WT mgCas12a-2 protein.
FIG. 17 illustrates results obtained by identifying non-specific DNase functions of existing Cas12a (AsCas12a, FnCas12a, or LbCas12a) and novel Cas12a (WT mgCas12a-1, d_mgCas12a-1, WTmgCas12a-2, or d_mgCas12a-2).
FIGS. 18A and 18B illustrate results obained by identifying whether the FnCas12a, WT mgCas12a-1, or WT mgCas12a-2 protein has a non-specific DNase function without crRNA.
FIG. 19 illustrates results obtained by identifying whether the mgCas12a can perform DNA cleavage using 5' handle of existing Cas12a.
FIGS. 20A and 20B illustrate DNA cleavage activity of the FnCas12a, mgCas12a-1, or mgCas12a-2 protein in divalent ions.

### Best Mode for Carrying out the Invention

In an aspect of the present invention, there is provided a novel Cas12a protein obtained from metagenome.

As used herein, the term "Cas12a" is a CRISPR-related protein and may also be referred to as Cpf1. In addition, Cpf1 is an effector protein found in type V CRISPR systems. Cas12a, which is a single effector protein, is similar to Cas9, which is an effector protein found in type II CRISPR systems, in that it combines with crRNA to cleave a target gene. However, the two differ in how they work. The Cas12a protein works with a single crRNA. Therefore, for the Cas12a protein, there is no need to simultaneously use crRNA and trans-activating crRNA (tracrRNA) or to create a single-guide RNA (sgRNA) by synthetic combination of tracrRNA and crRNA, as in Cas9.

In addition, unlike Cas9, the Cas12a system recognizes a PAM present at the 5' position of a target sequence. In addition, in the Cas12a system, a guide RNA that determines a target also has a shorter length than Cas9. In addition, Cas12a is advantageous in that it generates a 5' overhang (sticky end), rather than a blunt end, at a cleavage site in a target DNA, and thus enables more accurate and diverse gene editing.

Conventionally, the Cas12a proteins may be derived from the *Candidatus* genus, the *Lachnospira* genus, the *Butyrivibrio* genus, the *Peregrinibacteria* genus, the *Acidominococcus* genus, the *Porphyromonas* genus, the *Prevotella* genus, the *Francisella* genus, the *Candidatus Methanoplasma* genus, or the *Eubacterium* genus. Specifically, PbCas12a is a protein derived from Parcubacteria bacterium GWC2011_GWC2_44_17; PeCas12a is a protein derived from Peregrinibacteria Bacterium GW2011_GWA_33_10; AsCas12a is a protein derived from Acidaminococcus sp. BVBLG; PmCas12a is a protein derived from Porphyromonas macacae; LbCas12a is a protein derived from Lachnospiraceae bacterium ND2006; PcCas12a is a protein derived from Porphyromonas crevioricanis; PdCas12a is a protein derived from Prevotella disiens; and FnCas12a is a protein derived from Francisella novicida U112. However, each Cas12a protein may have different activity depending on the microorganism from which it is derived.

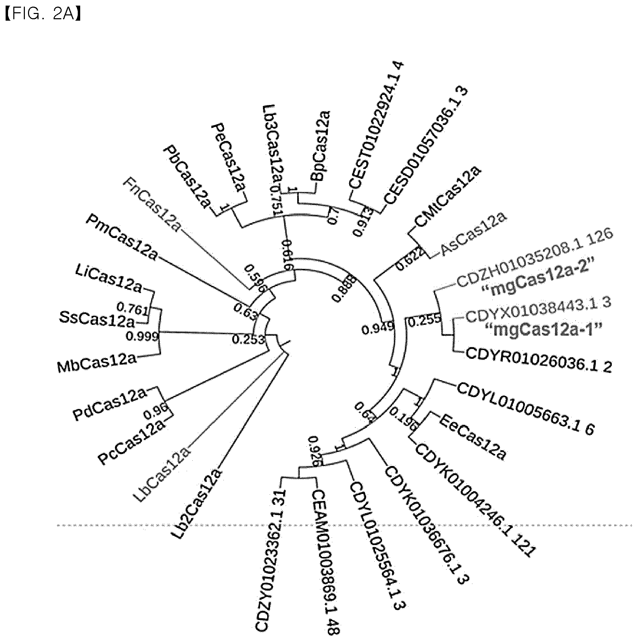

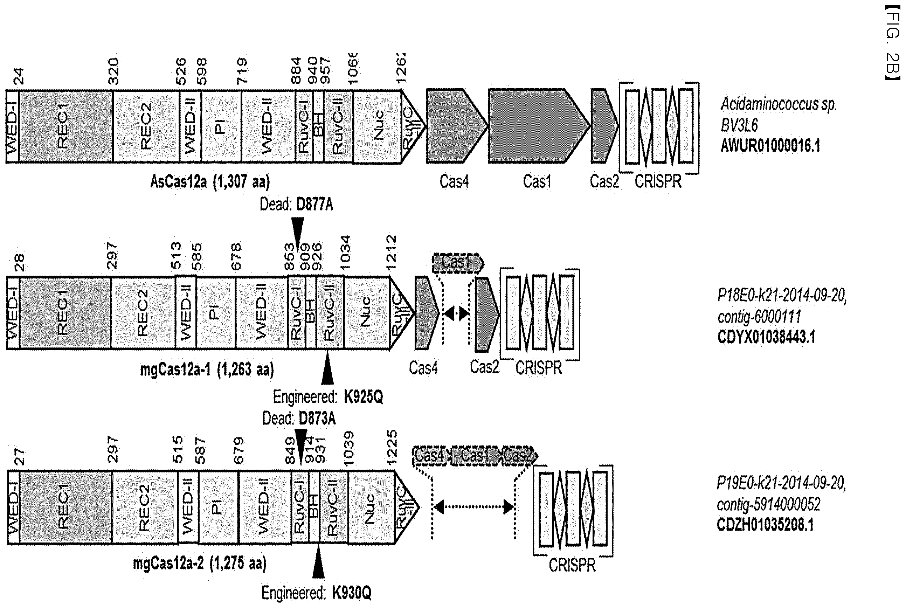

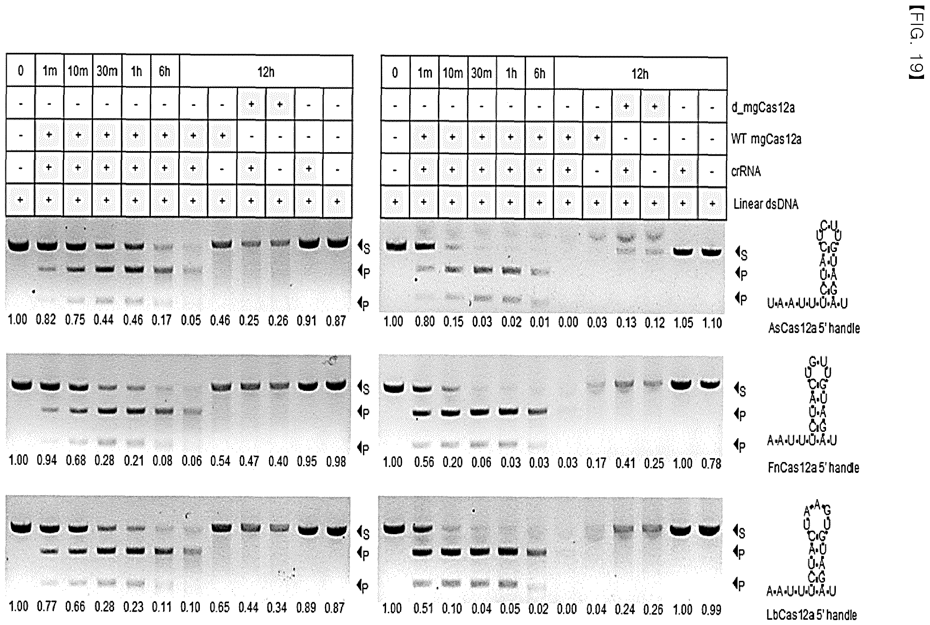

In the present invention, novel Cas12a's have been identified by analyzing genes in metagenomes. Hereinafter, metagenome-derived Cas12a may be referred to as mgCas 12a. Like AsCas12a, the mgCas 12a of the present invention includes WED, REC, PI, RuvC, BH, and NUC domains (FIG. 2). In addition, it was identified that similar to previously known Cas12a proteins, the mgCas12a protein of the present invention can perform gene cleavage with a gRNA including crRNA and 5'-handle. It was identified that the mgCas12a uses 5'-handle RNA having the same sequence as FnCas12a. Specifically, the 5'-handle RNA may have a sequence of AAUUUCUACUGUUGUAGAU (SEQ ID NO: 12). However, it was identified that the mgCas12a works even with a 5'-handle RNA in AsCas12a and LbCas12a (FIG. 19).

The mgCas12a may additionally include a tag for separation and purification. The tag may be bound to the N-terminus or C-terminus of the mgCas 12a. In addition, the tag may be bound simultaneously to the N-terminus and C-terminus of the mgCas 12a. One specific example of the tag may be a 6XHis tag.

As one specific example of the mgCas12a, there is provided a protein having the amino acid sequence of SEQ ID NO: 1. In addition, as long as activity of the mgCas12a is not changed, deletion or substitution of part of the amino acids may be made therein. Specifically, the mgCas12a may be a protein having the amino acid sequence of SEQ ID NO: 1, of which lysine (Lys) at position 925 is substituted with another amino acid. Here, the other amino acid may be any one selected from the group consisting of arginine (Arg), histidine (His), aspartic acid (Asp), glutamic acid (Glu), serine (Ser), threonine (Thr), asparagine (Asn), glutamine (Gln), tyrosine (Tyr), alanine (Ala), isoleucine (Ile), leucine (Leu), valine (Val), phenylalanine (Phe), methionine (Met), tryptophan (Trp), glycine (Gly), proline (Pro), and cysteine (Cys). Specifically, the protein may have the amino acid sequence of SEQ ID NO: 1, of which lysine at position 925 is substituted with glutamine. That is, the protein may have the amino acid sequence of SEQ ID NO: 5.

In addition, the gene that encodes the protein having the amino acid sequence of SEQ ID NO: 1 may be a polynucleotide having the nucleotide sequence of SEQ ID NO: 2. In addition, the mgCas12a having the amino acid sequence of SEQ ID NO: 1, according to the present invention, may have optimal activity at pH 7.0 to pH 7.9.

As another specific example of the mgCpf1, there is provided a protein having the amino acid sequence of SEQ ID NO: 3. In addition, as long as activity of the mgCpf1 is not changed, deletion or substitution of part of the amino acids may be made therein. Specifically, the mgCpf1 may be a protein having the amino acid sequence of SEQ ID NO: 3, of which lysine (Lys) at position 930 is substituted with another amino acid. Here, the other amino acid may be any one selected from the group consisting of arginine (Arg), histidine (His), aspartic acid (Asp), glutamic acid (Glu), serine (Ser), threonine (Thr), asparagine (Asn), glutamine (Gln), tyrosine (Tyr), alanine (Ala), isoleucine (Ile), leucine (Leu), valine (Val), phenylalanine (Phe), methionine (Met), tryptophan (Trp), glycine (Gly), proline (Pro), and cysteine (Cys). Specifically, the protein may have the amino acid sequence of SEQ ID NO: 3, of which lysine at position 930 is substituted with glutamine. That is, the protein may have the amino acid sequence of SEQ ID NO: 6.

The gene that encodes the protein having the amino acid sequence of SEQ ID NO: 3 may be a polynucleotide having the nucleotide sequence of SEQ ID NO: 4.

In addition, the mgCas12a having the amino acid sequence of SEQ ID NO: 3, according to the present invention, may have optimal activity at pH 7.0 to pH 7.9.

In another aspect of the present invention, there is provided an mgCas12a protein with decreased endonuclease activity. One specific example thereof may be mgCas12a having the amino acid sequence of SEQ ID NO: 1, of which aspartic acid (Asp) at position 877 is substituted with another amino acid. Here, the other amino acid may be any one selected from the group consisting of arginine (Arg), histidine (His), glutamic acid (Glu), serine (Ser), threonine (Thr), asparagine (Asn), glutamine (Gln), tyrosine (Tyr), alanine (Ala), lysine (Lys), isoleucine (Ile), leucine (Leu), valine (Val), phenylalanine (Phe), methionine (Met), tryptophan (Trp), glycine (Gly), proline (Pro), and cysteine (Cys). Specifically, the protein may be a protein obtained by substitution of the aspartic acid (Asp) with alanine (Ala).

Another specific example of the mgCas12a protein may be mgCas12a having the amino acid sequence of SEQ ID NO: 3, of which aspartic acid (Asp) at position 873 is substituted with another amino acid. Here, the other amino acid may be any one selected from the group consisting of arginine (Arg), histidine (His), glutamic acid (Glu), serine (Ser), threonine (Thr), asparagine (Asn), glutamine (Gln), tyrosine (Tyr), alanine (Ala), lysine (Lys), isoleucine (Ile), leucine (Leu), valine (Val), phenylalanine (Phe), methionine (Met), tryptophan (Trp), glycine (Gly), proline (Pro), and cysteine (Cys). Specifically, the protein may be a protein obtained by substitution of the aspartic acid (Asp) with alanine (Ala). Here, the mgCas12a with decreased endonuclease activity may be referred to as dead mgCas12a or d_mgCas12a. The d_mgCas12a may have the amino acid sequence of SEQ ID NO: 13 or SEQ ID NO: 14.

In addition, in yet another aspect of the present invention, there is provided a pharmaceutical composition for treating cancer, comprising as active ingredients: mgCas12a; and crRNA that targets a nucleic acid sequence specifically present in cancer cells. Here, the mgCas12a may have any one amino acid sequence selected from the group consisting of SEQ ID NO: 1, SEQ ID NO: 3, SEQ ID NO: 5, and SEQ ID NO: 6. As used herein, the term "nucleic acid sequence specifically present in cancer cells" refers to a nucleic acid sequence that is not present in normal cells and is present only in cancer cells. That is, this term refers to a sequence different from that in normal cells, and the two sequences may differ by at least one nucleic acid. In addition, such a difference may be caused by substitution or deletion of part of the gene. As one specific example, the nucleic acid sequence specifically present in cancer cells may be an SNP present in cancer cells. A target DNA having the above-mentioned sequence, which is present in cancer cells, and a guide RNA having a sequence complementary to the target DNA specifically bind to each other.

In particular, regarding the nucleic acid sequence specifically present in cancer cells, crRNAs can be created by finding specific SNPs, which exist only in cancer cells, through genome sequencing of various cancer tissues and using the same. This is done in a way of exhibiting cancer cell-specific toxicity, and thus makes it possible to develop patient-specific anti-cancer therapeutic drugs. In addition, the nucleic acid sequence specifically present in cancer cells may be a gene having high copy number variation (CNV) in cancer cells, unlike normal cells.

One specific example of the cancer may be any one selected from the group consisting of bladder cancer, bone cancer, blood cancer, breast cancer, melanoma, thyroid cancer, parathyroid cancer, bone marrow cancer, rectal cancer, throat cancer, laryngeal cancer, lung cancer, esophageal cancer, pancreatic cancer, gastric cancer, tongue cancer, skin cancer, brain tumor, uterine cancer, head or neck cancer, gallbladder cancer, oral cancer, colon cancer, perianal cancer, central nervous system tumor, liver cancer, and colorectal cancer. In particular, the cancer may be gastric cancer, colorectal cancer, liver cancer, lung cancer, and breast cancer, which are known as the five major cancers in Korea.

Here, crRNA that targets the nucleic acid sequence specifically present in cancer cells may include one or more gRNA sequences. For example, the crRNA may use a gRNA capable of simultaneously targeting exons 10 and 11 of BRCA1 present in ovarian cancer or breast cancer. In addition, the crRNA may use two or more gRNAs targeting exon 11 of BRCA1. As such, combination of gRNAs may be appropriately selected depending on purposes of cancer treatment and types of cancer. That is, different gRNAs may be selected and used.

### Mode for the Invention

Hereinafter, the present invention will be described in more detail by way of the following examples. However, the following examples are for illustrative purposes only, and the scope of the present invention is not limited thereto.

### Example 1. Discovery of metagenome-derived Cas12a protein

Metagenome nucleotide sequences were downloaded from the NCBI Genbank BLAST database and built into a local BLASTp database. In addition, 16 Cas12a's and various CRISPR-related protein (Cas1) amino acid sequences were downloaded from the Uniprot database. The MetaCRT program was used to find CRISPR repeats and spacer sequences in the metagenome. Then, only the metagenome sequences having the CRISPR sequence were extracted and their genes were predicted using the Prodigal program.

Among the predicted genes, those within a range that is 10 kb upstream or downstream of the CRISPR sequence were extracted, and the amino acid sequence of Cas12a was used to predict a Cas12a homolog among the genes in question. The Cas1 gene was used to predict whether there was a Cas1 homolog upstream or downstream of the Cas12a homolog; and Cas12a genes ranging from 800 aa to 1,500 aa, which had Cas1 around, were selected. For each of these genes, BLASTp was used in the NCBI Genbank non-redundant database to determine whether the gene was a gene that had already been reported or whether the gene was a gene having no association with CRISPR at all.

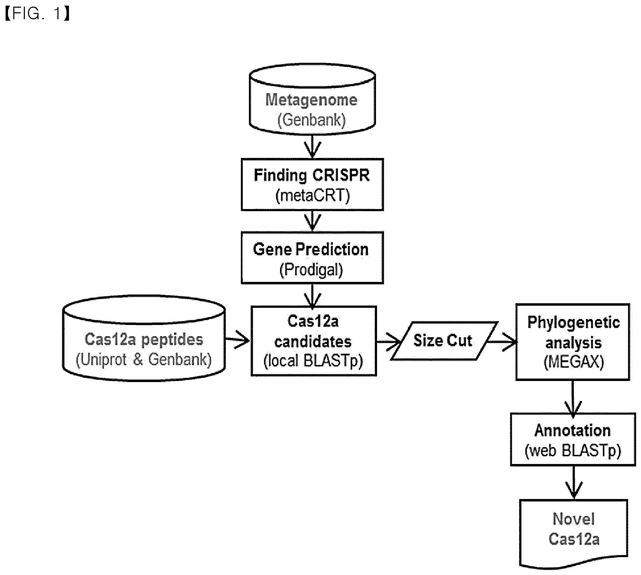

After removing fragmented Cas12a's that do not start with methionine (Met), these genes were aligned using a multiple alignment using fast fourier transform (MAFFT) program. Then, a phylogenetic tree was drawn with Neighbor-joining (100x bootstrap) using MEGA7. The gene that forms a monophyletic taxon with the previously known Cas12a gene was selected, and a phylogenetic tree thereof was drawn, together with the amino acid sequence of the existing Cas12a, using MEGA7, maximum-likelihood, and 1000x bootstrap, to examine their evolutionary relationship. Here, the process of discovering Cas12a from the metagenome is schematically illustrated in FIG. 1. In addition, the phylogenetic tree of the Cas12a is illustrated in FIG. 2A. Here, a novel protein having the amino acid sequence of SEQ ID NO: 1 was named WT mgCas12a-1. In addition, a novel protein having the amino acid sequence of SEQ ID NO: 3 was named WT mgCas12a-2. In addition, the structures of AsCas12a, mgCas12a-1, and mgCas12a-2 are illustrated in FIG. 2B.

### Example 2. Production of variants of mgCas12a

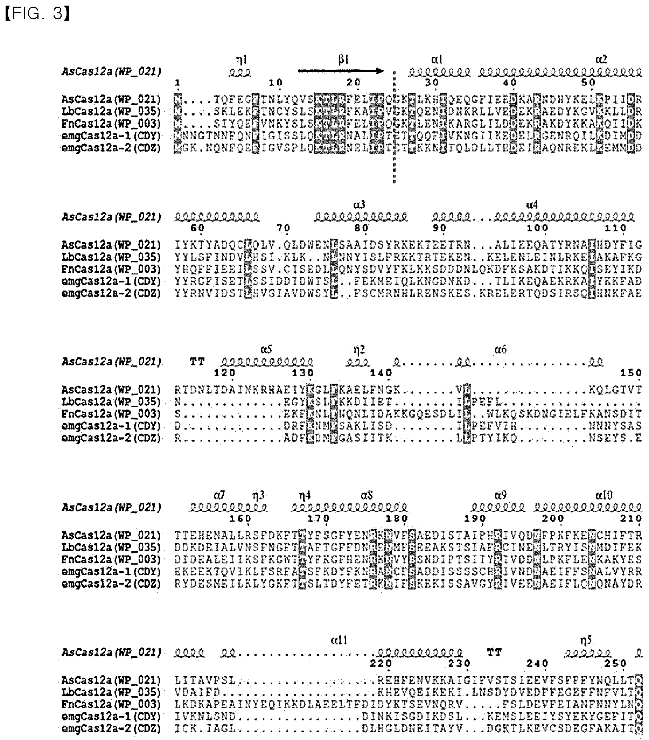

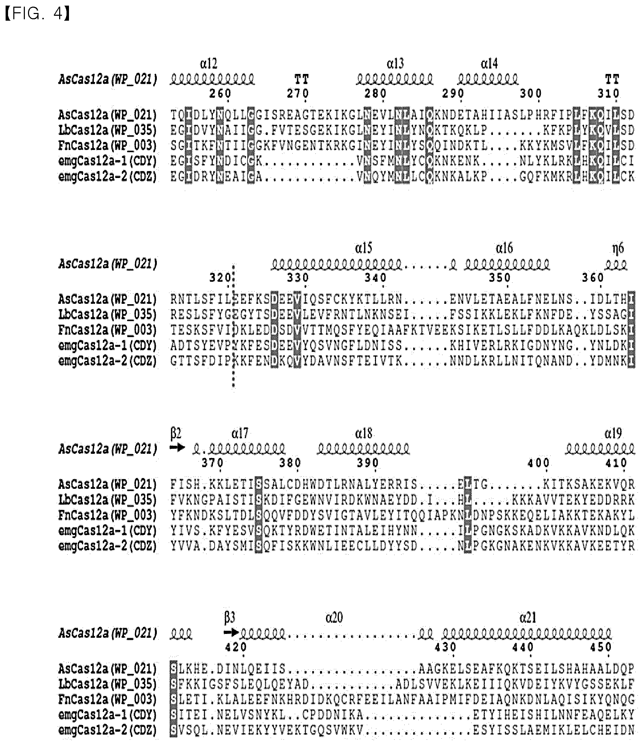

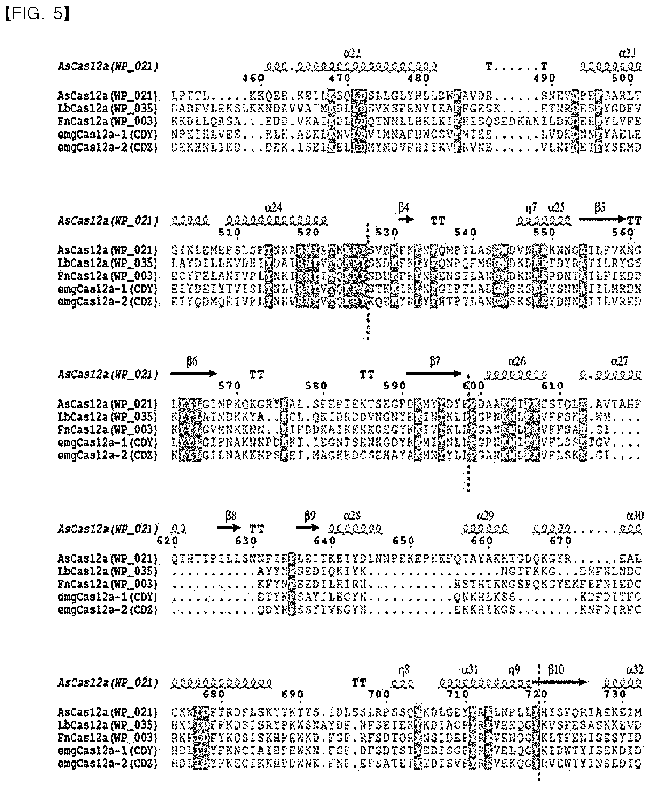

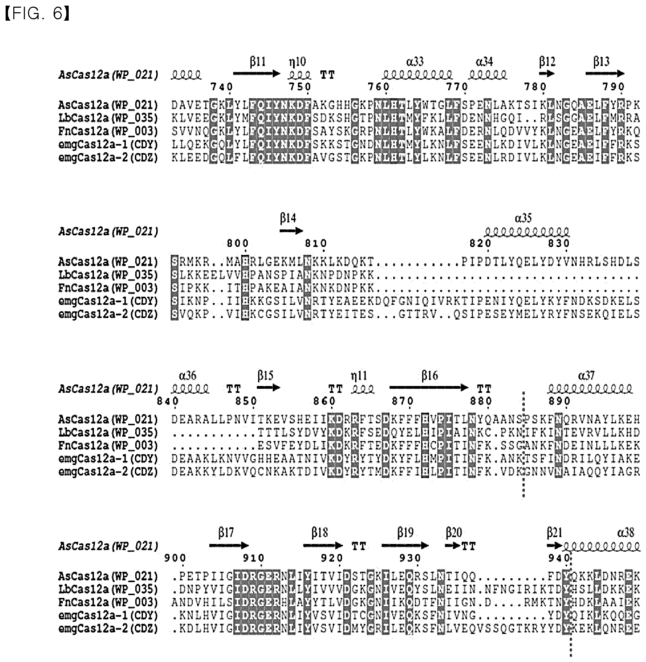

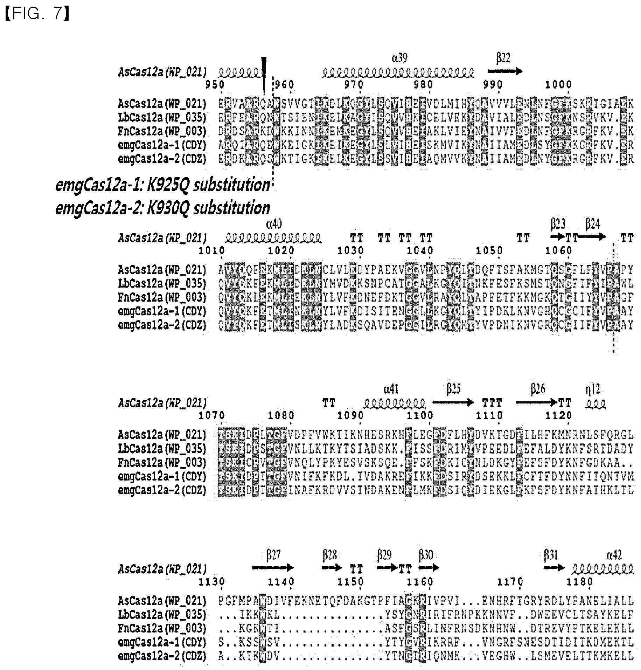

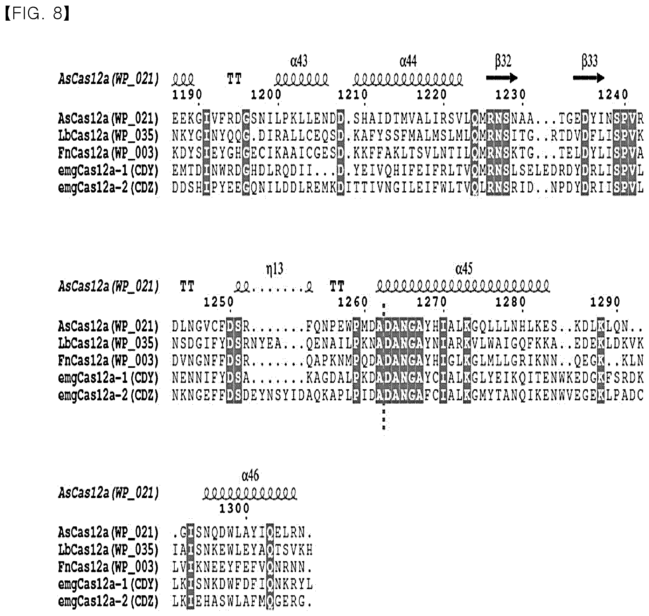

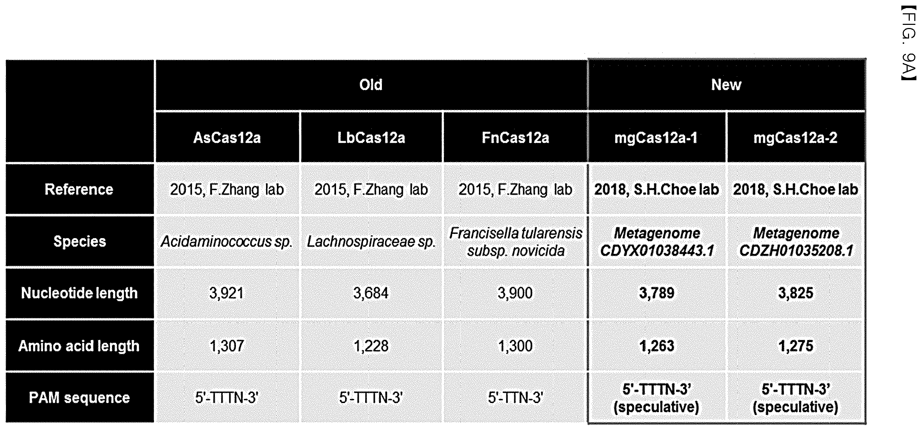

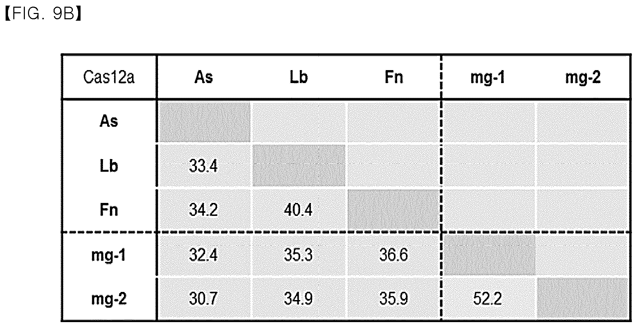

Cas12a candidates were aligned based on the structures of AsCas12a and LbCas12a using the ESPript program. For the WT mgCas12a-1 and WT mgCas12a-2, substitution of part of the amino acids was made to increase their endonuclease activity. The WT mgCas12a-1, in which the 925^{th} amino acid Lys(K) was substituted with Glu(Q), was named mgCas12a-1. In addition, the WT mgCas12a-2, in which the 930^{th} amino acid Lys(K) was substituted with Glu(Q), was named mgCas12a-2. The resulting variants were subjected to codon optimization in consideration of codon usages of humans, Arabidopsis, and E. coli, and then a request for gene synthesis thereof was made to Bionics. Here, the nucleotide sequences of the human codon-optimized mgCas12a-1 and mgCas12a-2 are shown in SEQ ID NO: 7 and SEQ ID NO: 8, respectively. In addition, the amino acid sequences of the existing Cas12a's (AsCas12a (SEQ ID NO: 9), LbCas12a (SEQ ID NO: 10), and FnCas12a (SEQ ID NO: 11)) and the Cas12a candidates (mgCas12a-1 and mgCas12a-2), which were aligned using the ESPript program, are illustrated in FIGS. 3 to 8; and the results obtained by comparing and summarizing their sequence information are illustrated in FIGS. 9A and 9B.

Then, each of the WT mgCas12a-1, WT mgCas12a-2, mgCas12a-1, and mgCas12a-2 genes, which had been cloned into pUC57 vector, was again inserted into pET28a-KanR-6xHis-BPNLS vector, and then cloning was performed. The cloned vector was transformed into the E. coli strains DH5a and Rosetta, respectively. A 5'-handle sequence of crRNA was extracted from the metagenome CRISPR repeat sequence. The extracted RNA was synthesized into a DNA oligo. Transcription of the DNA oligomer was performed using the MEGAshortscript T7 RNA transcriptase kit, and a concentration of the transcribed 5'-handle was checked by FLUOstar Omega.

### Example 3. Protein expression and purification

5 ml of the E. coli Rosetta (DE3), which was cultured overnight, was inoculated into 500 ml of liquid TB medium supplemented with 100 mg/ml of kanamycin antibiotic. The medium was cultured in an incubator at 37°C until the OD600 reached 0.6. For protein expression, treatment with 0.4 uM of isopropyl β-D-1-thiogalactopyranoside (IPTG) was performed, and then further culture was performed at 22°C for 16 to 18 hours. After centrifugation, the obtained cells were mixed with 10 ml of lysis buffer (20 mM HEPES pH 7.5, 100 mM KCl, 20 mM imidazole, 10% glycerol, and EDTA-free protease inhibitor cocktail), and then subjected to ultrasonication for cell disruption. The disruption was centrifuged three times at 6,000 rpm for 20 minutes each, and then filtered through a 0.22 micron filter.

Thereafter, washing and elution were performed using a nickel column (HisTrap FF, 5 ml) and 300 mM imidazole buffer, and the proteins were purified by affinity chromatography. The protein sizes were checked by SDS-PAGE electrophoresis, and dialysis was performed overnight against dialysis buffer (20 mM HEPES pH 7.5, 100 mM KCl, 1 mM DTT, 10% glycerol). Then, the proteins were selectively subjected to filtration and concentration (Amicon Ultra Centrifugal Filter 100,000 MWCO) depending on their size. For the proteins, Bradford quantitative method was used to measure their concentration. Then, the proteins were stored at - 80°C and used.

### Example 4. Identification of pH range suitable for mgCas12a through cleavage analysis

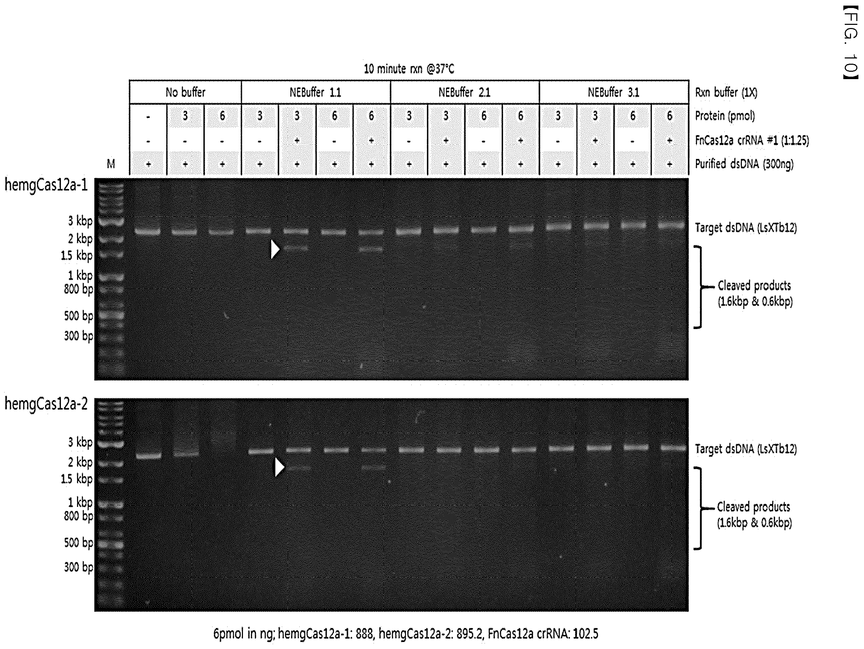

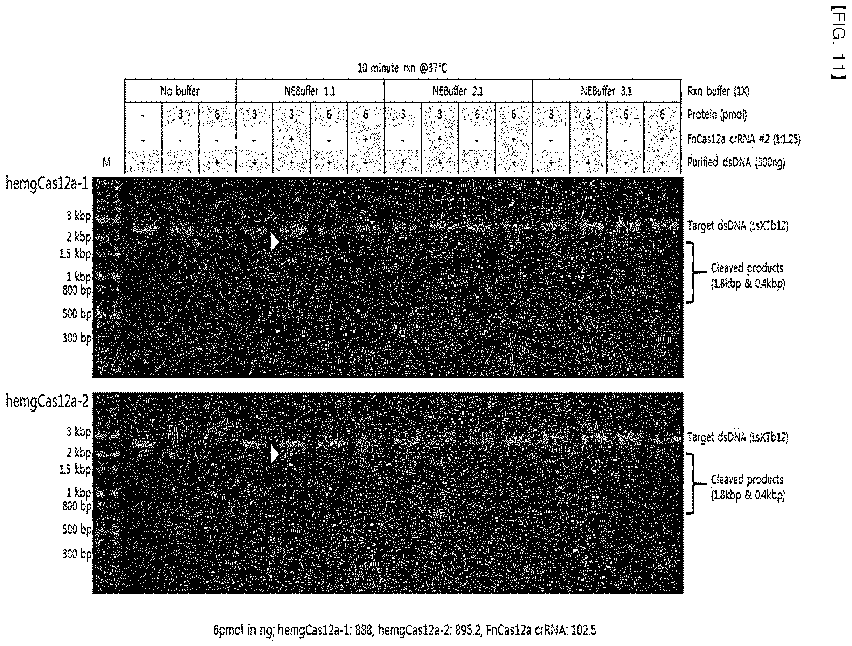

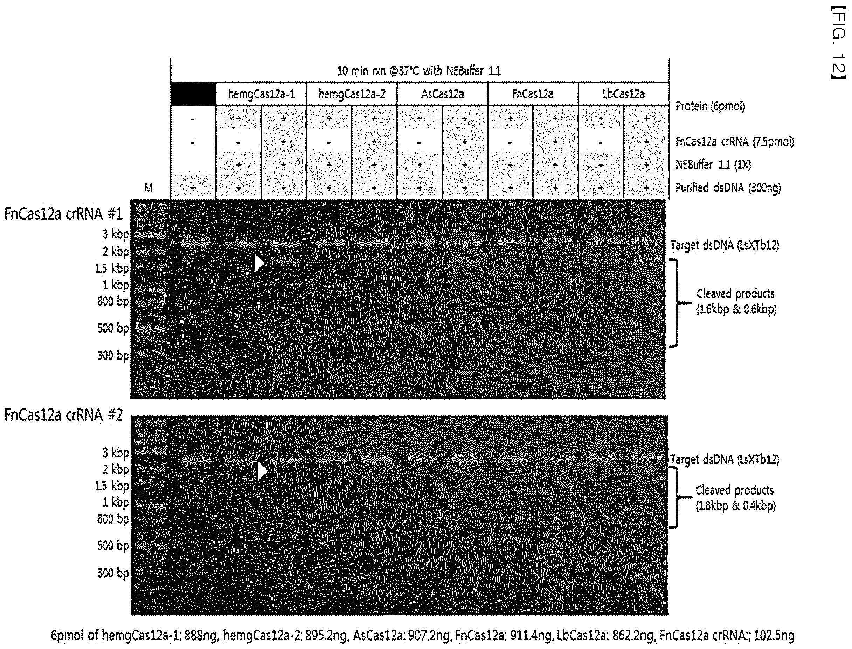

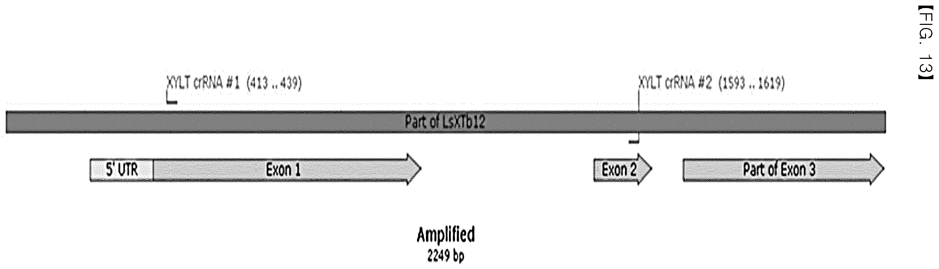

Xylosyltransferase of lettuce (Lactuca sativa) was amplified by PCR to predict a protospacer adjacent motif (PAM), and a guide RNA (gRNA) therefor was designed. For ribonucleoprotein (RNP) complexes for mgCas12a-1 and mgCas12a-2, each mgCas 12a protein was mixed with the gRNA at a molecular ratio of 1:1.25 at room temperature for 20 minutes, to produce each RNP complex. The purified xylosyltransferase PCR product was subjected to treatment with the RNPs at various concentrations. Then, concentration adjustment was conducted with NEBuffer 1.1 (IX Buffer Components, 10 mM Bis-Tris-Propane-HCl, 10 mM MgCl₂ and 100 µg/ml BSA), NEBuffer 2.1 (IX Buffer Components, 50 mM NaCl, 10 mM Tris-HCl, 10 mM MgCl₂ and 100 µg/ml BSA), and NEBuffer 3.1 (IX Buffer Components, 100 mM NaCl, 50 mM Tris-HCl, 10 mM MgCl₂ and 100 µg/ml BSA), and an in vitro cleavage analysis was performed at 37°C. Here, the NEBuffer 1.1, the NEBuffer 2.1, and the NEBuffer 3.1 had pH 7.0, pH 7.9, and pH 7.9 values, respectively, at 25°C. After each reaction was completed, the reaction was stopped by incubation at 65°C for 10 minutes, and the completed reaction was checked by 1.5% agarose gel electrophoresis. The results are illustrated in FIGS. 10 to 12. In FIGS. 10 to 12, the mgCas12a-1 and the mgCas12a-2 are designated by hemgCas12a-1 and hemgCas12a-2, respectively. In addition, the target nucleic acid sequence, which is in the xylosyltransferase, and the positions where the crRNAs bind were indicated in a diagram, and this diagram is illustrated in FIG. 13.

As illustrated in FIGS. 10 to 12, in a case where the mgCas12a-1 and crRNA complex was treated with the NEBuffer 1.1, the target dsDNA was cleaved. In addition, in a case where the mgCas12a-2 and crRNA complex was treated with the NEBuffer 1.1, the target dsDNA was cleaved. From these results, it was found that the mgCas12a-1 and mgCas12a-2 were active at pH 7.0.

### Example 5. Analysis of gene editing efficiency of mgCas12a in animal cells

### Example 5.1. Production of RNP including mgCas12a-1 or mgCas12a-2 for gene editing of CCR5 and DNMT1

HEK 293T cells were cultured in a 5% CO₂ incubator at 37°C in DMEM medium supplemented with 10% fetal bovine serum (FBS) and penicillin-streptomycin (P/S). Each 100 pmole of the mgCas12a-1 protein and the mgCas12a-2 protein, and 200 pmole of each of CCR5-targeting crRNA and DNMT1-targeting crRNA were incubated at room temperature for 20 minutes, to prepare each RNP. Here, the crRNA sequences for CCR5 and DNMT1 were synthesized by Integrated DNA Technologies (IDT), and are shown in Table 1 below.

**[Table 1]**

| **Genes** | **crRNA sequence (5'-3')** |
|---|---|
| CCR5 | CACCGAAUUUCUACUGUUGUAGAUGGAGUGAAGGAGAGUUGUCAAUUUUU UG (SEQ ID NO: 12) |
| DNMT 1 | GGUCAAUUUCUACUGUUGUAGAUGCUCAGCAGGCACCUGCCUCUUUU (SEQ ID NO: 13) |

The cultured HEK293T cells at 2 × 10⁵ were mixed with 20 µl of nucleofection reagent, and then mixed with 10 µl of RNP complex. Subsequently, 4D-Nucleofector device (Lonza) was used for transfection. 48 and 72 hours after transfection, genomic DNA was extracted from the cells using PureLink™ Genomic DNA Mini Kit (Invitrogen).

### Example 5.2. Sequencing analysis for target site

The genomic DNA extracted in Example 5.1 was amplified using adapter primers for CCR5 or DNMT 1 shown in Table 2 below.

**[Table 2]**

| **Genes** | **Adapter primer sequence (5'-3')** |
|---|---|
| CCR5 | TCGTCGGCAGCGTCAGATGTGTATAAGAGACAGGGTATTTCTGTTCAGATCAC (SEQ ID NO: 15) |
| | GTCTCGTGGGCTCGGAGATGTGTATAAGAGACAGGCCCATCAATTATAGAAAGCC (SEQ ID NO: 16) |
| DNMT1 | TCGTCGGCAGCGTCAGATGTGTATAAGAGACAGCTGCACACAGCAGGCCTTTG (SEQ ID NO: 17) |
| | GTCTCGTGGGCTCGGAGATGTGTATAAGAGACAGCCCAATAAGTGGCAGAGTGC |
| | (SEQ ID NO: 18) |

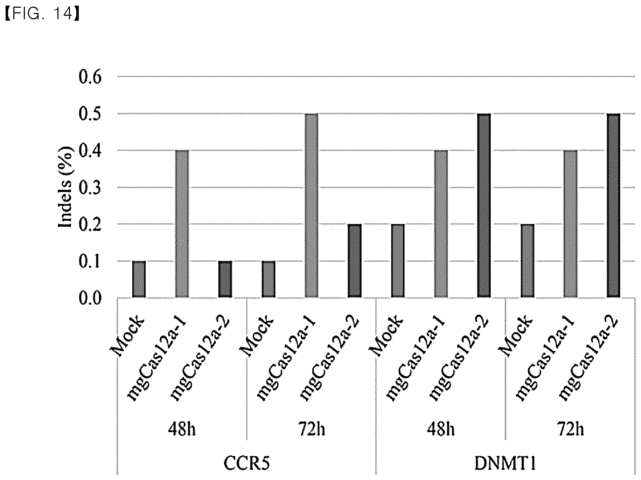

Subsequently, purification and sequencing library preparation were performed according to the protocol of Illumina, and then a deep-sequencing analysis was performed on the target site using MiniSeq equipment. The gene editing efficiency achieved by the mgCas12a-1 and mgCas12a-2 proteins is illustrated in FIG. 14, and the sequencing analysis results for the target site are shown in Table 3 below. As illustrated in FIG. 14, the mgCas12a-1 and mgCas12a-2 proteins exhibited higher gene editing efficiency than that of the mock protein.

**[Table 3]**

| **Samples** | **Genes** | **Time** | **Name** | **Total Sequences** | **With both indicator sequences** | **More than minimum frequency** | **Insertions** | **Deletions** | **Indel frequency** | **Indel frequency (%)** |
|---|---|---|---|---|---|---|---|---|---|---|
| 1 | CCR5 | 48h | Mock | 137952 | 137475 | 137196 | 0 | 187 | 187(0.1%) | 0.1 |
| 2 | | | mgCas12a-1 | 119684 | 119250 | 118952 | 36 | 418 | 454 (0.4%) | 0.4 |
| 3 | | | mgCas12a-2 | 112387 | 112077 | 111826 | 8 | 150 | 158(0.1%) | 0.1 |
| 4 | | 72h | Mock | 139323 | 138942 | 138647 | 8 | 179 | 187(0.1%) | 0.1 |
| 5 | | | mgCas12a-1 | 156705 | 156159 | 155857 | 39 | 738 | 777 (0.5%) | 0.5 |
| 6 | | | mgCas 12a-2 | 158717 | 158392 | 158048 | 5 | 237 | 242 (0.2%) | 0.2 |
| 7 | DNMT1 | 48h | Mock | 141182 | 136856 | 136469 | 19 | 316 | 335 (0.2%) | 0.2 |
| 8 | | | mgCas12a-1 | 122368 | 120871 | 120476 | 70 | 424 | 494 (0.4%) | 0.4 |
| 9 | | | mgCas12a-2 | 121928 | 120592 | 120218 | 46 | 509 | 555(0.5%) | 0.5 |
| 10 | | 72h | Mock | 98480 | 96480 | 96170 | 0 | 192 | 192 (0.2%) | 0.2 |
| 11 | | | mgCas12a-1 | 126317 | 123792 | 123370 | 2 | 511 | 513 (0.4%) | 0.4 |
| 12 | | | mgCas 12a-2 | 47398 | 47000 | 46738 | 12 | 199 | 211 (0.5%) | 0.5 |

### Example 6. Analysis of gene editing efficiency of mgCas12a in plant cells

### Example 6.1. Plant protoplast isolation

Tobacco seeds were sterilized by treatment with 50% Clorox for 1 minute. The sterilized seeds were placed on a medium for seed germination and cultured for a week. Then, the seeds were transferred to a magenta box used for culture, and grown for 3 weeks. The light culture condition used was 16 hours of light and 8 hours of darkness, and the seeds were grown at a temperature of 25°C to 28°C. For the plant, leaves grown for 4 to 6 weeks were used. The leaf was placed on a glass plate, and the leaf apex and petiole were cut therefrom so that only an inner part of the leaf was used. Here, the leaf was cut into pieces of 0.5 mm or smaller. The cut leaf pieces were placed in 10 mL of Enzyme solution and incubated on an orbital shaker (50 rpm) at room temperature for 3 to 4 hours in the dark.

After incubation, 10 mL of W5 solution was added and carefully mixed. A cell strainer (70 µm) was used to filter the protoplasts present in the Enzyme solution. The filtered protoplasts were centrifuged at 100 xg for 6 minutes. The supernatant was discarded, and the protoplast pellet was carefully suspended by addition of MMG solution. Then, the suspension was placed on ice for 10 to 30 minutes. For a part of the suspension, the number of protoplasts was counted using a Hem cytometer, which is a counter plate, and a microscope. Subsequently, MMG solution was further added for dilution so that the protoplast concentration reached 2×10⁶ cells/mL. The composition for each of the enzyme solution, MMG solution, and PEG solution is shown in Table 4 below.

**[Table 4]**

| **Enzyme solution** | **20 mL** |
|---|---|
| 1.0% Cellulase R10 | 200 mg |
| 0.5% Macerozyme R10 | 100 mg |
| 0.4 M Mannitol | 10 mL (0.8 M mannitol stock solution) |
| 20 mM MES, pH 5.7 | 4 mL (100 mM MES stock solution, pH 5.7) |
| 20 mM KCl | 200 µL (2 M KCl stock solution) |
| Combination of the above-mentioned reagents is performed, incubation is performed for 10 minutes at 60°C, and then combination with the following reagents is performed. | |
| 10 mM CaCl₂·2H₂O | 200 µL (1 M CaCl₂·2H₂O stock solution) |
| 0.1 % BSA | 200 µL (10% BSA stock solution) |

| **MMG solution** | **10 mL** |
|---|---|
| 0.4 M Mannitol | 5 mL (0.8 M mannitol stock solution) |
| 4 mM MES, pH 5.7 | 400 µL (0.1 M MES stock solution, pH5.7) |
| 15 mM MgCl₂ | 150 µL (1 M MgCl₂ stock solution) |
| Nuclease-free water | 4.45 mL |

| **PEG solution** | **5 mL** |
|---|---|
| 0.2 M Mannitol | 1.25 mL (0.8 M mannitol stock solution) |
| 40%W/V PEG-4000 | 2 g (polyethylene glycol 4000) |
| 100 mM CaCl₂·2H₂O | 500 µL (1 M CaCl₂·2H₂O stock solution) |
| Nuclease-free water | 1.5 mL |

| **W5 solution** | **50 mL** |
|---|---|
| 154 mM NaCl | 3.85 mL (2 M NaCl stock solution) |
| 125 mM CaCl₂·2H₂O | 6.25 mL (1 M CaCl₂·2H₂O stock solution) |
| 5 mM KCI | 125 µL (2 M KCl stock solution) |
| 2 mM MES, pH 5.7 | 500 µL (0.1 M MES stock solution) |
| Nuclease-free water | 39.275 mL |

### Example 6.2. Sequencing analysis for target site and identification of editing efficiency therefor

crRNA, mgCas 12a protein, and NEB buffer 1.1 were added to a 2 mL e-tube to a final volume of 20 µL, and then reaction was allowed to proceed at room temperature for 10 minutes. 200 µL (5 × 10⁵ cells) of the protoplast obtained in Example 6.1, and the reacted crRNA and mgCas12 protein (volume 20 µL) were added to an e-tube (2 mL), mixed well, and then cultured for 10 minutes in a clean bench. Subsequently, 220 µL of PEG solution, which was the same volume as the incubated volume, was added thereto and carefully mixed. The mixture was cultured at room temperature for 15 minutes. Then, 840 µL of W5 solution was added thereto and mixed well. After centrifugation at 100 xg for 2 minutes, the supernatant was discarded. Then, culture was performed in W5 solution for two days. Then, the cells were harvested and DNA was extracted therefrom.

Using the extracted DNA, the target portion was subjected to PCR, and then the target gene editing efficiency was identified by next-generation sequencing (NGS). The results are shown in Table 5 below. As shown in Table 5, the gene editing efficiency achieved by the mgCas12a-1 protein was 1.8-fold higher than that of FnCpf1.

**[Table 5]**

| Target gene | crRNA | Nuclease | Total Sequences | With both indicator sequences | More than minimum frequency | Insertions | Deletions | Indel frequency |
|---|---|---|---|---|---|---|---|---|
| *FucT14-1* | 2 | none | 161551 | 161421 | 160896 | 4 | 180 | 184 (0.1%) |
| | | mgCas12a-1 | 124361 | 124255 | 123844 | 3 | 168 | 171 (0.1%) |
| | | mgCas12a-2 | 99154 | 99053 | 98734 | 0 | 131 | 131 (0.1%) |
| | | FnCpfl | 50060 | 50022 | 49808 | 0 | 63 | 63 (0.1%) |
| | 4 | none | 161551 | 161411 | 160899 | 4 | 178 | 182 (0.1%) |
| | | mgCas12a-1 | 106782 | 106706 | 106330 | 0 | 1877 | 1877 (1.8%) |
| | | mgCas12a-2 | 126665 | 126544 | 126057 | 79 | 885 | 964 (0.8%) |
| | | FnCpfl | 64554 | 64501 | 64272 | 15 | 470 | 485 (0.8%) |
| *FucT14-2* | 2 | none | 49459 | 49422 | 49192 | 2 | 49 | 51 (0.1%) |
| | | mgCas12a-1 | 81191 | 81101 | 80738 | 0 | 90 | 90 (0.1%) |
| | | mgCas12a-2 | 83694 | 83614 | 83286 | 0 | 99 | 99(0.1%) |
| | | FnCpfl | 108803 | 108682 | 108260 | 0 | 112 | 112 (0.1%) |
| | 4 | none | 49459 | 49427 | 49199 | 2 | 49 | 51 (0.1%) |
| | | mgCas12a-1 | 54918 | 54854 | 54532 | 6 | 689 | 695 (1.3%) |
| | | mgCas12a-2 | 127825 | 127691 | 127213 | 2 | 143 | 145 (0.1%) |
| | | FnCpfl | 64265 | 64168 | 63882 | 0 | 162 | 162 (0.3%) |

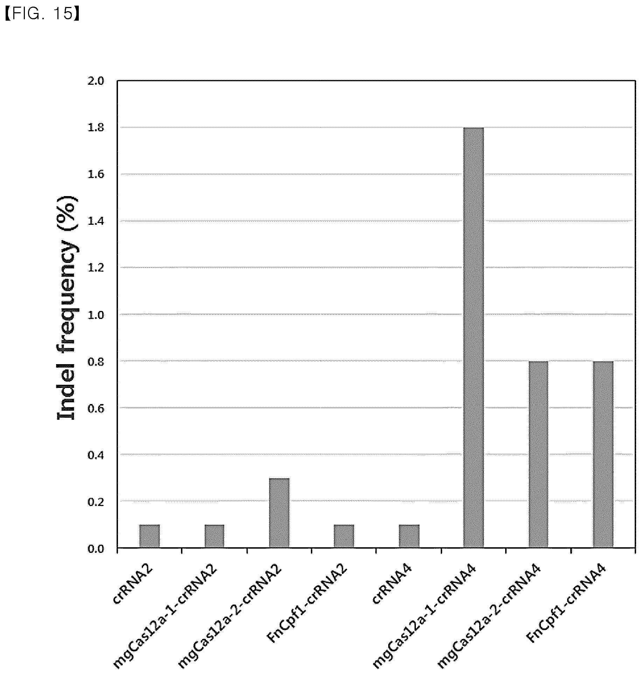

In addition, the gene editing efficiency achieved by using two crRNAs for the tobacco FucT14 genes was identified for each protein. The results are illustrated in FIG. 15. As illustrated in FIG. 15, the gene editing efficiency achieved by the mgCas12a-1 protein was 2-fold higher than that of FnCpf1. Here, the crRNAs and primer sequences for the target genes *NbFucT14*_*1* and *NbFucT14*_*2* are shown in Tables 6 and 7 below.

**[Table 6]**

| Target Gene | crRNA (primer name) | crRNA sequence (PAM site) |
|---|---|---|
| *NbFucT14*_ *1 NbFucT14*_ *2* | NbFTa14_1/2-2 | TTTGGATAATTTGTACTCTTGTCGATGT (SEQ ID NO: 19) |
| | NbFTa14_1/2-4 | TTTAGTCCACAAACAGCTAAGCCCACAT (SEQ ID NO: 20) |

**[Table 7]**

| Target gene | Primer name | Sequence | Size (bp) |
|---|---|---|---|
| *NbFucT14*_ *1* | NGS NbFTa14_1_F | TGAGCTGAAGATGGATTATG (SEQ ID NO: 21) | 216 |
| | NGS NbFTa14_1_R | TCATGCTTAAGATAAAAGAG (SEQ ID NO: 22) | |
| *NbFucT14*_ *2* | NGS NbFTa14_2_F | TCATGAGCTTAAGATGGATC (SEQ ID NO: 23) | 217 |
| | NGS NbFTa14_2_R | GTTTAAGCTAAAAGAACTAC (SEQ ID NO: 24) | |

### Example 7. Comparison of gene editing efficiency between FnCas12a and mgCas12a

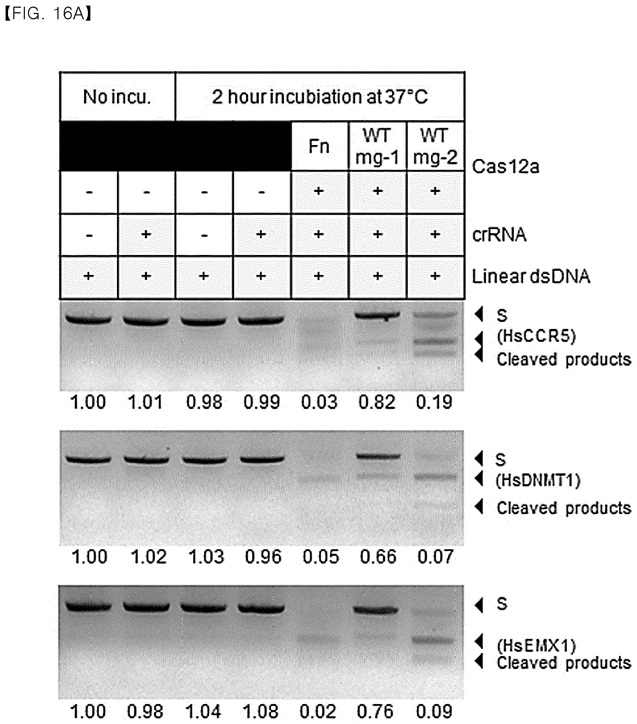

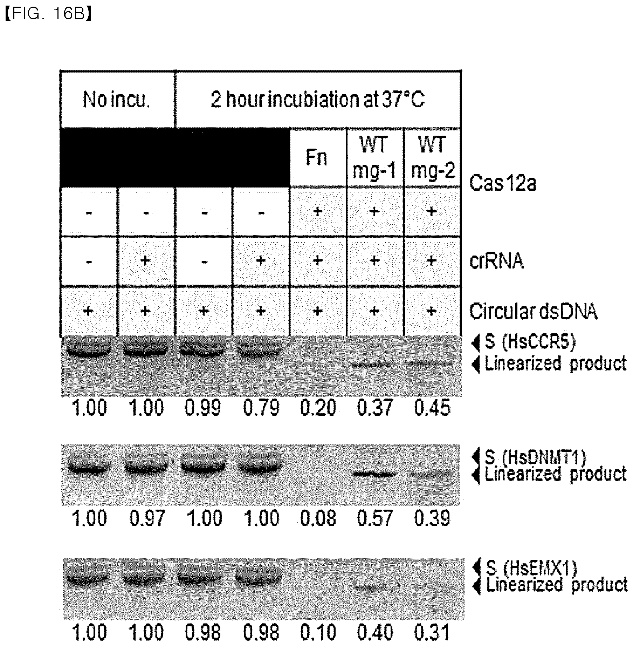

To form each ribonucleoprotein (RNP) complex consisting of FnCas12a, WT mgCas12a-1 or WT mgCas12a-2 protein, and crRNA, 6 pmol of FnCas12a, WT mgCas12a-1, or WT mgCas12a-2 protein, and 7.5 pmol of crRNA were mixed with NEB1.1 buffer and IX distilled water at room temperature for 30 minutes. To identify dsDNA cleavage activity using the crRNA-dependent Cas12a (FnCas12a, WT mgCas12a-1, or WT mgCas12a-2), 0.3 pmol of target dsDNA (linear or circular) was added thereto, and then reaction was allowed to proceed at 37°C for 2 hours. Here, HsCCR5, HsDNMT1, and HsEMX1 were used as DNA. In addition, the linear DNAs (SEQ ID NO: 27 to SEQ ID NO: 29) used in the experiment were PCR purified products, and the circular DNAs (SEQ ID NO: 30 to SEQ ID NO: 32) were purified plasmids. SDS and EDTA (gel loading dye, NEB) were added thereto, and then the mixture was stored at -20°C for 10 minutes to stop the reaction. Each DNA was loaded on a 1% agarose gel, and then subjected to electrophoresis to check the DNA cleavage activity caused by the FnCas12a, WT mgCas12a-1, or WT mgCas12a-2. The results are illustrated in FIGS. 16A (linear DNA) and 16B (circular DNA). In FIGS. 16A and 16B, S denotes a substrate, and each number indicated at the bottom of the gel denotes how dark the substrate DNA band is.

### Example 8. Identification of non-specific DNase activity of mgCas12a

To identify random DNase functions of the Cas12a (AsCas12a, FnCas12a, or LbCas12a) and the mgCas12a (WT mgCas12a-1, d_mgCas12a-1, WTmgCas12a-2, or d_mgCas12a-2), an experiment was performed in the same manner as in Example 7. Here, the d-mgCas12a-1 and the d_mgCas12a-2 refer to proteins obtained from the WT mgCas12a-1 and the WT mgCas12a-2, respectively, by substitution of Asp (at position 877 for the WT mgCas12a-1 or at position 873 for the WT mgCas12a-2) with Ala.

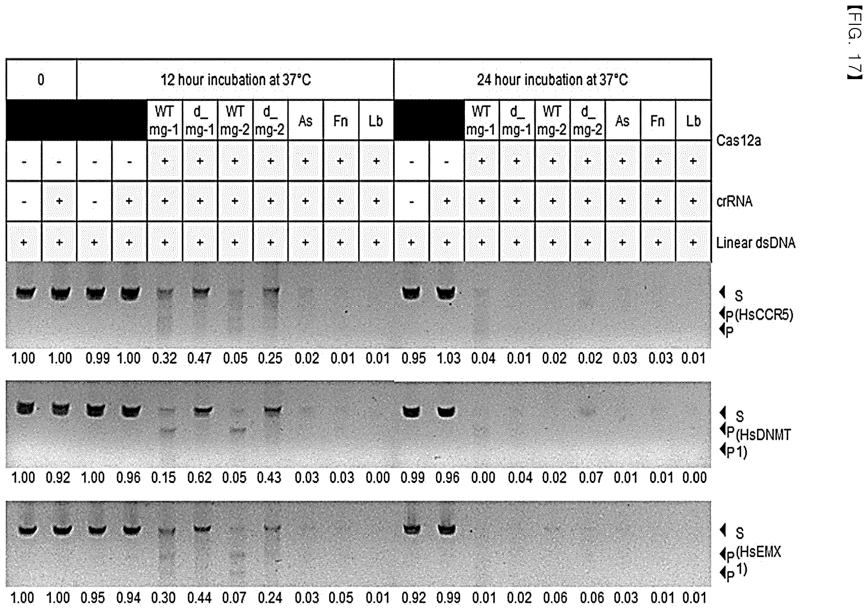

Specifically, to form each ribonucleoprotein (RNP) complex consisting of each of the 7 types of Cas12a and crRNA, 6 pmol of each Cas12a protein and 7.5 pmol of crRNA were allowed to react at room temperature for 30 minutes in the presence of NEB 1.1 buffer and IX distilled water. Subsequently, 0.3 pmol of target dsDNA was added thereto, and then reaction was allowed to proceed at 37°C for 12 hours or 24 hours. Here, HsCCR5, HsDNMT1, and HsEMX1 were used as DNA. SDS and EDTA (gel loading dye, NEB) were added thereto, and then the mixture was stored at -20°C for 10 minutes to stop the reaction. Each DNA was loaded on a 1% agarose gel, and then subjected to electrophoresis to check the DNA cleavage activity caused by the 7 types of Cas12a. The results are illustrated in FIG. 17. In FIG. 17, S denotes a substrate, and each number indicated at the bottom of the gel denotes how dark the substrate DNA band is.

As illustrated in FIG. 17, each ribonucleoprotein complex consisting of the WT mgCas12a-1, d_mgCas12a-1, WTmgCas12a-2, or d_mgCas12a-2, which is novel Cas12a, and crRNA exhibited a weaker non-specific DNase function than the ribonucleoprotein complex consisting of the AsCas12a, FnCas12a, or LbCas12a, which is existing Cas12a, and crRNA. In addition, overall, it could be presumed that reaction of the Cas12a RNP with DNA results in a non-specific DNase function.

### Example 9. Identification of non-specific DNase function of Cas12a under crRNA-free condition

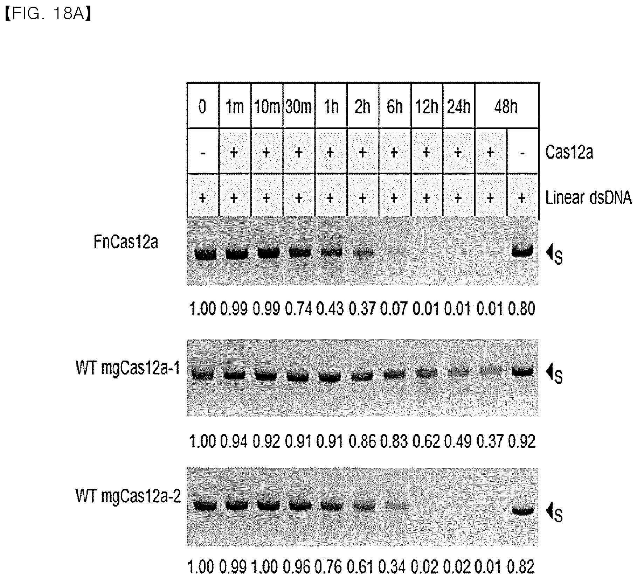

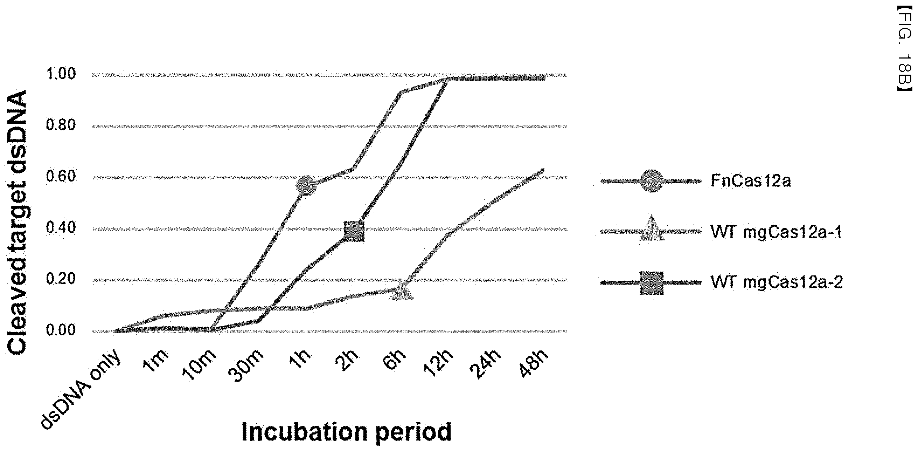

To identify whether Cas12a has a random DNase function even without crRNA, for the FnCas12a, WT mgCas12a-1, or WT mgCas12a-2 protein, an experiment was performed in the same manner as in Example 7 with varying times, except that a crRNA-free condition was used. The results are illustrated in FIGS. 18A and 18B. As illustrated in FIGS. 18A and 18B, the FnCas12a, WT mgCas12a-1, or WT mgCas12a-2 protein had a random DNase function even without crRNA, in which the random DNase function of the FnCas12a protein appeared first.

### Example 10. Identification of DNA cleavage function of mgCas12a using handle of existing Cas12a

To identify whether the new Cas12a (d_mgCas12a or WT mgCas12a) can perform DNA cleavage using a handle located at the 5' end of the existing Cas12a (AsCas12a, FnCas12a, or LbCas12a) sequence, an experiment was performed in the same manner as in Example 7 with varying reaction times, except that the handle of each of the AsCas12a, FnCas12a, or LbCas12a was used. The results are illustrated in FIG. 19.

As illustrated in FIG. 19, in a case where DNA cleavage was performed with the d_mgCas12a or WT mgCas12a protein using the handle of the AsCas12a, FnCas12a or LbCas12a, all d_mgCas12a or WT mgCas12a proteins using the three types of handles had a DNA cleavage function, although the DNA cleavage efficiency was slightly different depending on the respective handles. From these results, it was found that for DNA cleavage, the mgCas 12a can use the handle of the AsCas12a, FnCas12a, or LbCas12a.

### Example 11. Identification of activity of FnCas12a or mgCas12a in divalent ions

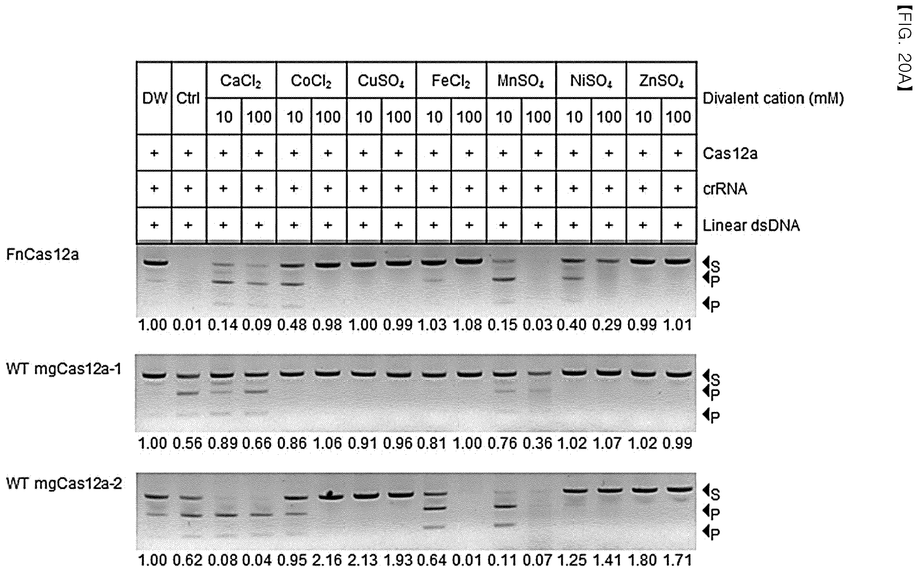

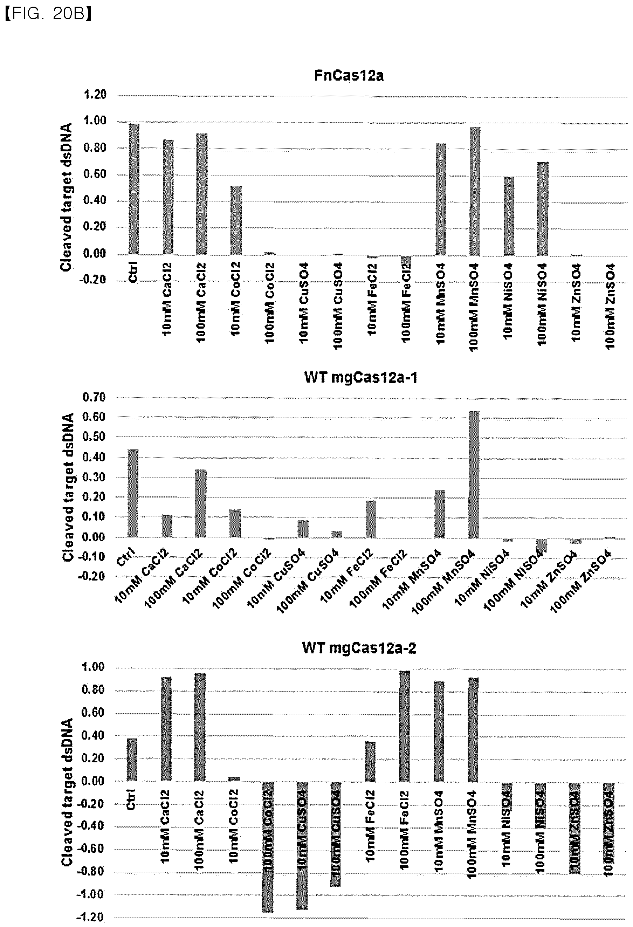

In addition, to identify DNA cleavage activity of the FnCas12a, mgCas12a-1, or mgCas12a-2 protein in divalent ions (CaCl₂, CoCl₂, CuSO₄, FeCl₂, MnSO₄, NiSO₄, or ZnSO₄), an experiment was performed in the same manner as in Example 4, except that a predetermined amount of divalent ions was used in place of the NEBuffer 1.1. The results are illustrated in FIGS. 20A and 20B. As illustrated in FIGS. 20A and 20B, the FnCas12a, mgCas12a-1, or mgCas12a-2 protein exhibited similar DNA cleavage activity in the same divalent ions.

## Claims

1. A Cas12a protein, having the amino acid sequence of SEQ ID NO: 1.

2. The Cas12a protein of claim 1, wherein the Cas12a protein having the amino acid sequence of SEQ ID NO: 1 is encoded by the nucleotide sequence of SEQ ID NO: 2.

3. The Cas12a protein of claim 1, wherein the protein has endonuclease activity.

4. The Cas12a protein of claim 1, wherein the Cas12a protein having the amino acid sequence of SEQ ID NO: 1 has optimal activity at pH 7.0 to pH 7.9.

5. A Cas12a protein, having the amino acid sequence of SEQ ID NO: 1, of which lysine (Lys) at position 925 is substituted with another amino acid.

6. The Cas12a protein of claim 5, wherein the other amino acid is any one selected from the group consisting of arginine (Arg), histidine (His), aspartic acid (Asp), glutamic acid (Glu), serine (Ser), threonine (Thr), asparagine (Asn), glutamine (Gln), tyrosine (Tyr), alanine (Ala), isoleucine (Ile), leucine (Leu), valine (Val), phenylalanine (Phe), methionine (Met), tryptophan (Trp), glycine (Gly), proline (Pro), and cysteine (Cys).

7. A Cas12a protein, having the amino acid sequence of SEQ ID NO: 3.

8. The Cas12a protein of claim 7, wherein the Cas12a protein having the amino acid sequence of SEQ ID NO: 3 is encoded by the nucleotide sequence of SEQ ID NO: 4.

9. The Cas12a protein of claim 7, wherein the protein has endonuclease activity.

10. The Cas12a protein of claim 7, wherein the Cas12a protein having the amino acid sequence of SEQ ID NO: 3 has optimal activity at pH 7.0 to pH 7.9.

11. A Cas12a protein, having the amino acid sequence of SEQ ID NO: 3, of which lysine (Lys) at position 930 is substituted with another amino acid.

12. The Cas12a protein of claim 11, wherein the other amino acid is any one selected from the group consisting of arginine (Arg), histidine (His), aspartic acid (Asp), glutamic acid (Glu), serine (Ser), threonine (Thr), asparagine (Asn), glutamine (Gln), tyrosine (Tyr), alanine (Ala), isoleucine (Ile), leucine (Leu), valine (Val), phenylalanine (Phe), methionine (Met), tryptophan (Trp), glycine (Gly), proline (Pro), and cysteine (Cys).

13. A Cas12a protein, having the amino acid sequence of SEQ ID NO: 1, of which aspartic acid (Asp) at position 877 is substituted with another amino acid.

14. The Cas12a protein of claim 13, wherein the other amino acid is any one selected from the group consisting of arginine (Arg), histidine (His), glutamic acid (Glu), serine (Ser), threonine (Thr), asparagine (Asn), glutamine (Gln), tyrosine (Tyr), alanine (Ala), lysine (Lys), isoleucine (Ile), leucine (Leu), valine (Val), phenylalanine (Phe), methionine (Met), tryptophan (Trp), glycine (Gly), proline (Pro), and cysteine (Cys).

15. The Cas12a protein of claim 13, wherein the protein has decreased endonuclease activity.

16. A Cas12a protein, having the amino acid sequence of SEQ ID NO: 3, of which aspartic acid (Asp) at position 873 is substituted with another amino acid.

17. The Cas12a protein of claim 16, wherein the other amino acid is any one selected from the group consisting of arginine (Arg), histidine (His), glutamic acid (Glu), serine (Ser), threonine (Thr), asparagine (Asn), glutamine (Gln), tyrosine (Tyr), alanine (Ala), lysine (Lys), isoleucine (Ile), leucine (Leu), valine (Val), phenylalanine (Phe), methionine (Met), tryptophan (Trp), glycine (Gly), proline (Pro), and cysteine (Cys).

18. The Cas12a protein of claim 16, wherein the protein has decreased endonuclease activity.

19. A pharmaceutical composition for treating cancer, comprising as active ingredients:
mgCas12a; and
crRNA that targets a nucleic acid sequence specifically present in cancer cells.

20. The pharmaceutical composition of claim 19, wherein the mgCas12a has any one amino acid sequence selected from the group consisting of SEQ ID NO: 1, SEQ ID NO: 3, SEQ ID NO: 5, and SEQ ID NO: 6.


<110>    g+flas life sciences
         Seoul National University R&DB Foundation

<120>    NOVEL CRISPR ASSOCIATED PROTEIN AND USE THEREOF

<130>    PCB907065GFS

<150>    KR 10-2018-0093336
<151>    2018-08-09

<160>    32

<170>    KoPatentIn 3.0

<210>    1
<211>    1263
<212>    PRT
<213>    Artificial Sequence

<220>
<223>    mgCas12a-1


<400>    1
Met Asn Asn Gly Thr Asn Asn Phe Gln Asn Phe Ile Gly Ile Ser Ser
  1               5                  10                  15

Leu Gln Lys Thr Leu Arg Asn Ala Leu Ile Pro Thr Glu Thr Thr Gln
             20                  25                  30

Gln Phe Ile Val Lys Asn Gly Ile Ile Lys Glu Asp Glu Leu Arg Gly
         35                  40                  45

Glu Asn Arg Gln Ile Leu Lys Asp Ile Met Asp Asp Tyr Tyr Arg Gly
     50                  55                  60

Phe Ile Ser Glu Thr Leu Ser Ser Ile Asp Asp Ile Asp Trp Thr Ser
 65                  70                  75                  80

Leu Phe Glu Lys Met Glu Ile Gln Leu Lys Asn Gly Asp Asn Lys Asp
                 85                  90                  95

Thr Leu Ile Lys Glu Gln Ala Glu Lys Arg Lys Ala Ile Tyr Lys Lys
            100                 105                 110

Phe Ala Asp Asp Asp Arg Phe Lys Asn Met Phe Ser Ala Lys Leu Ile
        115                 120                 125

Ser Asp Ile Leu Pro Glu Phe Val Ile His Asn Asn Asn Tyr Ser Ala
    130                 135                 140

Ser Glu Lys Glu Glu Lys Thr Gln Val Ile Lys Leu Phe Ser Arg Phe
145                 150                 155                 160

Ala Thr Ser Phe Lys Asp Tyr Phe Lys Asn Arg Ala Asn Cys Phe Ser
                165                 170                 175

Ala Asp Asp Ile Ser Ser Ser Ser Cys His Arg Ile Val Asn Asp Asn
            180                 185                 190

Ala Glu Ile Phe Phe Ser Asn Ala Leu Val Tyr Arg Arg Ile Val Lys
        195                 200                 205

Asn Leu Ser Asn Asp Asp Ile Asn Lys Ile Ser Gly Asp Ile Lys Asp
    210                 215                 220

Ser Leu Lys Glu Met Ser Leu Glu Glu Ile Tyr Ser Tyr Glu Lys Tyr
225                 230                 235                 240

Gly Glu Phe Ile Thr Gln Glu Gly Ile Ser Phe Tyr Asn Asp Ile Cys
                245                 250                 255

Gly Lys Val Asn Ser Phe Met Asn Leu Tyr Cys Gln Lys Asn Lys Glu
            260                 265                 270

Asn Lys Asn Leu Tyr Lys Leu Arg Lys Leu His Lys Gln Ile Leu Cys
        275                 280                 285

Ile Ala Asp Thr Ser Tyr Glu Val Pro Tyr Lys Phe Glu Ser Asp Glu
    290                 295                 300

Glu Val Tyr Gln Ser Val Asn Gly Phe Leu Asp Asn Ile Ser Ser Lys
305                 310                 315                 320

His Ile Val Glu Arg Leu Arg Lys Ile Gly Asp Asn Tyr Asn Gly Tyr
                325                 330                 335

Asn Leu Asp Lys Ile Tyr Ile Val Ser Lys Phe Tyr Glu Ser Val Ser
            340                 345                 350

Gln Lys Thr Tyr Arg Asp Trp Glu Thr Ile Asn Thr Ala Leu Glu Ile
        355                 360                 365

His Tyr Asn Asn Ile Leu Pro Gly Asn Gly Lys Ser Lys Ala Asp Lys
    370                 375                 380

Val Lys Lys Ala Val Lys Asn Asp Leu Gln Lys Ser Ile Thr Glu Ile
385                 390                 395                 400

Asn Glu Leu Val Ser Asn Tyr Lys Leu Cys Pro Asp Asp Asn Ile Lys
                405                 410                 415

Ala Glu Thr Tyr Ile His Glu Ile Ser His Ile Leu Asn Asn Phe Glu
            420                 425                 430

Ala Gln Glu Leu Lys Tyr Asn Pro Glu Ile His Leu Val Glu Ser Glu
        435                 440                 445

Leu Lys Ala Ser Glu Leu Lys Asn Val Leu Asp Val Ile Met Asn Ala
    450                 455                 460

Phe His Trp Cys Ser Val Phe Met Thr Glu Glu Leu Val Asp Lys Asp
465                 470                 475                 480

Asn Asn Phe Tyr Ala Glu Leu Glu Glu Ile Tyr Asp Glu Ile Tyr Thr
                485                 490                 495

Val Ile Ser Leu Tyr Asn Leu Val Arg Asn Tyr Val Thr Gln Lys Pro
            500                 505                 510

Tyr Ser Thr Lys Lys Ile Lys Leu Asn Phe Gly Ile Pro Thr Leu Ala
        515                 520                 525

Asp Gly Trp Ser Lys Ser Lys Glu Tyr Ser Asn Asn Ala Ile Ile Leu
    530                 535                 540

Met Arg Asp Asn Leu Tyr Tyr Leu Gly Ile Phe Asn Ala Lys Asn Lys
545                 550                 555                 560

Pro Asp Lys Lys Ile Ile Glu Gly Asn Thr Ser Glu Asn Lys Gly Asp
                565                 570                 575

Tyr Lys Lys Met Ile Tyr Asn Leu Leu Pro Gly Pro Asn Lys Met Ile
            580                 585                 590

Pro Lys Val Phe Leu Ser Ser Lys Thr Gly Val Glu Thr Tyr Lys Pro
        595                 600                 605

Ser Ala Tyr Ile Leu Glu Gly Tyr Lys Gln Asn Lys His Leu Lys Ser
    610                 615                 620

Ser Lys Asp Phe Asp Ile Thr Phe Cys His Asp Leu Ile Asp Tyr Phe
625                 630                 635                 640

Lys Asn Cys Ile Ala Ile His Pro Glu Trp Lys Asn Phe Gly Phe Asp
                645                 650                 655

Phe Ser Asp Thr Ser Thr Tyr Glu Asp Ile Ser Gly Phe Tyr Arg Glu
            660                 665                 670

Val Glu Leu Gln Gly Tyr Lys Ile Asp Trp Thr Tyr Ile Ser Glu Lys
        675                 680                 685

Asp Ile Asp Leu Leu Gln Glu Lys Gly Gln Leu Tyr Leu Phe Gln Ile
    690                 695                 700

Tyr Asn Lys Asp Phe Ser Lys Lys Ser Thr Gly Asn Asp Asn Leu His
705                 710                 715                 720

Thr Met Tyr Leu Lys Asn Leu Phe Ser Glu Glu Asn Leu Lys Asp Ile
                725                 730                 735

Val Leu Lys Leu Asn Gly Glu Ala Glu Ile Phe Phe Arg Lys Ser Ser
            740                 745                 750

Ile Lys Asn Pro Ile Ile His Lys Lys Gly Ser Ile Leu Val Asn Arg
        755                 760                 765

Thr Tyr Glu Ala Glu Glu Lys Asp Gln Phe Gly Asn Ile Gln Ile Val
    770                 775                 780

Arg Lys Thr Ile Pro Glu Asn Ile Tyr Gln Glu Leu Tyr Lys Tyr Phe
785                 790                 795                 800

Asn Asp Lys Ser Asp Lys Glu Leu Ser Asp Glu Ala Ala Lys Leu Lys
                805                 810                 815

Asn Val Val Gly His His Glu Ala Ala Thr Asn Ile Val Lys Asp Tyr
            820                 825                 830

Arg Tyr Thr Tyr Asp Lys Tyr Phe Leu His Met Pro Ile Thr Ile Asn
        835                 840                 845

Phe Lys Ala Asn Lys Thr Ser Phe Ile Asn Asp Arg Ile Leu Gln Tyr
    850                 855                 860

Ile Ala Lys Glu Lys Asn Leu His Val Ile Gly Ile Asp Arg Gly Glu
865                 870                 875                 880

Arg Asn Leu Ile Tyr Val Ser Val Ile Asp Thr Cys Gly Asn Ile Val
                885                 890                 895

Glu Gln Lys Ser Phe Asn Ile Val Asn Gly Tyr Asp Tyr Gln Ile Lys
            900                 905                 910

Leu Lys Gln Gln Glu Gly Ala Arg Gln Ile Ala Arg Lys Glu Trp Lys
        915                 920                 925

Glu Ile Gly Lys Ile Lys Glu Ile Lys Glu Gly Tyr Leu Ser Leu Val
    930                 935                 940

Ile His Glu Ile Ser Lys Met Val Ile Lys Tyr Asn Ala Ile Ile Ala
945                 950                 955                 960

Met Glu Asp Leu Ser Tyr Gly Phe Lys Lys Gly Arg Phe Lys Val Glu
                965                 970                 975

Arg Gln Val Tyr Gln Lys Phe Glu Thr Met Leu Ile Asn Lys Leu Asn
            980                 985                 990

Tyr Leu Val Phe Lys Asp Ile Ser Ile Thr Glu Asn Gly Gly Leu Leu
        995                 1000                1005

Lys Gly Tyr Gln Leu Thr Tyr Ile Pro Asp Lys Leu Lys Asn Val Gly
    1010                1015                1020

His Gln Cys Gly Cys Ile Phe Tyr Val Pro Ala Ala Tyr Thr Ser Lys
1025                1030                1035                1040

Ile Asp Pro Thr Thr Gly Phe Val Asn Ile Phe Lys Phe Lys Asp Leu
                1045                1050                1055

Thr Val Asp Ala Lys Arg Glu Phe Ile Lys Lys Phe Asp Ser Ile Arg
            1060                1065                1070

Tyr Asp Ser Glu Lys Lys Leu Phe Cys Phe Thr Phe Asp Tyr Asn Asn
        1075                1080                1085

Phe Ile Thr Gln Asn Thr Val Met Ser Lys Ser Ser Trp Ser Val Tyr
    1090                1095                1100

Thr Tyr Gly Val Arg Ile Lys Arg Arg Phe Val Asn Gly Arg Phe Ser
1105                1110                1115                1120

Asn Glu Ser Asp Thr Ile Asp Ile Thr Lys Asp Met Glu Lys Thr Leu
                1125                1130                1135

Glu Met Thr Asp Ile Asn Trp Arg Asp Gly His Asp Leu Arg Gln Asp
            1140                1145                1150

Ile Ile Asp Tyr Glu Ile Val Gln His Ile Phe Glu Ile Phe Arg Leu
        1155                1160                1165

Thr Val Gln Met Arg Asn Ser Leu Ser Glu Leu Glu Asp Arg Asp Tyr
    1170                1175                1180

Asp Arg Leu Ile Ser Pro Val Leu Asn Glu Asn Asn Ile Phe Tyr Asp
1185                1190                1195                1200

Ser Ala Lys Ala Gly Asp Ala Leu Pro Lys Asp Ala Asp Ala Asn Gly
                1205                1210                1215

Ala Tyr Cys Ile Ala Leu Lys Gly Leu Tyr Glu Ile Lys Gln Ile Thr
            1220                1225                1230

Glu Asn Trp Lys Glu Asp Gly Lys Phe Ser Arg Asp Lys Leu Lys Ile
        1235                1240                1245

Ser Asn Lys Asp Trp Phe Asp Phe Ile Gln Asn Lys Arg Tyr Leu
    1250                1255                1260

<210>    2
<211>    3792
<212>    DNA
<213>    Artificial Sequence

<220>
<223>    mgCas12a-1

<400>    2
atgaataacg gaacaaataa ctttcagaac tttatcggaa tttcttcttt gcagaagact      60

cttaggaatg ctctcattcc aacagaaaca acacagcaat ttattgttaa aaatggaata     120

attaaagaag atgaactcag aggagaaaat cgtcagatac ttaaagatat catggatgat     180

tattacagag gtttcatttc agaaacttta tcgtcaattg atgatattga ctggacctct     240

ttatttgaga aaatggaaat tcagttaaaa aatggagata ataaagacac tcttataaaa     300

gaacaggctg aaaaacgtaa ggcaatctat aaaaaatttg cagatgatga tagatttaaa     360

aatatgttca gtgcaaaatt aatctcagat attcttcctg aatttgtcat tcataacaat     420

aattattctg catcagaaaa ggaagaaaaa acacaggtaa ttaaattatt ttccagattt     480

gcaacatcat tcaaggacta ttttaaaaac agggctaatt gtttttctgc tgatgatata     540

tcttcttctt cttgtcatag aatagttaat gataatgcag aaatattttt tagtaatgca     600

ttggtgtata ggagaattgt aaaaaatctt tcaaatgatg atataaataa aatatccgga     660

gatattaagg attcattaaa ggaaatgtct ctggaggaaa tttattctta tgaaaaatat     720

ggggaattta ttacacagga aggtatatct ttttataatg atatatgcgg taaagtaaat     780

tcatttatga atttatattg ccagaaaaat aaagaaaaca aaaatctcta taagctgcga     840

aagcttcata aacagatact gtgcatagca gatacttctt atgaggtgcc gtataaattt     900

gaatcagatg aagaggttta tcaatcagtg aatggatttt tggacaatat tagttcaaaa     960

catatcgttg aaagattgcg taagattgga gacaactata acggctacaa tcttgataag    1020

atttatattg ttagtaaatt ctatgaatca gtttcacaaa agacatatag agattgggaa    1080

acaataaata ctgcattaga aattcattac aacaatatat tacccggaaa tggtaaatct    1140

aaagctgaca aggtaaaaaa agcggtaaag aatgatctgc aaaaaagcat tactgaaatc    1200

aatgagcttg ttagcaatta taaattatgt ccggatgata atattaaagc agagacatat    1260

atacatgaaa tatcacatat tttgaataat tttgaagcac aggagcttaa gtataatcct 1320

gaaattcatc tggtggaaag tgaattgaaa gcatctgaat taaaaaatgt tctcgatgta 1380

ataatgaatg cttttcattg gtgttcggtt ttcatgacag aggagctggt agataaagat 1440

aataattttt atgcggagtt agaagagata tatgacgaaa tatatacggt aatttcattg 1500

tataatcttg tgcgtaatta tgtaacgcag aagccatata gtacaaaaaa aattaaattg 1560

aattttggta ttcctacact agcggatgga tggagtaaaa gtaaagaata tagtaataat 1620

gcaattattc tcatgcgtga taatttgtac tatttaggaa tatttaatgc aaaaaataag 1680

cctgacaaaa agataattga aggtaataca tcagaaaata aaggggatta taagaagatg 1740

atttataatc ttctgccagg accaaataaa atgatcccca aggtattcct ctcttcaaaa 1800

accggagtgg aaacatataa gccgtctgcc tatatattgg agggctataa acaaaacaag 1860

catcttaaat cctctaagga ttttgatata acgttttgtc acgatttgat tgattatttt 1920

aagaactgta tagcaataca tcctgaatgg aagaattttg gctttgattt ttctgacacc 1980

tccacatatg aagatatcag cggattttac agagaagtcg aattgcaagg ttataaaatt 2040

gactggacat atatcagcga aaaggatatt gatttgttgc aggaaaaagg acagttatat 2100

ttatttcaaa tatataacaa agatttttcc aagaaaagta ccggaaatga taatcttcat 2160

actatgtatt tgaagaattt gtttagcgaa gagaatttaa aggatattgt actgaaatta 2220

aacggtgagg cggaaatctt ctttagaaaa tcaagcataa agaatccaat aattcataaa 2280

aaaggctcta ttcttgttaa tagaacatat gaagcagagg aaaaagatca atttggaaat 2340

atccagatag tcagaaaaac cataccggaa aatatatatc aggagcttta taaatatttc 2400

aatgataaaa gtgataaaga actttcggat gaagcagcta agcttaagaa tgtagtaggt 2460

catcatgagg ctgctacaaa catagtaaaa gattatagat atacatatga taaatatttt 2520

cttcatatgc ctattacaat caattttaaa gccaataaga caagctttat taatgacaga 2580

atattacaat atattgctaa agaaaagaat ttgcatgtaa taggcattga tcgtggtgaa 2640

agaaacctga tatatgtttc agtaattgat acttgtggaa atattgttga acaaaaatcg 2700

tttaacattg ttaatggata tgattatcag attaagctca agcagcagga gggggcgcga 2760

caaatcgcac gaaaagaatg gaaagaaatc ggcaaaataa aagaaattaa agaaggctat 2820

ttatctcttg taattcatga aatttcaaag atggttatta aatataatgc cataattgca 2880

atggaggatt taagctacgg atttaaaaaa ggtcgtttca aggttgagcg acaggtttac 2940

cagaagtttg agacaatgct tatcaacaaa ctcaactatc tggtatttaa agatatatcc 3000

ataactgaaa acggtggtct tctaaaggga tatcagctta catatattcc agataaactg 3060

aaaaatgtgg gtcatcaatg tggttgtata ttttacgtac ctgctgccta tacatcaaaa 3120

atagatccta caaccggatt tgtaaatata ttcaaattta aagatttaac agttgatgca 3180

aagagagaat ttataaaaaa atttgacagt atcagatatg attcagaaaa aaaactgttt 3240

tgttttacat ttgattataa taactttatt acgcaaaata ctgttatgtc aaagtcaagc 3300

tggagtgtat atacgtacgg agttaggata aaaagaagat ttgtcaatgg caggttctca 3360

aatgaatcgg atacaattga tataacaaaa gatatggaaa aaaccctcga aatgacagat 3420

ataaattgga gagatggtca tgatctgagg caggatatta ttgattatga aatcgtacaa 3480

cacatatttg agatttttag attgactgta caaatgagaa acagtttaag tgaattagaa 3540

gacagggatt atgaccgttt gatttctccg gtgctcaatg aaaataatat attttatgat 3600

tcagctaaag caggagatgc gttacctaaa gacgcagatg ctaatggtgc atattgtata 3660

gctctaaaag gcttgtatga aatcaaacaa attacagaga attggaaaga agacggtaag 3720

ttttcaagag ataaacttaa aatttccaat aaggactggt ttgactttat tcaaaataaa 3780

aggtatttat aa 3792

<210> 3
<211> 1275
<212> PRT
<213> Artificial Sequence

<220>
<223> mgCas12a-2

<400> 3
Met Gly Lys Asn Gln Asn Phe Gln Glu Phe Ile Gly Val Ser Pro Leu
1               5                   10                  15

Gln Lys Thr Leu Arg Asn Glu Leu Ile Pro Thr Glu Thr Thr Lys Lys
            20                  25                  30

Asn Ile Thr Gln Leu Asp Leu Leu Thr Glu Asp Glu Ile Arg Ala Gln
        35                  40                  45

Asn Arg Glu Lys Leu Lys Glu Met Met Asp Asp Tyr Tyr Arg Asn Val
    50                  55                  60

Ile Asp Ser Thr Leu His Val Gly Ile Ala Val Asp Trp Ser Tyr Leu
65                  70                  75                  80

Phe Ser Cys Met Arg Asn His Leu Arg Glu Asn Ser Lys Glu Ser Lys
                85                  90                  95

Arg Glu Leu Glu Arg Thr Gln Asp Ser Ile Arg Ser Gln Ile His Asn
            100                 105                 110

Lys Phe Ala Glu Arg Ala Asp Phe Lys Asp Met Phe Gly Ala Ser Ile
        115                 120                 125

Ile Thr Lys Leu Leu Pro Thr Tyr Ile Lys Gln Asn Ser Glu Tyr Ser
    130                 135                 140

Glu Arg Tyr Asp Glu Ser Met Glu Ile Leu Lys Leu Tyr Gly Lys Phe

145                 150                 155                 160

Thr Thr Ser Leu Thr Asp Tyr Phe Glu Thr Arg Lys Asn Ile Phe Ser
                165                 170                 175

Lys Glu Lys Ile Ser Ser Ala Val Gly Tyr Arg Ile Val Glu Glu Asn
            180                 185                 190

Ala Glu Ile Phe Leu Gln Asn Gln Asn Ala Tyr Asp Arg Ile Cys Lys
        195                 200                 205

Ile Ala Gly Leu Asp Leu His Gly Leu Asp Asn Glu Ile Thr Ala Tyr
    210                 215                 220

Val Asp Gly Lys Thr Leu Lys Glu Val Cys Ser Asp Glu Gly Phe Ala
225                 230                 235                 240

Lys Ala Ile Thr Gln Glu Gly Ile Asp Arg Tyr Asn Glu Ala Ile Gly
                245                 250                 255

Ala Val Asn Gln Tyr Met Asn Leu Leu Cys Gln Lys Asn Lys Ala Leu
            260                 265                 270

Lys Pro Gly Gln Phe Lys Met Lys Arg Leu His Lys Gln Ile Leu Cys
        275                 280                 285

Lys Gly Thr Thr Ser Phe Asp Ile Pro Lys Lys Phe Glu Asn Asp Lys
    290                 295                 300

Gln Val Tyr Asp Ala Val Asn Ser Phe Thr Glu Ile Val Thr Lys Asn
305                 310                 315                 320

Asn Asp Leu Lys Arg Leu Leu Asn Ile Thr Gln Asn Ala Asn Asp Tyr
                325                 330                 335

Asp Met Asn Lys Ile Tyr Val Val Ala Asp Ala Tyr Ser Met Ile Ser
            340                 345                 350

Gln Phe Ile Ser Lys Lys Trp Asn Leu Ile Glu Glu Cys Leu Leu Asp
        355                 360                 365

Tyr Tyr Ser Asp Asn Leu Pro Gly Lys Gly Asn Ala Lys Glu Asn Lys
    370                 375                 380

Val Lys Lys Ala Val Lys Glu Glu Thr Tyr Arg Ser Val Ser Gln Leu
385                 390                 395                 400

Asn Glu Val Ile Glu Lys Tyr Tyr Val Glu Lys Thr Gly Gln Ser Val
                405                 410                 415

Trp Lys Val Glu Ser Tyr Ile Ser Ser Leu Ala Glu Met Ile Lys Leu
            420                 425                 430

Glu Leu Cys His Glu Ile Asp Asn Asp Glu Lys His Asn Leu Ile Glu
        435                 440                 445

Asp Asp Glu Lys Ile Ser Glu Ile Lys Glu Leu Leu Asp Met Tyr Met
    450                 455                 460

Asp Val Phe His Ile Ile Lys Val Phe Arg Val Asn Glu Val Leu Asn
465                 470                 475                 480

Phe Asp Glu Thr Phe Tyr Ser Glu Met Asp Glu Ile Tyr Gln Asp Met

485                 490                 495

Gln Glu Ile Val Pro Leu Tyr Asn His Val Arg Asn Tyr Val Thr Gln
            500                 505                 510

Lys Pro Tyr Lys Gln Glu Lys Tyr Arg Leu Tyr Phe His Thr Pro Thr
        515                 520                 525

Leu Ala Asn Gly Trp Ser Lys Ser Lys Glu Tyr Asp Asn Asn Ala Ile
    530                 535                 540

Ile Leu Val Arg Glu Asp Lys Tyr Tyr Leu Gly Ile Leu Asn Ala Lys
545                 550                 555                 560

Lys Lys Pro Ser Lys Glu Ile Met Ala Gly Lys Glu Asp Cys Ser Glu
                565                 570                 575

His Ala Tyr Ala Lys Met Asn Tyr Tyr Leu Leu Pro Gly Ala Asn Lys
            580                 585                 590

Met Leu Pro Lys Val Phe Leu Ser Lys Lys Gly Ile Gln Asp Tyr His
        595                 600                 605

Pro Ser Ser Tyr Ile Val Glu Gly Tyr Asn Glu Lys Lys His Ile Lys
    610                 615                 620

Gly Ser Lys Asn Phe Asp Ile Arg Phe Cys Arg Asp Leu Ile Asp Tyr
625                 630                 635                 640

Phe Lys Glu Cys Ile Lys Lys His Pro Asp Trp Asn Lys Phe Asn Phe
                645                 650                 655

Glu Phe Ser Ala Thr Glu Thr Tyr Glu Asp Ile Ser Val Phe Tyr Arg
            660                 665                 670

Glu Val Glu Lys Gln Gly Tyr Arg Val Glu Trp Thr Tyr Ile Asn Ser
        675                 680                 685

Glu Asp Ile Gln Lys Leu Glu Glu Asp Gly Gln Leu Phe Leu Phe Gln
    690                 695                 700

Ile Tyr Asn Lys Asp Phe Ala Val Gly Ser Thr Gly Lys Pro Asn Leu
705                 710                 715                 720

His Thr Leu Tyr Leu Lys Asn Leu Phe Ser Glu Glu Asn Leu Arg Asp
                725                 730                 735

Ile Val Leu Lys Leu Asn Gly Glu Ala Glu Ile Phe Phe Arg Lys Ser
            740                 745                 750

Ser Val Gln Lys Pro Val Ile His Lys Cys Gly Ser Ile Leu Val Asn
        755                 760                 765

Arg Thr Tyr Glu Ile Thr Glu Ser Gly Thr Thr Arg Val Gln Ser Ile
    770                 775                 780

Pro Glu Ser Glu Tyr Met Glu Leu Tyr Arg Tyr Phe Asn Ser Glu Lys
785                 790                 795                 800

Gln Ile Glu Leu Ser Asp Glu Ala Lys Lys Tyr Leu Asp Lys Val Gln
                805                 810                 815

Cys Asn Lys Ala Lys Thr Asp Ile Val Lys Asp Tyr Arg Tyr Thr Met

820                 825                 830

Asp Lys Phe Phe Ile His Leu Pro Ile Thr Ile Asn Phe Lys Val Asp
        835                 840                 845

Lys Gly Asn Asn Val Asn Ala Ile Ala Gln Gln Tyr Ile Ala Gly Arg
    850                 855                 860

Lys Asp Leu His Val Ile Gly Ile Asp Arg Gly Glu Arg Asn Leu Ile
865                 870                 875                 880

Tyr Val Ser Val Ile Asp Met Tyr Gly Arg Ile Leu Glu Gln Lys Ser
                885                 890                 895

Phe Asn Leu Val Glu Gln Val Ser Ser Gln Gly Thr Lys Arg Tyr Tyr
            900                 905                 910

Asp Tyr Lys Glu Lys Leu Gln Asn Arg Glu Glu Glu Arg Asp Lys Ala
        915                 920                 925

Arg Lys Ser Trp Lys Thr Ile Gly Lys Ile Lys Glu Leu Lys Glu Gly
    930                 935                 940

Tyr Leu Ser Ser Val Ile His Glu Ile Ala Gln Met Val Val Lys Tyr
945                 950                 955                 960

Asn Ala Ile Ile Ala Met Glu Asp Leu Asn Tyr Gly Phe Lys Arg Gly
                965                 970                 975

Arg Phe Lys Val Glu Arg Gln Val Tyr Gln Lys Phe Glu Thr Met Leu
            980                 985                 990

Ile Ser Lys Leu Asn Tyr Leu Ala Asp Lys Ser Gln Ala Val Asp Glu
        995                 1000                1005

Pro Gly Gly Ile Leu Arg Gly Tyr Gln Met Thr Tyr Val Pro Asp Asn
    1010                1015                1020

Ile Lys Asn Val Gly Arg Gln Cys Gly Ile Ile Phe Tyr Val Pro Ala
1025                1030                1035                1040

Ala Tyr Thr Ser Lys Ile Asp Pro Thr Thr Gly Phe Ile Asn Ala Phe
                1045                1050                1055

Lys Arg Asp Val Val Ser Thr Asn Asp Ala Lys Glu Asn Phe Leu Met
            1060                1065                1070

Lys Phe Asp Ser Ile Gln Tyr Asp Ile Glu Lys Gly Leu Phe Lys Phe
        1075                1080                1085

Ser Phe Asp Tyr Lys Asn Phe Ala Thr His Lys Leu Thr Leu Ala Lys
    1090                1095                1100

Thr Lys Trp Asp Val Tyr Thr Asn Gly Thr Arg Ile Gln Asn Met Lys
1105                1110                1115                1120

Val Glu Gly His Trp Leu Ser Met Glu Val Glu Leu Thr Thr Lys Met
                1125                1130                1135

Lys Glu Leu Leu Asp Asp Ser His Ile Pro Tyr Glu Glu Gly Gln Asn
            1140                1145                1150

Ile Leu Asp Asp Leu Arg Glu Met Lys Asp Ile Thr Thr Ile Val Asn

1155                1160                1165

Gly Ile Leu Glu Ile Phe Trp Leu Thr Val Gln Leu Arg Asn Ser Arg
    1170                1175                1180

Ile Asp Asn Pro Asp Tyr Asp Arg Ile Ile Ser Pro Val Leu Asn Lys
1185                1190                1195                1200

Asn Gly Glu Phe Phe Asp Ser Asp Glu Tyr Asn Ser Tyr Ile Asp Ala
                1205                1210                1215

Gln Lys Ala Pro Leu Pro Ile Asp Ala Asp Ala Asn Gly Ala Phe Cys
            1220                1225                1230

Ile Ala Leu Lys Gly Met Tyr Thr Ala Asn Gln Ile Lys Glu Asn Trp
        1235                1240                1245

Val Glu Gly Glu Lys Leu Pro Ala Asp Cys Leu Lys Ile Glu His Ala
    1250                1255                1260

Ser Trp Leu Ala Phe Met Gln Gly Glu Arg Gly
1265                1270                1275

<210>    4
<211>    3828
<212>    DNA
<213>    Artificial Sequence

<220>
<223>    mgCas12a-2

<400>    4
atgggtaaaa atcaaaattt tcaggaattt attggggtat caccacttca aaagacttta      60

agaaacgaat taatcccaac agaaacaaca aaaaagaata ttactcagct tgatcttttg     120

actgaggatg aaatccgcgc gcaaaatcga gagaagctga aagagatgat ggatgactac     180

taccggaatg tgattgatag cactttgcat gtgggtatag ctgttgattg gagctattta     240

ttttcgtgta tgcgaaatca tctaagggag aattccaaag agtcaaagcg ggaattggaa     300

cgaacacagg attctattcg ttcacaaatc cataataagt ttgctgaacg agcggatttt     360

aaggatatgt ttggagcatc gataataaca aaattacttc cgacatatat aaaacagaat     420

tcagaatatt ccgagcggta tgacgagagc atggaaattt tgaaactgta tggaaaattc     480

acaacatcgt tgaccgatta ctttgagaca agaaagaata tcttttctaa agagaaaata     540

tcttctgccg ttggatatcg aatcgtagag gaaaatgctg agatcttctt gcagaatcag     600

aatgcttacg acagaatctg taagatagcg ggactggatt tacatggatt ggataatgaa     660

ataacagcat atgttgatgg aaaaacatta aaagaagtat gttcggatga aggatttgca     720

aaggctatta cacaagaagg gattgatcgc tacaacgagg caatcggtgc agtaaatcaa     780

tatatgaatc tgttatgcca gaagaataag gcattaaaac cgggacaatt taagatgaag     840

cggctacata aacagattct ttgcaaagga acaacctctt tcgatattcc aaagaagttt     900

gaaaatgata aacaggtgta tgacgcagtt aattctttta cagagatagt aacgaagaat 960

aatgatttga agcgactgtt aaatattaca cagaatgcaa atgattatga catgaataaa 1020

atctatgtag tagccgatgc atatagtatg atttcacagt ttatcagtaa aaaatggaat 1080

ctgattgaag aatgcttgct ggattattat agcgataatt tgccgggaaa aggaaatgcg 1140

aaagaaaaca aagttaaaaa ggcggtaaag gaagaaacgt atcgcagtgt ttcacagttg 1200

aatgaagtta ttgagaaata ttatgtggaa aagaccggac agtcagtatg gaaagtggaa 1260

agttatattt ctagtctggc agaaatgatt aagctggaat tgtgccacga gatagataac 1320

gatgagaagc ataatctgat tgaagatgat gagaagatat ccgagattaa ggaactgttg 1380

gatatgtaca tggatgtatt tcatattata aaagtgttcc gggtgaatga agtattgaat 1440

ttcgatgaaa ccttttattc ggagatggat gagatctatc aggatatgca ggaaatcgtt 1500

ccattataca atcatgttcg aaactatgtt acacagaaac catataagca ggagaaatat 1560

cgtttatatt tccacactcc aacattggca aatggctggt ccaagagtaa ggaatatgac 1620

aacaacgcaa ttatattggt gcgagaagat aaatattatt taggtattct gaatgcgaaa 1680

aagaaaccat cgaaagaaat tatggcgggc aaagaggatt gttcagaaca tgcatatgca 1740

aagatgaatt attatttgtt gccgggcgcg aacaagatgc ttccaaaagt atttttatct 1800

aagaaaggaa tacaggacta tcacccatca tcatatattg ttgaaggata taatgaaaag 1860

aaacatatta aaggttccaa gaattttgat atccggtttt gtagggattt gattgactac 1920

ttcaaggaat gcattaaaaa acatccggat tggaataagt ttaactttga attttctgcg 1980

acagaaacat atgaggatat cagtgtcttt tatcgcgaag ttgaaaagca aggatatcgc 2040

gtagagtgga cgtatatcaa tagtgaagat attcagaaac tggaagaaga tggacagttg 2100

tttttatttc agatatataa caaagatttt gctgtgggaa gtacaggtaa accaaatctt 2160

catacattgt atctgaaaaa tctgttcagc gaagaaaatt tgcgggacat tgtattaaaa 2220

ctaaatgggg aagcagaaat attcttccgt aaatcaagtg ttcaaaaacc ggtgattcat 2280

aagtgcggca gtattttagt caatcgtacc tatgagatta ccgagagtgg aacaacacgg 2340

gtacaatcaa ttccggaaag tgaatacatg gaattatatc gctactttaa tagtgaaaag 2400

cagatagaat tatcagatga ggcaaaaaaa tatttggaca aggtgcaatg taataaggca 2460

aagacagata ttgtgaaaga ctaccgatac accatggaca agttttttat tcatcttccg 2520

attacgatta attttaaggt tgataagggt aacaatgtta atgccattgc acagcaatat 2580

attgcagggc ggaaagattt acatgtgata ggaattgatc gaggagaacg gaatctgatt 2640

tacgtttctg taattgacat gtatggtaga attttagagc agaaatcctt taaccttgtg 2700

gaacaggtat cgtcgcaggg aacgaagcga tattacgatt acaaagaaaa attacagaac 2760

cgggaagagg aacgggataa agcaagaaag agttggaaga caatcggcaa gattaaggaa 2820

ttaaaagagg ggtatctgtc gtcagtaatt catgagattg cacagatggt cgtaaagtat 2880

aacgcaatca ttgcaatgga agatttgaat tatggattta agcggggaag attcaaagta 2940

gagcgccagg tatatcagaa atttgaaacg atgttgatca gtaagttgaa ttatctggca 3000

gataaatctc aggctgtgga tgaaccggga ggtatattac ggggatatca gatgacttat 3060

gtgccggata atattaagaa tgttggaaga caatgtggaa taatctttta tgtgccggca 3120

gcatatacct ccaagattga tccgacaacc ggatttatca atgcatttaa gcgggatgtg 3180

gtatcaacaa atgatgcaaa agagaatttc ctgatgaagt ttgattctat tcagtacgat 3240

atagaaaaag gcttatttaa gttttcattt gattacaaaa attttgccac acataaactt 3300

acacttgcga agacaaaatg ggacgtatat acaaatggaa ctcgaataca aaacatgaaa 3360

gttgaaggac attggctttc aatggaagtt gaacttacaa cgaaaatgaa agagttgctg 3420

gatgactcgc atattccata tgaagaagga cagaatatat tggatgattt gcgggagatg 3480

aaagatataa caaccattgt gaatggtata ttggaaatct tctggttgac agtccagctt 3540

cggaatagca ggatagataa tccggattac gatagaatta tctcaccggt attgaataaa 3600

aatggagaat tttttgattc tgatgaatat aattcatata ttgatgcgca aaaggcaccg 3660

ttaccgatag atgccgatgc aaatggcgca ttttgcattg cattaaaagg aatgtatact 3720

gccaatcaga tcaaagaaaa ctgggttgaa ggggagaaac ttccggcgga ttgcttgaag 3780

atcgaacatg cgagttggtt agcatttatg caaggagaaa ggggatag 3828

<210> 5
<211> 1263
<212> PRT
<213> Artificial Sequence

<220>
<223> engineered mgCas12a-1(K925Q)

<400> 5
Met Asn Asn Gly Thr Asn Asn Phe Gln Asn Phe Ile Gly Ile Ser Ser
1               5                   10                  15

Leu Gln Lys Thr Leu Arg Asn Ala Leu Ile Pro Thr Glu Thr Thr Gln
            20                  25                  30

Gln Phe Ile Val Lys Asn Gly Ile Ile Lys Glu Asp Glu Leu Arg Gly
        35                  40                  45

Glu Asn Arg Gln Ile Leu Lys Asp Ile Met Asp Asp Tyr Tyr Arg Gly
    50                  55                  60

Phe Ile Ser Glu Thr Leu Ser Ser Ile Asp Asp Ile Asp Trp Thr Ser
65                  70                  75                  80

Leu Phe Glu Lys Met Glu Ile Gln Leu Lys Asn Gly Asp Asn Lys Asp

85                  90                  95

Thr Leu Ile Lys Glu Gln Ala Glu Lys Arg Lys Ala Ile Tyr Lys Lys
            100                 105                 110

Phe Ala Asp Asp Asp Arg Phe Lys Asn Met Phe Ser Ala Lys Leu Ile
        115                 120                 125

Ser Asp Ile Leu Pro Glu Phe Val Ile His Asn Asn Asn Tyr Ser Ala
    130                 135                 140

Ser Glu Lys Glu Glu Lys Thr Gln Val Ile Lys Leu Phe Ser Arg Phe
145                 150                 155                 160

Ala Thr Ser Phe Lys Asp Tyr Phe Lys Asn Arg Ala Asn Cys Phe Ser
                165                 170                 175

Ala Asp Asp Ile Ser Ser Ser Ser Cys His Arg Ile Val Asn Asp Asn
            180                 185                 190

Ala Glu Ile Phe Phe Ser Asn Ala Leu Val Tyr Arg Arg Ile Val Lys
        195                 200                 205

Asn Leu Ser Asn Asp Asp Ile Asn Lys Ile Ser Gly Asp Ile Lys Asp
    210                 215                 220

Ser Leu Lys Glu Met Ser Leu Glu Glu Ile Tyr Ser Tyr Glu Lys Tyr
225                 230                 235                 240

Gly Glu Phe Ile Thr Gln Glu Gly Ile Ser Phe Tyr Asn Asp Ile Cys
                245                 250                 255

Gly Lys Val Asn Ser Phe Met Asn Leu Tyr Cys Gln Lys Asn Lys Glu
            260                 265                 270

Asn Lys Asn Leu Tyr Lys Leu Arg Lys Leu His Lys Gln Ile Leu Cys
        275                 280                 285

Ile Ala Asp Thr Ser Tyr Glu Val Pro Tyr Lys Phe Glu Ser Asp Glu
    290                 295                 300

Glu Val Tyr Gln Ser Val Asn Gly Phe Leu Asp Asn Ile Ser Ser Lys
305                 310                 315                 320

His Ile Val Glu Arg Leu Arg Lys Ile Gly Asp Asn Tyr Asn Gly Tyr
                325                 330                 335

Asn Leu Asp Lys Ile Tyr Ile Val Ser Lys Phe Tyr Glu Ser Val Ser
            340                 345                 350

Gln Lys Thr Tyr Arg Asp Trp Glu Thr Ile Asn Thr Ala Leu Glu Ile
        355                 360                 365

His Tyr Asn Asn Ile Leu Pro Gly Asn Gly Lys Ser Lys Ala Asp Lys
    370                 375                 380

Val Lys Lys Ala Val Lys Asn Asp Leu Gln Lys Ser Ile Thr Glu Ile
385                 390                 395                 400

Asn Glu Leu Val Ser Asn Tyr Lys Leu Cys Pro Asp Asp Asn Ile Lys
                405                 410                 415

Ala Glu Thr Tyr Ile His Glu Ile Ser His Ile Leu Asn Asn Phe Glu

            420                 425                 430

Ala Gln Glu Leu Lys Tyr Asn Pro Glu Ile His Leu Val Glu Ser Glu
        435                 440                 445

Leu Lys Ala Ser Glu Leu Lys Asn Val Leu Asp Val Ile Met Asn Ala
    450                 455                 460

Phe His Trp Cys Ser Val Phe Met Thr Glu Glu Leu Val Asp Lys Asp
465                 470                 475                 480

Asn Asn Phe Tyr Ala Glu Leu Glu Glu Ile Tyr Asp Glu Ile Tyr Thr
                485                 490                 495

Val Ile Ser Leu Tyr Asn Leu Val Arg Asn Tyr Val Thr Gln Lys Pro
            500                 505                 510

Tyr Ser Thr Lys Lys Ile Lys Leu Asn Phe Gly Ile Pro Thr Leu Ala
        515                 520                 525

Asp Gly Trp Ser Lys Ser Lys Glu Tyr Ser Asn Asn Ala Ile Ile Leu
    530                 535                 540

Met Arg Asp Asn Leu Tyr Tyr Leu Gly Ile Phe Asn Ala Lys Asn Lys
545                 550                 555                 560

Pro Asp Lys Lys Ile Ile Glu Gly Asn Thr Ser Glu Asn Lys Gly Asp
                565                 570                 575

Tyr Lys Lys Met Ile Tyr Asn Leu Leu Pro Gly Pro Asn Lys Met Ile
            580                 585                 590

Pro Lys Val Phe Leu Ser Ser Lys Thr Gly Val Glu Thr Tyr Lys Pro
        595                 600                 605

Ser Ala Tyr Ile Leu Glu Gly Tyr Lys Gln Asn Lys His Leu Lys Ser
    610                 615                 620

Ser Lys Asp Phe Asp Ile Thr Phe Cys His Asp Leu Ile Asp Tyr Phe
625                 630                 635                 640

Lys Asn Cys Ile Ala Ile His Pro Glu Trp Lys Asn Phe Gly Phe Asp
                645                 650                 655

Phe Ser Asp Thr Ser Thr Tyr Glu Asp Ile Ser Gly Phe Tyr Arg Glu
            660                 665                 670

Val Glu Leu Gln Gly Tyr Lys Ile Asp Trp Thr Tyr Ile Ser Glu Lys
        675                 680                 685

Asp Ile Asp Leu Leu Gln Glu Lys Gly Gln Leu Tyr Leu Phe Gln Ile
    690                 695                 700

Tyr Asn Lys Asp Phe Ser Lys Lys Ser Thr Gly Asn Asp Asn Leu His
705                 710                 715                 720

Thr Met Tyr Leu Lys Asn Leu Phe Ser Glu Glu Asn Leu Lys Asp Ile
                725                 730                 735

Val Leu Lys Leu Asn Gly Glu Ala Glu Ile Phe Phe Arg Lys Ser Ser
            740                 745                 750

Ile Lys Asn Pro Ile Ile His Lys Lys Gly Ser Ile Leu Val Asn Arg

755                 760                 765

Thr Tyr Glu Ala Glu Glu Lys Asp Gln Phe Gly Asn Ile Gln Ile Val
    770                 775                 780

Arg Lys Thr Ile Pro Glu Asn Ile Tyr Gln Glu Leu Tyr Lys Tyr Phe
785                 790                 795                 800

Asn Asp Lys Ser Asp Lys Glu Leu Ser Asp Glu Ala Ala Lys Leu Lys
                805                 810                 815

Asn Val Val Gly His His Glu Ala Ala Thr Asn Ile Val Lys Asp Tyr
            820                 825                 830

Arg Tyr Thr Tyr Asp Lys Tyr Phe Leu His Met Pro Ile Thr Ile Asn
        835                 840                 845

Phe Lys Ala Asn Lys Thr Ser Phe Ile Asn Asp Arg Ile Leu Gln Tyr
    850                 855                 860

Ile Ala Lys Glu Lys Asn Leu His Val Ile Gly Ile Asp Arg Gly Glu
865                 870                 875                 880

Arg Asn Leu Ile Tyr Val Ser Val Ile Asp Thr Cys Gly Asn Ile Val
                885                 890                 895

Glu Gln Lys Ser Phe Asn Ile Val Asn Gly Tyr Asp Tyr Gln Ile Lys
            900                 905                 910

Leu Lys Gln Gln Glu Gly Ala Arg Gln Ile Ala Arg Gln Glu Trp Lys
        915                 920                 925

Glu Ile Gly Lys Ile Lys Glu Ile Lys Glu Gly Tyr Leu Ser Leu Val
    930                 935                 940

Ile His Glu Ile Ser Lys Met Val Ile Lys Tyr Asn Ala Ile Ile Ala
945                 950                 955                 960

Met Glu Asp Leu Ser Tyr Gly Phe Lys Lys Gly Arg Phe Lys Val Glu
                965                 970                 975

Arg Gln Val Tyr Gln Lys Phe Glu Thr Met Leu Ile Asn Lys Leu Asn
            980                 985                 990

Tyr Leu Val Phe Lys Asp Ile Ser Ile Thr Glu Asn Gly Gly Leu Leu
        995                 1000                1005

Lys Gly Tyr Gln Leu Thr Tyr Ile Pro Asp Lys Leu Lys Asn Val Gly
    1010                1015                1020

His Gln Cys Gly Cys Ile Phe Tyr Val Pro Ala Ala Tyr Thr Ser Lys
1025                1030                1035                1040

Ile Asp Pro Thr Thr Gly Phe Val Asn Ile Phe Lys Phe Lys Asp Leu
                1045                1050                1055

Thr Val Asp Ala Lys Arg Glu Phe Ile Lys Lys Phe Asp Ser Ile Arg
            1060                1065                1070

Tyr Asp Ser Glu Lys Lys Leu Phe Cys Phe Thr Phe Asp Tyr Asn Asn
        1075                1080                1085

Phe Ile Thr Gln Asn Thr Val Met Ser Lys Ser Ser Trp Ser Val Tyr

1090                1095                1100

Thr Tyr Gly Val Arg Ile Lys Arg Arg Phe Val Asn Gly Arg Phe Ser
1105                1110                1115                1120

Asn Glu Ser Asp Thr Ile Asp Ile Thr Lys Asp Met Glu Lys Thr Leu
                1125                1130                1135

Glu Met Thr Asp Ile Asn Trp Arg Asp Gly His Asp Leu Arg Gln Asp
            1140                1145                1150

Ile Ile Asp Tyr Glu Ile Val Gln His Ile Phe Glu Ile Phe Arg Leu
        1155                1160                1165

Thr Val Gln Met Arg Asn Ser Leu Ser Glu Leu Glu Asp Arg Asp Tyr
    1170                1175                1180

Asp Arg Leu Ile Ser Pro Val Leu Asn Glu Asn Asn Ile Phe Tyr Asp
1185                1190                1195                1200

Ser Ala Lys Ala Gly Asp Ala Leu Pro Lys Asp Ala Asp Ala Asn Gly
                1205                1210                1215

Ala Tyr Cys Ile Ala Leu Lys Gly Leu Tyr Glu Ile Lys Gln Ile Thr
            1220                1225                1230

Glu Asn Trp Lys Glu Asp Gly Lys Phe Ser Arg Asp Lys Leu Lys Ile
        1235                1240                1245

Ser Asn Lys Asp Trp Phe Asp Phe Ile Gln Asn Lys Arg Tyr Leu
    1250                1255                1260

<210>    6
<211>    1275
<212>    PRT
<213>    Artificial Sequence

<220>
<223>    engineered mgCas12a-2(K930Q)

<400>    6
Met Gly Lys Asn Gln Asn Phe Gln Glu Phe Ile Gly Val Ser Pro Leu
1               5                   10                  15

Gln Lys Thr Leu Arg Asn Glu Leu Ile Pro Thr Glu Thr Thr Lys Lys
            20                  25                  30

Asn Ile Thr Gln Leu Asp Leu Leu Thr Glu Asp Glu Ile Arg Ala Gln
        35                  40                  45

Asn Arg Glu Lys Leu Lys Glu Met Met Asp Asp Tyr Tyr Arg Asn Val
    50                  55                  60

Ile Asp Ser Thr Leu His Val Gly Ile Ala Val Asp Trp Ser Tyr Leu
65                  70                  75                  80

Phe Ser Cys Met Arg Asn His Leu Arg Glu Asn Ser Lys Glu Ser Lys
                85                  90                  95

Arg Glu Leu Glu Arg Thr Gln Asp Ser Ile Arg Ser Gln Ile His Asn
        100                 105                 110

Lys Phe Ala Glu Arg Ala Asp Phe Lys Asp Met Phe Gly Ala Ser Ile
    115                 120                 125

Ile Thr Lys Leu Leu Pro Thr Tyr Ile Lys Gln Asn Ser Glu Tyr Ser
130                 135                 140

Glu Arg Tyr Asp Glu Ser Met Glu Ile Leu Lys Leu Tyr Gly Lys Phe
145                 150                 155                 160

Thr Thr Ser Leu Thr Asp Tyr Phe Glu Thr Arg Lys Asn Ile Phe Ser
                165                 170                 175

Lys Glu Lys Ile Ser Ser Ala Val Gly Tyr Arg Ile Val Glu Glu Asn
            180                 185                 190

Ala Glu Ile Phe Leu Gln Asn Gln Asn Ala Tyr Asp Arg Ile Cys Lys
        195                 200                 205

Ile Ala Gly Leu Asp Leu His Gly Leu Asp Asn Glu Ile Thr Ala Tyr
    210                 215                 220

Val Asp Gly Lys Thr Leu Lys Glu Val Cys Ser Asp Glu Gly Phe Ala
225                 230                 235                 240

Lys Ala Ile Thr Gln Glu Gly Ile Asp Arg Tyr Asn Glu Ala Ile Gly
                245                 250                 255

Ala Val Asn Gln Tyr Met Asn Leu Leu Cys Gln Lys Asn Lys Ala Leu
            260                 265                 270

Lys Pro Gly Gln Phe Lys Met Lys Arg Leu His Lys Gln Ile Leu Cys
        275                 280                 285

Lys Gly Thr Thr Ser Phe Asp Ile Pro Lys Lys Phe Glu Asn Asp Lys
    290                 295                 300

Gln Val Tyr Asp Ala Val Asn Ser Phe Thr Glu Ile Val Thr Lys Asn
305                 310                 315                 320

Asn Asp Leu Lys Arg Leu Leu Asn Ile Thr Gln Asn Ala Asn Asp Tyr
                325                 330                 335

Asp Met Asn Lys Ile Tyr Val Val Ala Asp Ala Tyr Ser Met Ile Ser
            340                 345                 350

Gln Phe Ile Ser Lys Lys Trp Asn Leu Ile Glu Glu Cys Leu Leu Asp
        355                 360                 365

Tyr Tyr Ser Asp Asn Leu Pro Gly Lys Gly Asn Ala Lys Glu Asn Lys
    370                 375                 380

Val Lys Lys Ala Val Lys Glu Glu Thr Tyr Arg Ser Val Ser Gln Leu
385                 390                 395                 400

Asn Glu Val Ile Glu Lys Tyr Tyr Val Glu Lys Thr Gly Gln Ser Val
                405                 410                 415

Trp Lys Val Glu Ser Tyr Ile Ser Ser Leu Ala Glu Met Ile Lys Leu
            420                 425                 430

Glu Leu Cys His Glu Ile Asp Asn Asp Glu Lys His Asn Leu Ile Glu
        435                 440                 445

Asp Asp Glu Lys Ile Ser Glu Ile Lys Glu Leu Leu Asp Met Tyr Met
    450                 455                 460

Asp Val Phe His Ile Ile Lys Val Phe Arg Val Asn Glu Val Leu Asn
465                 470                 475                 480

Phe Asp Glu Thr Phe Tyr Ser Glu Met Asp Glu Ile Tyr Gln Asp Met
                485                 490                 495

Gln Glu Ile Val Pro Leu Tyr Asn His Val Arg Asn Tyr Val Thr Gln
            500                 505                 510

Lys Pro Tyr Lys Gln Glu Lys Tyr Arg Leu Tyr Phe His Thr Pro Thr
        515                 520                 525

Leu Ala Asn Gly Trp Ser Lys Ser Lys Glu Tyr Asp Asn Asn Ala Ile
    530                 535                 540

Ile Leu Val Arg Glu Asp Lys Tyr Tyr Leu Gly Ile Leu Asn Ala Lys
545                 550                 555                 560

Lys Lys Pro Ser Lys Glu Ile Met Ala Gly Lys Glu Asp Cys Ser Glu
                565                 570                 575

His Ala Tyr Ala Lys Met Asn Tyr Tyr Leu Leu Pro Gly Ala Asn Lys
            580                 585                 590

Met Leu Pro Lys Val Phe Leu Ser Lys Lys Gly Ile Gln Asp Tyr His
        595                 600                 605

Pro Ser Ser Tyr Ile Val Glu Gly Tyr Asn Glu Lys Lys His Ile Lys
    610                 615                 620

Gly Ser Lys Asn Phe Asp Ile Arg Phe Cys Arg Asp Leu Ile Asp Tyr
625                 630                 635                 640

Phe Lys Glu Cys Ile Lys Lys His Pro Asp Trp Asn Lys Phe Asn Phe
                645                 650                 655

Glu Phe Ser Ala Thr Glu Thr Tyr Glu Asp Ile Ser Val Phe Tyr Arg
            660                 665                 670

Glu Val Glu Lys Gln Gly Tyr Arg Val Glu Trp Thr Tyr Ile Asn Ser
        675                 680                 685

Glu Asp Ile Gln Lys Leu Glu Glu Asp Gly Gln Leu Phe Leu Phe Gln
    690                 695                 700

Ile Tyr Asn Lys Asp Phe Ala Val Gly Ser Thr Gly Lys Pro Asn Leu
705                 710                 715                 720

His Thr Leu Tyr Leu Lys Asn Leu Phe Ser Glu Glu Asn Leu Arg Asp
                725                 730                 735

Ile Val Leu Lys Leu Asn Gly Glu Ala Glu Ile Phe Phe Arg Lys Ser
            740                 745                 750

Ser Val Gln Lys Pro Val Ile His Lys Cys Gly Ser Ile Leu Val Asn
        755                 760                 765

Arg Thr Tyr Glu Ile Thr Glu Ser Gly Thr Thr Arg Val Gln Ser Ile
    770                 775                 780

Pro Glu Ser Glu Tyr Met Glu Leu Tyr Arg Tyr Phe Asn Ser Glu Lys
785                 790                 795                 800

Gln Ile Glu Leu Ser Asp Glu Ala Lys Lys Tyr Leu Asp Lys Val Gln
                805                 810                 815

Cys Asn Lys Ala Lys Thr Asp Ile Val Lys Asp Tyr Arg Tyr Thr Met
            820                 825                 830

Asp Lys Phe Phe Ile His Leu Pro Ile Thr Ile Asn Phe Lys Val Asp
        835                 840                 845

Lys Gly Asn Asn Val Asn Ala Ile Ala Gln Gln Tyr Ile Ala Gly Arg
    850                 855                 860

Lys Asp Leu His Val Ile Gly Ile Asp Arg Gly Glu Arg Asn Leu Ile
865                 870                 875                 880

Tyr Val Ser Val Ile Asp Met Tyr Gly Arg Ile Leu Glu Gln Lys Ser
                885                 890                 895

Phe Asn Leu Val Glu Gln Val Ser Ser Gln Gly Thr Lys Arg Tyr Tyr
            900                 905                 910

Asp Tyr Lys Glu Lys Leu Gln Asn Arg Glu Glu Glu Arg Asp Lys Ala
        915                 920                 925

Arg Gln Ser Trp Lys Thr Ile Gly Lys Ile Lys Glu Leu Lys Glu Gly
    930                 935                 940

Tyr Leu Ser Ser Val Ile His Glu Ile Ala Gln Met Val Val Lys Tyr
945                 950                 955                 960

Asn Ala Ile Ile Ala Met Glu Asp Leu Asn Tyr Gly Phe Lys Arg Gly
                965                 970                 975

Arg Phe Lys Val Glu Arg Gln Val Tyr Gln Lys Phe Glu Thr Met Leu
            980                 985                 990

Ile Ser Lys Leu Asn Tyr Leu Ala Asp Lys Ser Gln Ala Val Asp Glu
        995                 1000                1005

Pro Gly Gly Ile Leu Arg Gly Tyr Gln Met Thr Tyr Val Pro Asp Asn
    1010                1015                1020

Ile Lys Asn Val Gly Arg Gln Cys Gly Ile Ile Phe Tyr Val Pro Ala
1025                1030                1035                1040

Ala Tyr Thr Ser Lys Ile Asp Pro Thr Thr Gly Phe Ile Asn Ala Phe
                1045                1050                1055

Lys Arg Asp Val Val Ser Thr Asn Asp Ala Lys Glu Asn Phe Leu Met
            1060                1065                1070

Lys Phe Asp Ser Ile Gln Tyr Asp Ile Glu Lys Gly Leu Phe Lys Phe
        1075                1080                1085

Ser Phe Asp Tyr Lys Asn Phe Ala Thr His Lys Leu Thr Leu Ala Lys
    1090                1095                1100

Thr Lys Trp Asp Val Tyr Thr Asn Gly Thr Arg Ile Gln Asn Met Lys
1105                1110                1115                1120

Val Glu Gly His Trp Leu Ser Met Glu Val Glu Leu Thr Thr Lys Met
                1125                1130                1135

Lys Glu Leu Leu Asp Asp Ser His Ile Pro Tyr Glu Glu Gly Gln Asn
            1140                1145                1150

Ile Leu Asp Asp Leu Arg Glu Met Lys Asp Ile Thr Thr Ile Val Asn
        1155                1160                1165

Gly Ile Leu Glu Ile Phe Trp Leu Thr Val Gln Leu Arg Asn Ser Arg
    1170                1175                1180

Ile Asp Asn Pro Asp Tyr Asp Arg Ile Ile Ser Pro Val Leu Asn Lys
1185                1190                1195                1200

Asn Gly Glu Phe Phe Asp Ser Asp Glu Tyr Asn Ser Tyr Ile Asp Ala
                1205                1210                1215

Gln Lys Ala Pro Leu Pro Ile Asp Ala Asp Ala Asn Gly Ala Phe Cys
            1220                1225                1230

Ile Ala Leu Lys Gly Met Tyr Thr Ala Asn Gln Ile Lys Glu Asn Trp
        1235                1240                1245

Val Glu Gly Glu Lys Leu Pro Ala Asp Cys Leu Lys Ile Glu His Ala
    1250                1255                1260

Ser Trp Leu Ala Phe Met Gln Gly Glu Arg Gly
1265                1270                1275


<210>    7
<211>    3789
<212>    DNA
<213>    Artificial Sequence

<220>
<223>    human codon optimized engineered mgCas12a-1


<400>    7
atgaacaatg gcaccaacaa tttccagaac tttatcggaa ttagcagtct gcaaaagact      60

ctccggaatg cccttatacc caccgagaca acccagcagt tcatcgtgaa aaacgggatt     120

atcaaggaag acgagctgcg cggcgaaaat cggcaaattt tgaaagatat aatggacgat     180

tattaccgcg gttttatctc tgagactctg agctccattg acgatatcga ctggacctca     240

ctcttcgaaa agatggagat tcagcttaaa aacggcgata ataaggacac actgataaaa     300

gaacaggctg agaagcggaa agccatctat aagaaatttg cagatgacga tcgcttcaag     360

aacatgttta gcgccaaatt gattagtgac atcctgccgg aattcgttat tcacaataac     420

aattactctg ctagcgagaa ggaagagaaa acccaagtca taaagctctt ttcccggttc     480

gccacttcat ttaaagatta tttcaagaac cgcgcaaatt gctttagcgc cgacgatatc     540

agttctagct cctgtcatcg gattgtgaac gacaatgctg aaatcttctt ttcaaacgcc     600

cttgtatacc gccggattgt gaaaaatctg agcaacgatg acataaataa gatcagtgga 660

gatattaaag actctttgaa ggagatgagc ctggaagaga tctattccta cgaaaaatat 720

ggggagttca ttacccagga aggcatatca ttttacaacg atatctgcgg taaggttaat 780

agcttcatga acctctattg tcagaaaaat aaggagaaca aaaatcttta caagctgcgc 840

aaattgcaca agcaaattct gtgcatcgca gacacaagtt atgaagtccc ttacaaattt 900

gagtctgatg aagaggtgta tcagagcgta aacggcttcc tcgacaatat ttcctcaaag 960

catatagtgg aacggcttcg caaaatcgga gataactaca atgggtataa cctggacaag 1020

atttacatcg ttagcaaatt ttatgagagt gtctctcaga agacctaccg ggattgggaa 1080

actattaata ccgccttgga gatacactat aacaatatcc tgcccggcaa cggtaaaagc 1140

aaggctgaca aagtgaagaa agccgtaaag aatgatctcc aaaaatccat tacagaaatc 1200

aacgagcttg tgtcaaatta caagctgtgt ccggacgata acattaaagc agaaacctat 1260

atacatgaga tcagccacat tttgaataac ttcgaagccc aggagctgaa gtacaatcca 1320

gaaatccatc tcgttgagag tgaacttaaa gcttctgagc tgaagaacgt cttggacgtg 1380

attatgaatg cctttcactg gtgcagcgta ttcatgactg aagagctggt ggataaagac 1440

aacaattttt atgcagaact cgaggaaata tacgatgaga tctataccgt tatttccctt 1500

tacaacctgg tccgcaatta tgtgacacag aagccctact caaccaaaaa gatcaaattg 1560

aacttcggca ttccgactct ggccgacgga tggagcaaga gtaaagaata ttctaataac 1620

gctataatcc tcatgcggga taatctttac tatctgggga tttttaacgc caagaataaa 1680

cctgacaaga aaatcattga gggcaacacc agcgaaaata agggtgatta caaaaagatg 1740

atatataact tgctgcccgg cccgaataaa atgatcccaa aggtattcct ctcctcaaaa 1800

acaggagtgg agacctacaa gcccagcgca tatattcttg aagggtacaa acaaaacaag 1860

catctgaaaa gttctaagga ctttgatatc actttctgtc acgacttgat tgattatttt 1920

aaaaattgca tagccatcca tccggagtgg aagaacttcg gctttgactt cagcgatacc 1980

tccacatacg aagacatttc aggtttttat cgcgaggttg aactgcaggg ctacaaaatc 2040

gattggacct atattagcga gaaggacata gatctccttc aggaaaaagg acaactgtac 2100

ttgttccaga tctataataa ggactttagt aaaaagtcta ctgggaacga taatctgcac 2160

accatgtacc tcaaaaacct tttcagcgag gaaaatctga aggacattgt cttgaaactg 2220

aacggcgagg ctgaaatctt tttccggaag tcctcaatta aaaatcctat aatccataag 2280

aaaggtagca ttctcgtgaa ccgcacatat gaggccgaag agaaggatca gtttggcaat 2340

atccaaattg tacggaaaac catacccgaa aacatctacc aggagcttta taagtacttc 2400

aatgacaaaa gtgataagga actgtctgac gaggcagcca aattgaagaa cgtggttgga 2460

caccatgaag ctgccactaa tattgtcaaa gattatcgct acacctatga caagtacttt 2520

ctgcacatgc cgatcacaat taacttcaaa gcaaataaga ccagctttat aaacgatcgg 2580

attctccagt atattgccaa agagaagaat cttcatgtga tcgggattga ccgcggcgaa 2640

cggaacctga tatacgtatc cgtgatcgat acttgtggta atattgttga gcaaaaatca 2700

ttcaacatcg tcaatggcta tgactaccag attaagttga aacagcaaga aggagctcgc 2760

cagatagccc ggcaggagtg gaaggaaatc gggaaaatta aggagatcaa agaaggctat 2820

ctgagcctcg tgattcacga gataagtaag atggtaatca aatacaacgc aattatcgcc 2880

atggaagatc tttcttatgg ttttaagaaa ggccgcttca aggtggagcg gcaagtttac 2940

cagaaatttg aaaccatgct gattaataag ttgaactatc tggtcttcaa agacataagc 3000

atcacagaga atggagggct ccttaagggc taccagctga cctatattcc agataaattg 3060

aagaacgtgg gtcatcaatg cggctgtatc ttttacgtac ccgctgccta tacttccaaa 3120

attgacccga ccacaggatt cgtgaatata tttaagttca aagatctgac cgttgacgca 3180

aagcgcgaat ttatcaaaaa gttcgattca attcggtacg acagcgagaa aaagctcttt 3240

tgcttcactt ttgattataa caatttcatc acccagaaca cagtcatgag taaatctagc 3300

tggtccgtgt acacctatgg ggtacgcatt aagcggcgct ttgtgaatgg ccggttctca 3360

aacgaaagcg acactataga tatcaccaaa gacatggaga agacacttga aatgaccgat 3420

attaattggc gcgacggtca cgatctgcgg caggacatca ttgattacga gatagttcaa 3480

catatctttg aaattttccg cttgactgtc cagatgcgga acagtctgtc tgagctcgaa 3540

gaccgcgatt atgaccggct tatcagccct gtgctgaatg agaacaatat tttttacgat 3600

tccgccaaag ctggcgacgc cttgcccaag gatgcagacg ccaacggagc ttattgtata 3660

gccctgaaag ggctctacga aatcaagcag attaccgaga attggaaaga agatggcaag 3720

ttctcacgcg acaaacttaa gatcagcaac aaagattggt ttgacttcat tcaaaataag 3780

cggtatctg 3789

<210> 8
<211> 3825
<212> DNA
<213> Artificial Sequence

<220>
<223> human codon optimized engineered mgCas12a-2

<400> 8
atgggcaaaa accaaaattt ccaagaattt atcggagtga gccccctgca gaagaccctc 60

cggaacgagc ttattccgac tgagaccaca aagaaaaata taacccagct ggacttgctg 120

actgaagatg agatccgcgc ccagaaccgg gaaaagctca aagagatgat ggacgattat 180

taccgcaatg ttattgacag tacccttcac gtcgggatcg ctgtggattg gtcttatctg 240

ttcagctgca tgcggaacca tttgcgcgaa aattccaagg agtcaaaacg ggaactggag 300

cgcacacagg acagcattcg gagtcagata cacaacaagt ttgccgaacg cgcagatttc 360

aaagacatgt ttggcgcctc tatcattacc aagctccttc ctacttacat caaacaaaat 420

agcgagtatt ccgaacggta cgatgagtca atggaaattc tgaagttgta tggtaaattc 480

accacaagcc tgaccgacta ctttgagact cgcaagaaca tattcagtaa agaaaagatc 540

tctagcgctg taggctatcg gattgtggag gaaaatgccg agatctttct ccagaaccag 600

aatgcatacg atcgcatttg taaaatagcc ggacttgacc tgcatgggtt ggataacgaa 660

atcaccgctt atgttgacgg caagacactg aaagaggtct gctccgatga aggtttcgcc 720

aaggcaatta cccaagaggg catcgaccgg tacaatgaag ccattggagc tgtgaaccag 780

tatatgaatc tcctttgtca gaaaaacaag gccctgaaac ccgggcaatt taagatgaaa 840

cgcttgcaca agcagatact gtgcaaaggc actacctcat tcgatatccc gaagaaattt 900

gagaatgaca agcaggtata cgatgcagtg aacagcttca cagaaattgt taccaaaaat 960

aacgacctca agcggcttct gaatatcact caaaacgcca atgattatga catgaacaaa 1020

atttacgtcg tggctgatgc ctatagtatg atatctcagt ttatcagcaa gaaatggaat 1080

ttgattgagg aatgtctgct cgactactat tccgataacc ttccaggtaa gggcaatgca 1140

aaagagaaca aggtaaaaaa ggccgtgaaa gaagagacct accgctcagt tagccagctg 1200

aatgaagtca tcgagaagta ttacgtggaa aaaacaggac aaagtgtatg gaaggtggag 1260

tcttatatta gctccttggc tgaaatgata aaactggagc tctgccatga aatcgacaac 1320

gatgagaagc acaatcttat tgaagacgat gagaaaatct cagaaattaa ggagctgttg 1380

gacatgtaca tggatgtttt ccatataatc aaagtctttc gggtgaacga agtactgaat 1440

ttcgacgaga ccttttatag cgaaatggat gagatttacc aggacatgca ggaaatcgtg 1500

cccctctata accacgttcg caattacgtc actcaaaagc cgtataaaca ggagaagtac 1560

cggctttatt tccatacccc tacactggcc aacgggtgga gtaaatctaa ggaatacgat 1620

aataacgcaa ttatattggt gcgcgaggac aaatattacc tgggcatcct caatgccaag 1680

aaaaagccca gcaaagaaat tatggctggt aaggaggatt gttccgaaca cgcctatgca 1740

aaaatgaact actatcttct gccgggcgcc aataagatgt tgccaaaagt atttctgtca 1800

aagaaaggaa tccaggacta ccatcccagc agttatattg tggaggggta caacgaaaag 1860

aaacacataa agggctctaa aaatttcgat atccggtttt gccgcgacct cattgattat 1920

ttcaaggagt gtatcaaaaa gcatccggac tggaacaaat ttaatttcga atttagcgct 1980

accgagactt acgaagatat ttccgttttc tatcgggagg tcgaaaagca aggttaccgc 2040

gtggagtgga cctatataaa ctcagaagac atccagaaac ttgaggaaga tggccagctg 2100

tttttgttcc aaatttacaa taaggacttt gccgtaggaa gcacagggaa acctaacctg 2160

cacaccctct atcttaagaa tctgttcagt gaggaaaact tgcgggatat cgtgctgaaa 2220

ctcaatggcg aggcagaaat ttttttccgc aagtctagcg ttcagaaacc cgtcatacat 2280

aagtgcggtt ccatccttgt gaaccggact tacgagatta ccgaatcagg cacaacccgc 2340

gtacagagca tcccggagag tgaatatatg gagctgtacc ggtattttaa ttctgaaaaa 2400

caaattgagt tgagcgacga agccaagaaa tacctggata aggtgcagtg taacaaagct 2460

aagactgaca tagttaaaga ttatcgctac accatggaca agttctttat ccacctccca 2520

attacaatca atttcaaagt cgataaggga aacaatgtga acgccattgc acagcaatat 2580

atagccgggc ggaaagacct tcatgtaatc ggcattgatc gcggtgagcg gaatctgatc 2640

tacgtgtccg ttattgacat gtatggccgc atattggaac agaagtcatt taacctggtc 2700

gagcaggtga gcagtcaagg aaccaaacgg tactatgatt acaaggaaaa actccagaat 2760

cgcgaggaag agcgggacaa ggctcgccag tcttggaaaa ctatcgggaa gattaaagaa 2820

cttaaggagg gctatctgag ctccgtaatc cacgaaattg cccaaatggt ggttaaatac 2880

aacgcaataa tcgccatgga ggatttgaat tatggtttca agcggggccg ctttaaagtc 2940

gaacggcagg tgtaccagaa gttcgagacc atgctgattt caaaactcaa ctatcttgct 3000

gacaagagcc aagccgtaga tgaacccgga gggattctgc gcggctacca gatgacatat 3060

gtgccggaca atattaaaaa cgttggtcgg cagtgcggca taatctttta cgtccctgca 3120

gcctatacca gtaagattga tcccactacc ggattcatca atgcttttaa acgcgacgtg 3180

gtatctacaa acgatgccaa ggagaatttc ttgatgaaat ttgacagcat tcaatacgat 3240

atagaaaagg ggctgttcaa attttccttc gactataaga actttgcaac ccataaactc 3300

actcttgcca agaccaaatg ggatgtgtac acaaatggca cccggattca gaacatgaag 3360

gttgagggtc actggctgtc aatggaagtc gagttgacta ccaaaatgaa ggaactgctc 3420

gacgatagcc atattccgta tgaggaaggc cagaatatcc ttgacgatct gcgcgagatg 3480

aaagacatta caaccatagt gaacggaatc ttggaaattt tctggctgac tgtacaactc 3540

cggaatagtc gcatcgataa cccagactac gatcggatta tatctcccgt gcttaataag 3600

aacggggagt ttttcgacag cgatgaatat aattcctaca tcgacgctca gaaagccccg 3660

ctgcctattg atgcagacgc caacggcgct ttttgtatcg ccttgaaggg tatgtatacc 3720

gcaaatcaga ttaaagagaa ctgggttgaa ggcgagaagc tgcccgccga ttgcctcaaa 3780

atagaacacg cttcatggct tgccttcatg caaggagagc gcggg 3825

<210> 9
<211> 1307
<212> PRT

<213>    Artificial Sequence

<220>
<223>    AsCas12a

<400>    9
Met Thr Gln Phe Glu Gly Phe Thr Asn Leu Tyr Gln Val Ser Lys Thr
1               5                   10                  15

Leu Arg Phe Glu Leu Ile Pro Gln Gly Lys Thr Leu Lys His Ile Gln
            20                  25                  30

Glu Gln Gly Phe Ile Glu Glu Asp Lys Ala Arg Asn Asp His Tyr Lys
        35                  40                  45

Glu Leu Lys Pro Ile Ile Asp Arg Ile Tyr Lys Thr Tyr Ala Asp Gln
    50                  55                  60

Cys Leu Gln Leu Val Gln Leu Asp Trp Glu Asn Leu Ser Ala Ala Ile
65                  70                  75                  80

Asp Ser Tyr Arg Lys Glu Lys Thr Glu Glu Thr Arg Asn Ala Leu Ile
                85                  90                  95

Glu Glu Gln Ala Thr Tyr Arg Asn Ala Ile His Asp Tyr Phe Ile Gly
            100                 105                 110

Arg Thr Asp Asn Leu Thr Asp Ala Ile Asn Lys Arg His Ala Glu Ile
        115                 120                 125

Tyr Lys Gly Leu Phe Lys Ala Glu Leu Phe Asn Gly Lys Val Leu Lys
    130                 135                 140

Gln Leu Gly Thr Val Thr Thr Thr Glu His Glu Asn Ala Leu Leu Arg
145                 150                 155                 160

Ser Phe Asp Lys Phe Thr Thr Tyr Phe Ser Gly Phe Tyr Glu Asn Arg
                165                 170                 175

Lys Asn Val Phe Ser Ala Glu Asp Ile Ser Thr Ala Ile Pro His Arg
            180                 185                 190

Ile Val Gln Asp Asn Phe Pro Lys Phe Lys Glu Asn Cys His Ile Phe
        195                 200                 205

Thr Arg Leu Ile Thr Ala Val Pro Ser Leu Arg Glu His Phe Glu Asn
    210                 215                 220

Val Lys Lys Ala Ile Gly Ile Phe Val Ser Thr Ser Ile Glu Glu Val
225                 230                 235                 240

Phe Ser Phe Pro Phe Tyr Asn Gln Leu Leu Thr Gln Thr Gln Ile Asp
                245                 250                 255

Leu Tyr Asn Gln Leu Leu Gly Gly Ile Ser Arg Glu Ala Gly Thr Glu
            260                 265                 270

Lys Ile Lys Gly Leu Asn Glu Val Leu Asn Leu Ala Ile Gln Lys Asn
        275                 280                 285

Asp Glu Thr Ala His Ile Ile Ala Ser Leu Pro His Arg Phe Ile Pro
    290                 295                 300

Leu Phe Lys Gln Ile Leu Ser Asp Arg Asn Thr Leu Ser Phe Ile Leu
305                 310                 315                 320

Glu Glu Phe Lys Ser Asp Glu Glu Val Ile Gln Ser Phe Cys Lys Tyr
                325                 330                 335

Lys Thr Leu Leu Arg Asn Glu Asn Val Leu Glu Thr Ala Glu Ala Leu
            340                 345                 350

Phe Asn Glu Leu Asn Ser Ile Asp Leu Thr His Ile Phe Ile Ser His
        355                 360                 365

Lys Lys Leu Glu Thr Ile Ser Ser Ala Leu Cys Asp His Trp Asp Thr
    370                 375                 380

Leu Arg Asn Ala Leu Tyr Glu Arg Arg Ile Ser Glu Leu Thr Gly Lys
385                 390                 395                 400

Ile Thr Lys Ser Ala Lys Glu Lys Val Gln Arg Ser Leu Lys His Glu
                405                 410                 415

Asp Ile Asn Leu Gln Glu Ile Ile Ser Ala Ala Gly Lys Glu Leu Ser
            420                 425                 430

Glu Ala Phe Lys Gln Lys Thr Ser Glu Ile Leu Ser His Ala His Ala
        435                 440                 445

Ala Leu Asp Gln Pro Leu Pro Thr Thr Leu Lys Lys Gln Glu Glu Lys
    450                 455                 460

Glu Ile Leu Lys Ser Gln Leu Asp Ser Leu Leu Gly Leu Tyr His Leu
465                 470                 475                 480

Leu Asp Trp Phe Ala Val Asp Glu Ser Asn Glu Val Asp Pro Glu Phe
                485                 490                 495

Ser Ala Arg Leu Thr Gly Ile Lys Leu Glu Met Glu Pro Ser Leu Ser
            500                 505                 510

Phe Tyr Asn Lys Ala Arg Asn Tyr Ala Thr Lys Lys Pro Tyr Ser Val
        515                 520                 525

Glu Lys Phe Lys Leu Asn Phe Gln Met Pro Thr Leu Ala Ser Gly Trp
    530                 535                 540

Asp Val Asn Lys Glu Lys Asn Asn Gly Ala Ile Leu Phe Val Lys Asn
545                 550                 555                 560

Gly Leu Tyr Tyr Leu Gly Ile Met Pro Lys Gln Lys Gly Arg Tyr Lys
                565                 570                 575

Ala Leu Ser Phe Glu Pro Thr Glu Lys Thr Ser Glu Gly Phe Asp Lys
            580                 585                 590

Met Tyr Tyr Asp Tyr Phe Pro Asp Ala Ala Lys Met Ile Pro Lys Cys
        595                 600                 605

Ser Thr Gln Leu Lys Ala Val Thr Ala His Phe Gln Thr His Thr Thr
    610                 615                 620

Pro Ile Leu Leu Ser Asn Asn Phe Ile Glu Pro Leu Glu Ile Thr Lys
625                 630                 635                 640

Glu Ile Tyr Asp Leu Asn Asn Pro Glu Lys Glu Pro Lys Lys Phe Gln
                645                 650                 655

Thr Ala Tyr Ala Lys Lys Thr Gly Asp Gln Lys Gly Tyr Arg Glu Ala
            660                 665                 670

Leu Cys Lys Trp Ile Asp Phe Thr Arg Asp Phe Leu Ser Lys Tyr Thr
        675                 680                 685

Lys Thr Thr Ser Ile Asp Leu Ser Ser Leu Arg Pro Ser Ser Gln Tyr
    690                 695                 700

Lys Asp Leu Gly Glu Tyr Tyr Ala Glu Leu Asn Pro Leu Leu Tyr His
705                 710                 715                 720

Ile Ser Phe Gln Arg Ile Ala Glu Lys Glu Ile Met Asp Ala Val Glu
                725                 730                 735

Thr Gly Lys Leu Tyr Leu Phe Gln Ile Tyr Asn Lys Asp Phe Ala Lys
            740                 745                 750

Gly His His Gly Lys Pro Asn Leu His Thr Leu Tyr Trp Thr Gly Leu
        755                 760                 765

Phe Ser Pro Glu Asn Leu Ala Lys Thr Ser Ile Lys Leu Asn Gly Gln
    770                 775                 780

Ala Glu Leu Phe Tyr Arg Pro Lys Ser Arg Met Lys Arg Met Ala His
785                 790                 795                 800

Arg Leu Gly Glu Lys Met Leu Asn Lys Lys Leu Lys Asp Gln Lys Thr
                805                 810                 815

Pro Ile Pro Asp Thr Leu Tyr Gln Glu Leu Tyr Asp Tyr Val Asn His
            820                 825                 830

Arg Leu Ser His Asp Leu Ser Asp Glu Ala Arg Ala Leu Leu Pro Asn
        835                 840                 845

Val Ile Thr Lys Glu Val Ser His Glu Ile Ile Lys Asp Arg Arg Phe
    850                 855                 860

Thr Ser Asp Lys Phe Phe Phe His Val Pro Ile Thr Leu Asn Tyr Gln
865                 870                 875                 880

Ala Ala Asn Ser Pro Ser Lys Phe Asn Gln Arg Val Asn Ala Tyr Leu
                885                 890                 895

Lys Glu His Pro Glu Thr Pro Ile Ile Gly Ile Asp Arg Gly Glu Arg
            900                 905                 910

Asn Leu Ile Tyr Ile Thr Val Ile Asp Ser Thr Gly Lys Ile Leu Glu
        915                 920                 925

Gln Arg Ser Leu Asn Thr Ile Gln Gln Phe Asp Tyr Gln Lys Lys Leu
    930                 935                 940

Asp Asn Arg Glu Lys Glu Arg Val Ala Ala Arg Gln Ala Trp Ser Val
945                 950                 955                 960

Val Gly Thr Ile Lys Asp Leu Lys Gln Gly Tyr Leu Ser Gln Val Ile
                965                 970                 975

His Glu Ile Val Asp Leu Met Ile His Tyr Gln Ala Val Val Val Leu
            980                 985                 990

Glu Asn Leu Asn Phe Gly Phe Lys Ser Lys Arg Thr Gly Ile Ala Glu
        995                 1000                1005

Lys Ala Val Tyr Gln Gln Phe Glu Lys Met Leu Ile Asp Lys Leu Asn
    1010                1015                1020

Cys Leu Val Leu Lys Asp Tyr Pro Ala Glu Lys Val Gly Gly Val Leu
1025                1030                1035                1040

Asn Pro Tyr Gln Leu Thr Asp Gln Phe Thr Ser Phe Ala Lys Met Gly
                1045                1050                1055

Thr Gln Ser Gly Phe Leu Phe Tyr Val Pro Ala Pro Tyr Thr Ser Lys
            1060                1065                1070

Ile Asp Pro Leu Thr Gly Phe Val Asp Pro Phe Val Trp Lys Thr Ile
        1075                1080                1085

Lys Asn His Glu Ser Arg Lys His Phe Leu Glu Gly Phe Asp Phe Leu
    1090                1095                1100

His Tyr Asp Val Lys Thr Gly Asp Phe Ile Leu His Phe Lys Met Asn
1105                1110                1115                1120

Arg Asn Leu Ser Phe Gln Arg Gly Leu Pro Gly Phe Met Pro Ala Trp
                1125                1130                1135

Asp Ile Val Phe Glu Lys Asn Glu Thr Gln Phe Asp Ala Lys Gly Thr
            1140                1145                1150

Pro Phe Ile Ala Gly Lys Arg Ile Val Pro Val Ile Glu Asn His Arg
        1155                1160                1165

Phe Thr Gly Arg Tyr Arg Asp Leu Tyr Pro Ala Asn Glu Leu Ile Ala
    1170                1175                1180

Leu Leu Glu Glu Lys Gly Ile Val Phe Arg Asp Gly Ser Asn Ile Leu
1185                1190                1195                1200

Pro Lys Leu Leu Glu Asn Asp Asp Ser His Ala Ile Asp Thr Met Val
                1205                1210                1215

Ala Leu Ile Arg Ser Val Leu Gln Met Arg Asn Ser Asn Ala Ala Thr
            1220                1225                1230

Gly Glu Asp Tyr Ile Asn Ser Pro Val Arg Asp Leu Asn Gly Val Cys
        1235                1240                1245

Phe Asp Ser Arg Phe Gln Asn Pro Glu Trp Pro Met Asp Ala Asp Ala
    1250                1255                1260

Asn Gly Ala Tyr His Ile Ala Leu Lys Gly Gln Leu Leu Leu Asn His
1265                1270                1275                1280

Leu Lys Glu Ser Lys Asp Leu Lys Leu Gln Asn Gly Ile Ser Asn Gln
                1285                1290                1295

Asp Trp Leu Ala Tyr Ile Gln Glu Leu Arg Asn
            1300                1305

<210>    10
<211>    1228
<212>    PRT
<213>    Artificial Sequence

<220>
<223>    LbCas12a


<400>    10
Ala Ala Ser Lys Leu Glu Lys Phe Thr Asn Cys Tyr Ser Leu Ser Lys
1               5                   10                  15

Thr Leu Arg Phe Lys Ala Ile Pro Val Gly Lys Thr Gln Glu Asn Ile
            20                  25                  30

Asp Asn Lys Arg Leu Leu Val Glu Asp Glu Lys Arg Ala Glu Asp Tyr
        35                  40                  45

Lys Gly Val Lys Lys Leu Leu Asp Arg Tyr Tyr Leu Ser Phe Ile Asn
    50                  55                  60

Asp Val Leu His Ser Ile Lys Leu Lys Asn Leu Asn Asn Tyr Ile Ser
65                  70                  75                  80

Leu Phe Arg Lys Lys Thr Arg Thr Glu Lys Glu Asn Lys Glu Leu Glu
                85                  90                  95

Asn Leu Glu Ile Asn Leu Arg Lys Glu Ile Ala Lys Ala Phe Lys Gly
            100                 105                 110

Ala Ala Gly Tyr Lys Ser Leu Phe Lys Lys Asp Ile Ile Glu Thr Ile
        115                 120                 125

Leu Pro Glu Ala Ala Asp Asp Lys Asp Glu Ile Ala Leu Val Asn Ser
    130                 135                 140

Phe Asn Gly Phe Thr Thr Ala Phe Thr Gly Phe Phe Asp Asn Arg Glu
145                 150                 155                 160

Asn Xaa Phe Ser Glu Glu Ala Lys Ser Thr Ser Ile Ala Phe Arg Cys
                165                 170                 175

Ile Asn Glu Asn Leu Thr Arg Tyr Ile Ser Asn Xaa Asp Ile Phe Glu
            180                 185                 190

Lys Val Asp Ala Ile Phe Asp Lys His Glu Val Gln Glu Ile Lys Glu
        195                 200                 205

Lys Ile Leu Asn Ser Asp Tyr Asp Val Glu Asp Phe Phe Glu Gly Glu
    210                 215                 220

Phe Phe Asn Phe Val Leu Thr Gln Glu Gly Ile Asp Val Tyr Asn Ala
225                 230                 235                 240

Ile Ile Gly Gly Phe Val Thr Glu Ser Gly Glu Lys Ile Lys Gly Leu
                245                 250                 255

Asn Glu Tyr Ile Asn Leu Tyr Asn Ala Lys Thr Lys Gln Ala Leu Pro
            260                 265                 270

Lys Phe Lys Pro Leu Tyr Lys Gln Val Leu Ser Asp Arg Glu Ser Leu
        275                 280                 285

Ser Phe Tyr Gly Glu Gly Tyr Thr Ser Asp Glu Glu Val Leu Glu Val
    290                 295                 300

Phe Arg Asn Thr Leu Asn Lys Asn Ser Glu Ile Phe Ser Ser Ile Lys
305                 310                 315                 320

Lys Leu Glu Lys Leu Phe Lys Asn Phe Asp Glu Tyr Ser Ser Ala Gly
                325                 330                 335

Ile Phe Val Lys Asn Gly Pro Ala Ile Ser Thr Ile Ser Lys Asp Ile
            340                 345                 350

Phe Gly Glu Trp Asn Leu Ile Arg Asp Lys Trp Asn Ala Glu Tyr Asp
        355                 360                 365

Asp Ile His Leu Lys Lys Lys Ala Val Val Thr Glu Lys Tyr Glu Asp
    370                 375                 380

Asp Arg Arg Lys Ser Phe Lys Lys Ile Gly Ser Phe Ser Leu Glu Gln
385                 390                 395                 400

Leu Gln Glu Tyr Ala Asp Ala Asp Leu Ser Val Val Glu Lys Leu Lys
                405                 410                 415

Glu Ile Ile Ile Gln Lys Val Asp Glu Ile Tyr Lys Val Tyr Gly Ser
            420                 425                 430

Ser Glu Lys Leu Phe Asp Ala Asp Phe Val Leu Glu Lys Ser Leu Lys
        435                 440                 445

Lys Asn Asp Ala Val Val Ala Ile Xaa Lys Asp Leu Leu Asp Ser Val
    450                 455                 460

Lys Ser Phe Glu Asn Tyr Ile Lys Ala Phe Phe Gly Glu Gly Lys Glu
465                 470                 475                 480

Thr Asn Arg Asp Glu Ser Phe Tyr Gly Asp Phe Val Leu Ala Tyr Asp
                485                 490                 495

Ile Leu Leu Lys Val Asp His Ile Tyr Asp Ala Ile Arg Asn Tyr Val
            500                 505                 510

Thr Gln Lys Pro Tyr Ser Lys Asp Lys Phe Lys Leu Tyr Phe Gln Asn
        515                 520                 525

Pro Gln Phe Xaa Gly Gly Trp Asp Lys Asp Lys Glu Thr Asp Tyr Arg
    530                 535                 540

Ala Thr Ile Leu Arg Tyr Gly Ser Lys Tyr Tyr Leu Ala Ile Xaa Asp
545                 550                 555                 560

Lys Lys Tyr Ala Lys Cys Leu Gln Lys Ile Asp Lys Asp Asp Val Asn
                565                 570                 575

Gly Asn Tyr Glu Lys Ile Asn Tyr Lys Leu Leu Pro Gly Pro Asn Lys
            580                 585                 590

Xaa Leu Pro Lys Val Phe Phe Ser Lys Lys Trp Xaa Ala Tyr Tyr Asn
        595                 600                 605

Pro Ser Glu Asp Ile Gln Lys Ile Tyr Lys Asn Gly Thr Phe Lys Lys
    610                 615                 620

Gly Asp Xaa Phe Asn Leu Asn Asp Cys His Lys Leu Ile Asp Phe Phe
625                 630                 635                 640

Lys Asp Ser Ile Ser Arg Tyr Pro Lys Trp Ser Asn Ala Tyr Asp Phe
                645                 650                 655

Asn Phe Ser Glu Thr Glu Lys Tyr Lys Asp Ile Ala Gly Phe Tyr Arg
            660                 665                 670

Glu Val Glu Glu Gln Gly Tyr Lys Val Ser Phe Glu Ser Ala Ser Lys
        675                 680                 685

Lys Glu Val Asp Lys Leu Val Glu Glu Gly Lys Leu Tyr Xaa Phe Gln
    690                 695                 700

Ile Tyr Asn Lys Asp Phe Ser Asp Lys Ser His Gly Thr Pro Asn Leu
705                 710                 715                 720

His Thr Xaa Tyr Phe Lys Leu Leu Phe Asp Glu Asn Asn His Gly Gln
                725                 730                 735

Ile Arg Leu Ser Gly Gly Ala Glu Leu Phe Xaa Arg Arg Ala Ser Leu
            740                 745                 750

Lys Lys Glu Glu Leu Val Val His Pro Ala Asn Ser Pro Ile Ala Asn
        755                 760                 765

Lys Asn Pro Asp Asn Pro Lys Lys Thr Thr Thr Leu Ser Tyr Asp Val
    770                 775                 780

Tyr Lys Asp Lys Arg Phe Ser Glu Asp Gln Tyr Glu Leu His Ile Pro
785                 790                 795                 800

Ile Ala Ile Asn Lys Cys Pro Lys Asn Ile Phe Lys Ile Asn Thr Glu
                805                 810                 815

Val Arg Val Leu Leu Lys His Asp Asp Asn Pro Tyr Val Ile Gly Ile
            820                 825                 830

Asp Arg Gly Glu Arg Asn Leu Leu Tyr Ile Val Val Val Asp Gly Lys
        835                 840                 845

Gly Asn Ile Val Glu Gln Tyr Ser Leu Asn Glu Ile Ile Asn Asn Phe
    850                 855                 860

Asn Gly Ile Arg Ile Lys Thr Asp Tyr His Ser Leu Leu Asp Lys Lys
865                 870                 875                 880

Glu Lys Glu Arg Phe Glu Ala Arg Gln Asn Trp Thr Ser Ile Glu Asn
                885                 890                 895

Ile Lys Glu Leu Lys Ala Gly Tyr Ile Ser Gln Val Val His Lys Ile
            900                 905                 910

Cys Glu Leu Val Glu Lys Tyr Asp Ala Val Ile Ala Leu Glu Asp Leu
        915                 920                 925

Asn Ser Gly Phe Lys Asn Ser Arg Val Lys Val Glu Lys Gln Val Tyr
    930                 935                 940

Gln Lys Phe Glu Lys Xaa Leu Ile Asp Lys Leu Asn Tyr Xaa Val Asp
945                 950                 955                 960

Lys Lys Ser Asn Pro Cys Ala Thr Gly Gly Ala Leu Lys Gly Tyr Gln
                965                 970                 975

Ile Thr Asn Lys Phe Glu Ser Phe Lys Ser Xaa Ser Thr Gln Asn Gly
            980                 985                 990

Phe Ile Phe Tyr Ile Pro Ala Trp Leu Thr Ser Lys Ile Asp Pro Ser
        995                 1000                1005

Thr Gly Phe Val Asn Leu Leu Lys Thr Lys Tyr Thr Ser Ile Ala Asp
    1010                1015                1020

Ser Lys Lys Phe Ile Ser Ser Phe Asp Arg Ile Xaa Tyr Val Pro Glu
1025                1030                1035                1040

Glu Asp Leu Phe Glu Phe Ala Leu Asp Tyr Lys Asn Phe Ser Arg Thr
                1045                1050                1055

Asp Ala Asp Tyr Ile Lys Lys Trp Lys Leu Tyr Ser Tyr Gly Asn Arg
            1060                1065                1070

Ile Arg Ile Phe Ala Ala Ala Lys Lys Asn Asn Val Phe Ala Trp Glu
        1075                1080                1085

Glu Val Cys Leu Thr Ser Ala Tyr Lys Glu Leu Phe Asn Lys Tyr Gly
    1090                1095                1100

Ile Asn Tyr Gln Gln Gly Asp Ile Arg Ala Leu Leu Cys Glu Gln Ser
1105                1110                1115                1120

Asp Lys Ala Phe Tyr Ser Ser Phe Xaa Ala Leu Xaa Ser Leu Xaa Leu
                1125                1130                1135

Gln Xaa Arg Asn Ser Ile Thr Gly Arg Thr Asp Val Asp Phe Leu Ile
            1140                1145                1150

Ser Pro Val Lys Asn Ser Asp Gly Ile Phe Tyr Asp Ser Arg Asn Tyr
        1155                1160                1165

Glu Ala Gln Glu Asn Ala Ile Leu Pro Lys Asn Ala Asp Ala Asn Gly
    1170                1175                1180

Ala Tyr Asn Ile Ala Arg Lys Val Leu Trp Ala Ile Gly Gln Phe Lys
1185                1190                1195                1200

Lys Ala Glu Asp Glu Lys Leu Asp Lys Val Lys Ile Ala Ile Ser Asn
                1205                1210                1215

Lys Glu Trp Leu Glu Tyr Ala Gln Thr Ser Val Lys
            1220                1225


<210>   11
<211>   1300
<212>   PRT
<213>   Artificial Sequence

<220>
<223>   FnCas12a

<400>    11
Met Ser Ile Tyr Gln Glu Phe Val Asn Lys Tyr Ser Leu Ser Lys Thr
1               5                   10                  15

Leu Arg Phe Glu Leu Ile Pro Gln Gly Lys Thr Leu Glu Asn Ile Lys
            20                  25                  30

Ala Arg Gly Leu Ile Leu Asp Asp Glu Lys Arg Ala Lys Asp Tyr Lys
        35                  40                  45

Lys Ala Lys Gln Ile Ile Asp Lys Tyr His Gln Phe Phe Ile Glu Glu
    50                  55                  60

Ile Leu Ser Ser Val Cys Ile Ser Glu Asp Leu Leu Gln Asn Tyr Ser
65                  70                  75                  80

Asp Val Tyr Phe Lys Leu Lys Lys Ser Asp Asp Asp Asn Leu Gln Lys
                85                  90                  95

Asp Phe Lys Ser Ala Lys Asp Thr Ile Lys Lys Gln Ile Ser Glu Tyr
            100                 105                 110

Ile Lys Asp Ser Glu Lys Phe Lys Asn Leu Phe Asn Gln Asn Leu Ile
        115                 120                 125

Asp Ala Lys Lys Gly Gln Glu Ser Asp Leu Ile Leu Trp Leu Lys Gln
    130                 135                 140

Ser Lys Asp Asn Gly Ile Glu Leu Phe Lys Ala Asn Ser Asp Ile Thr
145                 150                 155                 160

Asp Ile Asp Glu Ala Leu Glu Ile Ile Lys Ser Phe Lys Gly Trp Thr
                165                 170                 175

Thr Tyr Phe Lys Gly Phe His Glu Asn Arg Lys Asn Val Tyr Ser Ser
            180                 185                 190

Asn Asp Ile Pro Thr Ser Ile Ile Tyr Arg Ile Val Asp Asp Asn Leu
        195                 200                 205

Pro Lys Phe Leu Glu Asn Lys Ala Lys Tyr Glu Ser Leu Lys Asp Lys
    210                 215                 220

Ala Pro Glu Ala Ile Asn Tyr Glu Gln Ile Lys Lys Asp Leu Ala Glu
225                 230                 235                 240

Glu Leu Thr Phe Asp Ile Asp Tyr Lys Thr Ser Glu Val Asn Gln Arg
                245                 250                 255

Val Phe Ser Leu Asp Glu Val Phe Glu Ile Ala Asn Phe Asn Asn Tyr
            260                 265                 270

Leu Asn Gln Ser Gly Ile Thr Lys Phe Asn Thr Ile Ile Gly Gly Lys
        275                 280                 285

Phe Val Asn Gly Glu Asn Thr Lys Arg Lys Gly Ile Asn Glu Tyr Ile
    290                 295                 300

Asn Leu Tyr Ser Gln Gln Ile Asn Asp Lys Thr Leu Lys Lys Tyr Lys
305                 310                 315                 320

Met Ser Val Leu Phe Lys Gln Ile Leu Ser Asp Thr Glu Ser Lys Ser

325                 330                 335

Phe Val Ile Asp Lys Leu Glu Asp Asp Ser Asp Val Val Thr Thr Met
            340                 345                 350

Gln Ser Phe Tyr Glu Gln Ile Ala Ala Phe Lys Thr Val Glu Glu Lys
        355                 360                 365

Ser Ile Lys Glu Thr Leu Ser Leu Leu Phe Asp Asp Leu Lys Ala Gln
    370                 375                 380

Lys Leu Asp Leu Ser Lys Ile Tyr Phe Lys Asn Asp Lys Ser Leu Thr
385                 390                 395                 400

Asp Leu Ser Gln Gln Val Phe Asp Asp Tyr Ser Val Ile Gly Thr Ala
                405                 410                 415

Val Leu Glu Tyr Ile Thr Gln Gln Ile Ala Pro Lys Asn Leu Asp Asn
            420                 425                 430

Pro Ser Lys Lys Glu Gln Glu Leu Ile Ala Lys Lys Thr Glu Lys Ala
        435                 440                 445

Lys Tyr Leu Ser Leu Glu Thr Ile Lys Leu Ala Leu Glu Glu Phe Asn
    450                 455                 460

Lys His Arg Asp Ile Asp Lys Gln Cys Arg Phe Glu Glu Ile Leu Ala
465                 470                 475                 480

Asn Phe Ala Ala Ile Pro Met Ile Phe Asp Glu Ile Ala Gln Asn Lys
                485                 490                 495

Asp Asn Leu Ala Gln Ile Ser Ile Lys Tyr Gln Asn Gln Gly Lys Lys
            500                 505                 510

Asp Leu Leu Gln Ala Ser Ala Glu Asp Asp Val Lys Ala Ile Lys Asp
        515                 520                 525

Leu Leu Asp Gln Thr Asn Asn Leu Leu His Lys Leu Lys Ile Phe His
    530                 535                 540

Ile Ser Gln Ser Glu Asp Lys Ala Asn Ile Leu Asp Lys Asp Glu His
545                 550                 555                 560

Phe Tyr Leu Val Phe Glu Glu Cys Tyr Phe Glu Leu Ala Asn Ile Val
                565                 570                 575

Pro Leu Tyr Asn Lys Ile Arg Asn Tyr Ile Thr Gln Lys Pro Tyr Ser
            580                 585                 590

Asp Glu Lys Phe Lys Leu Asn Phe Glu Asn Ser Thr Leu Ala Asn Gly
        595                 600                 605

Trp Asp Lys Asn Lys Glu Pro Asp Asn Thr Ala Ile Leu Phe Ile Lys
    610                 615                 620

Asp Asp Lys Tyr Tyr Leu Gly Val Met Asn Lys Lys Asn Asn Lys Ile
625                 630                 635                 640

Phe Asp Asp Lys Ala Ile Lys Glu Asn Lys Gly Glu Gly Tyr Lys Lys
                645                 650                 655

Ile Val Tyr Lys Leu Leu Pro Gly Ala Asn Lys Met Leu Pro Lys Val

660                 665                 670

Phe Phe Ser Ala Lys Ser Ile Lys Phe Tyr Asn Pro Ser Glu Asp Ile
        675                 680                 685

Leu Arg Ile Arg Asn His Ser Thr His Thr Lys Asn Gly Ser Pro Gln
    690                 695                 700

Lys Gly Tyr Glu Lys Phe Glu Phe Asn Ile Glu Asp Cys Arg Lys Phe
705                 710                 715                 720

Ile Asp Phe Tyr Lys Gln Ser Ile Ser Lys His Pro Glu Trp Lys Asp
                725                 730                 735

Phe Gly Phe Arg Phe Ser Asp Thr Gln Arg Tyr Asn Ser Ile Asp Glu
            740                 745                 750

Phe Tyr Arg Glu Val Glu Asn Gln Gly Tyr Lys Leu Thr Phe Glu Asn
        755                 760                 765

Ile Ser Glu Ser Tyr Ile Asp Ser Val Val Asn Gln Gly Lys Leu Tyr
    770                 775                 780

Leu Phe Gln Ile Tyr Asn Lys Asp Phe Ser Ala Tyr Ser Lys Gly Arg
785                 790                 795                 800

Pro Asn Leu His Thr Leu Tyr Trp Lys Ala Leu Phe Asp Glu Arg Asn
                805                 810                 815

Leu Gln Asp Val Val Tyr Lys Leu Asn Gly Glu Ala Glu Leu Phe Tyr
            820                 825                 830

Arg Lys Gln Ser Ile Pro Lys Lys Ile Thr His Pro Ala Lys Glu Ala
        835                 840                 845

Ile Ala Asn Lys Asn Lys Asp Asn Pro Lys Lys Glu Ser Val Phe Glu
    850                 855                 860

Tyr Asp Leu Ile Lys Asp Lys Arg Phe Thr Glu Asp Lys Phe Phe Phe
865                 870                 875                 880

His Cys Pro Ile Thr Ile Asn Phe Lys Ser Ser Gly Ala Asn Lys Phe
                885                 890                 895

Asn Asp Glu Ile Asn Leu Leu Leu Lys Glu Lys Ala Asn Asp Val His
            900                 905                 910

Ile Leu Ser Ile Asp Arg Gly Glu Arg His Leu Ala Tyr Tyr Thr Leu
        915                 920                 925

Val Asp Gly Lys Gly Asn Ile Ile Lys Gln Asp Thr Phe Asn Ile Ile
    930                 935                 940

Gly Asn Asp Arg Met Lys Thr Asn Tyr His Asp Lys Leu Ala Ala Ile
945                 950                 955                 960

Glu Lys Asp Arg Asp Ser Ala Arg Lys Asp Trp Lys Lys Ile Asn Asn
                965                 970                 975

Ile Lys Glu Met Lys Glu Gly Tyr Leu Ser Gln Val Val His Glu Ile
            980                 985                 990

Ala Lys Leu Val Ile Glu Tyr Asn Ala Ile Val Val Phe Glu Asp Leu

995                 1000                1005

Asn Phe Gly Phe Lys Arg Gly Arg Phe Lys Val Glu Lys Gln Val Tyr
    1010                1015                1020

Gln Lys Leu Glu Lys Met Leu Ile Glu Lys Leu Asn Tyr Leu Val Phe
1025                1030                1035                1040

Lys Asp Asn Glu Phe Asp Lys Thr Gly Gly Val Leu Arg Ala Tyr Gln
                1045                1050                1055

Leu Thr Ala Pro Phe Glu Thr Phe Lys Lys Met Gly Lys Gln Thr Gly
            1060                1065                1070

Ile Ile Tyr Tyr Val Pro Ala Gly Phe Thr Ser Lys Ile Cys Pro Val
        1075                1080                1085

Thr Gly Phe Val Asn Gln Leu Tyr Pro Lys Tyr Glu Ser Val Ser Lys
    1090                1095                1100

Ser Gln Glu Phe Phe Ser Lys Phe Asp Lys Ile Cys Tyr Asn Leu Asp
1105                1110                1115                1120

Lys Gly Tyr Phe Glu Phe Ser Phe Asp Tyr Lys Asn Phe Gly Asp Lys
                1125                1130                1135

Ala Ala Lys Gly Lys Trp Thr Ile Ala Ser Phe Gly Ser Arg Leu Ile
            1140                1145                1150

Asn Phe Arg Asn Ser Asp Lys Asn His Asn Trp Asp Thr Arg Glu Val
        1155                1160                1165

Tyr Pro Thr Lys Glu Leu Glu Lys Leu Leu Lys Asp Tyr Ser Ile Glu
    1170                1175                1180

Tyr Gly His Gly Glu Cys Ile Lys Ala Ala Ile Cys Gly Glu Ser Asp
1185                1190                1195                1200

Lys Lys Phe Phe Ala Lys Leu Thr Ser Val Leu Asn Thr Ile Leu Gln
                1205                1210                1215

Met Arg Asn Ser Lys Thr Gly Thr Glu Leu Asp Tyr Leu Ile Ser Pro
            1220                1225                1230

Val Ala Asp Val Asn Gly Asn Phe Phe Asp Ser Arg Gln Ala Pro Lys
        1235                1240                1245

Asn Met Pro Gln Asp Ala Asp Ala Asn Gly Ala Tyr His Ile Gly Leu
    1250                1255                1260

Lys Gly Leu Met Leu Leu Gly Arg Ile Lys Asn Asn Gln Glu Gly Lys
1265                1270                1275                1280

Lys Leu Asn Leu Val Ile Lys Asn Glu Glu Tyr Phe Glu Phe Val Gln
                1285                1290                1295

Asn Arg Asn Asn
            1300


<210>   12
<211>   19
<212>   RNA

<213>    Artificial Sequence

<220>
<223>    5'-handle RNA


<400>    12
aauuucuacu guuguagau                                                    19


<210>    13
<211>    1263
<212>    PRT
<213>    Artificial Sequence

<220>
<223>    d_mgCas12a-1


<400>    13
Met Asn Asn Gly Thr Asn Asn Phe Gln Asn Phe Ile Gly Ile Ser Ser
1               5                   10                  15

Leu Gln Lys Thr Leu Arg Asn Ala Leu Ile Pro Thr Glu Thr Thr Gln
            20                  25                  30

Gln Phe Ile Val Lys Asn Gly Ile Ile Lys Glu Asp Glu Leu Arg Gly
        35                  40                  45

Glu Asn Arg Gln Ile Leu Lys Asp Ile Met Asp Asp Tyr Tyr Arg Gly
    50                  55                  60

Phe Ile Ser Glu Thr Leu Ser Ser Ile Asp Asp Ile Asp Trp Thr Ser
65                  70                  75                  80

Leu Phe Glu Lys Met Glu Ile Gln Leu Lys Asn Gly Asp Asn Lys Asp
                85                  90                  95

Thr Leu Ile Lys Glu Gln Ala Glu Lys Arg Lys Ala Ile Tyr Lys Lys
            100                 105                 110

Phe Ala Asp Asp Asp Arg Phe Lys Asn Met Phe Ser Ala Lys Leu Ile
        115                 120                 125

Ser Asp Ile Leu Pro Glu Phe Val Ile His Asn Asn Asn Tyr Ser Ala
    130                 135                 140

Ser Glu Lys Glu Glu Lys Thr Gln Val Ile Lys Leu Phe Ser Arg Phe
145                 150                 155                 160

Ala Thr Ser Phe Lys Asp Tyr Phe Lys Asn Arg Ala Asn Cys Phe Ser
                165                 170                 175

Ala Asp Asp Ile Ser Ser Ser Ser Cys His Arg Ile Val Asn Asp Asn
            180                 185                 190

Ala Glu Ile Phe Phe Ser Asn Ala Leu Val Tyr Arg Arg Ile Val Lys
        195                 200                 205

Asn Leu Ser Asn Asp Asp Ile Asn Lys Ile Ser Gly Asp Ile Lys Asp
    210                 215                 220

Ser Leu Lys Glu Met Ser Leu Glu Glu Ile Tyr Ser Tyr Glu Lys Tyr

225                 230                 235                 240

Gly Glu Phe Ile Thr Gln Glu Gly Ile Ser Phe Tyr Asn Asp Ile Cys
                245                 250                 255

Gly Lys Val Asn Ser Phe Met Asn Leu Tyr Cys Gln Lys Asn Lys Glu
            260                 265                 270

Asn Lys Asn Leu Tyr Lys Leu Arg Lys Leu His Lys Gln Ile Leu Cys
        275                 280                 285

Ile Ala Asp Thr Ser Tyr Glu Val Pro Tyr Lys Phe Glu Ser Asp Glu
    290                 295                 300

Glu Val Tyr Gln Ser Val Asn Gly Phe Leu Asp Asn Ile Ser Ser Lys
305                 310                 315                 320

His Ile Val Glu Arg Leu Arg Lys Ile Gly Asp Asn Tyr Asn Gly Tyr
                325                 330                 335

Asn Leu Asp Lys Ile Tyr Ile Val Ser Lys Phe Tyr Glu Ser Val Ser
            340                 345                 350

Gln Lys Thr Tyr Arg Asp Trp Glu Thr Ile Asn Thr Ala Leu Glu Ile
        355                 360                 365

His Tyr Asn Asn Ile Leu Pro Gly Asn Gly Lys Ser Lys Ala Asp Lys
    370                 375                 380

Val Lys Lys Ala Val Lys Asn Asp Leu Gln Lys Ser Ile Thr Glu Ile
385                 390                 395                 400

Asn Glu Leu Val Ser Asn Tyr Lys Leu Cys Pro Asp Asp Asn Ile Lys
                405                 410                 415

Ala Glu Thr Tyr Ile His Glu Ile Ser His Ile Leu Asn Asn Phe Glu
            420                 425                 430

Ala Gln Glu Leu Lys Tyr Asn Pro Glu Ile His Leu Val Glu Ser Glu
        435                 440                 445

Leu Lys Ala Ser Glu Leu Lys Asn Val Leu Asp Val Ile Met Asn Ala
    450                 455                 460

Phe His Trp Cys Ser Val Phe Met Thr Glu Glu Leu Val Asp Lys Asp
465                 470                 475                 480

Asn Asn Phe Tyr Ala Glu Leu Glu Glu Ile Tyr Asp Glu Ile Tyr Thr
                485                 490                 495

Val Ile Ser Leu Tyr Asn Leu Val Arg Asn Tyr Val Thr Gln Lys Pro
            500                 505                 510

Tyr Ser Thr Lys Lys Ile Lys Leu Asn Phe Gly Ile Pro Thr Leu Ala
        515                 520                 525

Asp Gly Trp Ser Lys Ser Lys Glu Tyr Ser Asn Asn Ala Ile Ile Leu
    530                 535                 540

Met Arg Asp Asn Leu Tyr Tyr Leu Gly Ile Phe Asn Ala Lys Asn Lys
545                 550                 555                 560

Pro Asp Lys Lys Ile Ile Glu Gly Asn Thr Ser Glu Asn Lys Gly Asp

565                 570                 575

Tyr Lys Lys Met Ile Tyr Asn Leu Leu Pro Gly Pro Asn Lys Met Ile
            580                 585                 590

Pro Lys Val Phe Leu Ser Ser Lys Thr Gly Val Glu Thr Tyr Lys Pro
        595                 600                 605

Ser Ala Tyr Ile Leu Glu Gly Tyr Lys Gln Asn Lys His Leu Lys Ser
    610                 615                 620

Ser Lys Asp Phe Asp Ile Thr Phe Cys His Asp Leu Ile Asp Tyr Phe
625                 630                 635                 640

Lys Asn Cys Ile Ala Ile His Pro Glu Trp Lys Asn Phe Gly Phe Asp
                645                 650                 655

Phe Ser Asp Thr Ser Thr Tyr Glu Asp Ile Ser Gly Phe Tyr Arg Glu
            660                 665                 670

Val Glu Leu Gln Gly Tyr Lys Ile Asp Trp Thr Tyr Ile Ser Glu Lys
        675                 680                 685

Asp Ile Asp Leu Leu Gln Glu Lys Gly Gln Leu Tyr Leu Phe Gln Ile
    690                 695                 700

Tyr Asn Lys Asp Phe Ser Lys Lys Ser Thr Gly Asn Asp Asn Leu His
705                 710                 715                 720

Thr Met Tyr Leu Lys Asn Leu Phe Ser Glu Glu Asn Leu Lys Asp Ile
                725                 730                 735

Val Leu Lys Leu Asn Gly Glu Ala Glu Ile Phe Phe Arg Lys Ser Ser
            740                 745                 750

Ile Lys Asn Pro Ile Ile His Lys Lys Gly Ser Ile Leu Val Asn Arg
        755                 760                 765

Thr Tyr Glu Ala Glu Glu Lys Asp Gln Phe Gly Asn Ile Gln Ile Val
    770                 775                 780

Arg Lys Thr Ile Pro Glu Asn Ile Tyr Gln Glu Leu Tyr Lys Tyr Phe
785                 790                 795                 800

Asn Asp Lys Ser Asp Lys Glu Leu Ser Asp Glu Ala Ala Lys Leu Lys
                805                 810                 815

Asn Val Val Gly His His Glu Ala Ala Thr Asn Ile Val Lys Asp Tyr
            820                 825                 830

Arg Tyr Thr Tyr Asp Lys Tyr Phe Leu His Met Pro Ile Thr Ile Asn
        835                 840                 845

Phe Lys Ala Asn Lys Thr Ser Phe Ile Asn Asp Arg Ile Leu Gln Tyr
    850                 855                 860

Ile Ala Lys Glu Lys Asn Leu His Val Ile Gly Ile Ala Arg Gly Glu
865                 870                 875                 880

Arg Asn Leu Ile Tyr Val Ser Val Ile Asp Thr Cys Gly Asn Ile Val
                885                 890                 895

Glu Gln Lys Ser Phe Asn Ile Val Asn Gly Tyr Asp Tyr Gln Ile Lys

900                 905                 910

Leu Lys Gln Gln Glu Gly Ala Arg Gln Ile Ala Arg Lys Glu Trp Lys
        915                 920                 925

Glu Ile Gly Lys Ile Lys Glu Ile Lys Glu Gly Tyr Leu Ser Leu Val
    930                 935                 940

Ile His Glu Ile Ser Lys Met Val Ile Lys Tyr Asn Ala Ile Ile Ala
945                 950                 955                 960

Met Glu Asp Leu Ser Tyr Gly Phe Lys Lys Gly Arg Phe Lys Val Glu
                965                 970                 975

Arg Gln Val Tyr Gln Lys Phe Glu Thr Met Leu Ile Asn Lys Leu Asn
            980                 985                 990

Tyr Leu Val Phe Lys Asp Ile Ser Ile Thr Glu Asn Gly Gly Leu Leu
        995                 1000                1005

Lys Gly Tyr Gln Leu Thr Tyr Ile Pro Asp Lys Leu Lys Asn Val Gly
    1010                1015                1020

His Gln Cys Gly Cys Ile Phe Tyr Val Pro Ala Ala Tyr Thr Ser Lys
1025                1030                1035                1040

Ile Asp Pro Thr Thr Gly Phe Val Asn Ile Phe Lys Phe Lys Asp Leu
                1045                1050                1055

Thr Val Asp Ala Lys Arg Glu Phe Ile Lys Lys Phe Asp Ser Ile Arg
            1060                1065                1070

Tyr Asp Ser Glu Lys Lys Leu Phe Cys Phe Thr Phe Asp Tyr Asn Asn
        1075                1080                1085

Phe Ile Thr Gln Asn Thr Val Met Ser Lys Ser Ser Trp Ser Val Tyr
    1090                1095                1100

Thr Tyr Gly Val Arg Ile Lys Arg Arg Phe Val Asn Gly Arg Phe Ser
1105                1110                1115                1120

Asn Glu Ser Asp Thr Ile Asp Ile Thr Lys Asp Met Glu Lys Thr Leu
                1125                1130                1135

Glu Met Thr Asp Ile Asn Trp Arg Asp Gly His Asp Leu Arg Gln Asp
            1140                1145                1150

Ile Ile Asp Tyr Glu Ile Val Gln His Ile Phe Glu Ile Phe Arg Leu
        1155                1160                1165

Thr Val Gln Met Arg Asn Ser Leu Ser Glu Leu Glu Asp Arg Asp Tyr
    1170                1175                1180

Asp Arg Leu Ile Ser Pro Val Leu Asn Glu Asn Asn Ile Phe Tyr Asp
1185                1190                1195                1200

Ser Ala Lys Ala Gly Asp Ala Leu Pro Lys Asp Ala Asp Ala Asn Gly
                1205                1210                1215

Ala Tyr Cys Ile Ala Leu Lys Gly Leu Tyr Glu Ile Lys Gln Ile Thr
            1220                1225                1230

Glu Asn Trp Lys Glu Asp Gly Lys Phe Ser Arg Asp Lys Leu Lys Ile

1235                1240                1245

Ser Asn Lys Asp Trp Phe Asp Phe Ile Gln Asn Lys Arg Tyr Leu
    1250                1255                1260

<210>    14
<211>    1275
<212>    PRT
<213>    Artificial Sequence

<220>
<223>    d_mgCas12a-2

<400>    14
Met Gly Lys Asn Gln Asn Phe Gln Glu Phe Ile Gly Val Ser Pro Leu
1               5                   10                  15

Gln Lys Thr Leu Arg Asn Glu Leu Ile Pro Thr Glu Thr Thr Lys Lys
            20                  25                  30

Asn Ile Thr Gln Leu Asp Leu Leu Thr Glu Asp Glu Ile Arg Ala Gln
        35                  40                  45

Asn Arg Glu Lys Leu Lys Glu Met Met Asp Asp Tyr Tyr Arg Asn Val
    50                  55                  60

Ile Asp Ser Thr Leu His Val Gly Ile Ala Val Asp Trp Ser Tyr Leu
65                  70                  75                  80

Phe Ser Cys Met Arg Asn His Leu Arg Glu Asn Ser Lys Glu Ser Lys
                85                  90                  95

Arg Glu Leu Glu Arg Thr Gln Asp Ser Ile Arg Ser Gln Ile His Asn
            100                 105                 110

Lys Phe Ala Glu Arg Ala Asp Phe Lys Asp Met Phe Gly Ala Ser Ile
        115                 120                 125

Ile Thr Lys Leu Leu Pro Thr Tyr Ile Lys Gln Asn Ser Glu Tyr Ser
    130                 135                 140

Glu Arg Tyr Asp Glu Ser Met Glu Ile Leu Lys Leu Tyr Gly Lys Phe
145                 150                 155                 160

Thr Thr Ser Leu Thr Asp Tyr Phe Glu Thr Arg Lys Asn Ile Phe Ser
                165                 170                 175

Lys Glu Lys Ile Ser Ser Ala Val Gly Tyr Arg Ile Val Glu Glu Asn
            180                 185                 190

Ala Glu Ile Phe Leu Gln Asn Gln Asn Ala Tyr Asp Arg Ile Cys Lys
        195                 200                 205

Ile Ala Gly Leu Asp Leu His Gly Leu Asp Asn Glu Ile Thr Ala Tyr
    210                 215                 220

Val Asp Gly Lys Thr Leu Lys Glu Val Cys Ser Asp Glu Gly Phe Ala
225                 230                 235                 240

Lys Ala Ile Thr Gln Glu Gly Ile Asp Arg Tyr Asn Glu Ala Ile Gly
                245                 250                 255

Ala Val Asn Gln Tyr Met Asn Leu Leu Cys Gln Lys Asn Lys Ala Leu
            260                 265                 270

Lys Pro Gly Gln Phe Lys Met Lys Arg Leu His Lys Gln Ile Leu Cys
        275                 280                 285

Lys Gly Thr Thr Ser Phe Asp Ile Pro Lys Lys Phe Glu Asn Asp Lys
    290                 295                 300

Gln Val Tyr Asp Ala Val Asn Ser Phe Thr Glu Ile Val Thr Lys Asn
305                 310                 315                 320

Asn Asp Leu Lys Arg Leu Leu Asn Ile Thr Gln Asn Ala Asn Asp Tyr
                325                 330                 335

Asp Met Asn Lys Ile Tyr Val Val Ala Asp Ala Tyr Ser Met Ile Ser
            340                 345                 350

Gln Phe Ile Ser Lys Lys Trp Asn Leu Ile Glu Glu Cys Leu Leu Asp
        355                 360                 365

Tyr Tyr Ser Asp Asn Leu Pro Gly Lys Gly Asn Ala Lys Glu Asn Lys
    370                 375                 380

Val Lys Lys Ala Val Lys Glu Glu Thr Tyr Arg Ser Val Ser Gln Leu
385                 390                 395                 400

Asn Glu Val Ile Glu Lys Tyr Tyr Val Glu Lys Thr Gly Gln Ser Val
                405                 410                 415

Trp Lys Val Glu Ser Tyr Ile Ser Ser Leu Ala Glu Met Ile Lys Leu
            420                 425                 430

Glu Leu Cys His Glu Ile Asp Asn Asp Glu Lys His Asn Leu Ile Glu
        435                 440                 445

Asp Asp Glu Lys Ile Ser Glu Ile Lys Glu Leu Leu Asp Met Tyr Met
    450                 455                 460

Asp Val Phe His Ile Ile Lys Val Phe Arg Val Asn Glu Val Leu Asn
465                 470                 475                 480

Phe Asp Glu Thr Phe Tyr Ser Glu Met Asp Glu Ile Tyr Gln Asp Met
                485                 490                 495

Gln Glu Ile Val Pro Leu Tyr Asn His Val Arg Asn Tyr Val Thr Gln
            500                 505                 510

Lys Pro Tyr Lys Gln Glu Lys Tyr Arg Leu Tyr Phe His Thr Pro Thr
        515                 520                 525

Leu Ala Asn Gly Trp Ser Lys Ser Lys Glu Tyr Asp Asn Asn Ala Ile
    530                 535                 540

Ile Leu Val Arg Glu Asp Lys Tyr Tyr Leu Gly Ile Leu Asn Ala Lys
545                 550                 555                 560

Lys Lys Pro Ser Lys Glu Ile Met Ala Gly Lys Glu Asp Cys Ser Glu
                565                 570                 575

His Ala Tyr Ala Lys Met Asn Tyr Tyr Leu Leu Pro Gly Ala Asn Lys
            580                 585                 590

Met Leu Pro Lys Val Phe Leu Ser Lys Lys Gly Ile Gln Asp Tyr His
        595                 600                 605

Pro Ser Ser Tyr Ile Val Glu Gly Tyr Asn Glu Lys Lys His Ile Lys
    610                 615                 620

Gly Ser Lys Asn Phe Asp Ile Arg Phe Cys Arg Asp Leu Ile Asp Tyr
625                 630                 635                 640

Phe Lys Glu Cys Ile Lys Lys His Pro Asp Trp Asn Lys Phe Asn Phe
                645                 650                 655

Glu Phe Ser Ala Thr Glu Thr Tyr Glu Asp Ile Ser Val Phe Tyr Arg
            660                 665                 670

Glu Val Glu Lys Gln Gly Tyr Arg Val Glu Trp Thr Tyr Ile Asn Ser
        675                 680                 685

Glu Asp Ile Gln Lys Leu Glu Glu Asp Gly Gln Leu Phe Leu Phe Gln
    690                 695                 700

Ile Tyr Asn Lys Asp Phe Ala Val Gly Ser Thr Gly Lys Pro Asn Leu
705                 710                 715                 720

His Thr Leu Tyr Leu Lys Asn Leu Phe Ser Glu Glu Asn Leu Arg Asp
                725                 730                 735

Ile Val Leu Lys Leu Asn Gly Glu Ala Glu Ile Phe Phe Arg Lys Ser
            740                 745                 750

Ser Val Gln Lys Pro Val Ile His Lys Cys Gly Ser Ile Leu Val Asn
        755                 760                 765

Arg Thr Tyr Glu Ile Thr Glu Ser Gly Thr Thr Arg Val Gln Ser Ile
    770                 775                 780

Pro Glu Ser Glu Tyr Met Glu Leu Tyr Arg Tyr Phe Asn Ser Glu Lys
785                 790                 795                 800

Gln Ile Glu Leu Ser Asp Glu Ala Lys Lys Tyr Leu Asp Lys Val Gln
                805                 810                 815

Cys Asn Lys Ala Lys Thr Asp Ile Val Lys Asp Tyr Arg Tyr Thr Met
            820                 825                 830

Asp Lys Phe Phe Ile His Leu Pro Ile Thr Ile Asn Phe Lys Val Asp
        835                 840                 845

Lys Gly Asn Asn Val Asn Ala Ile Ala Gln Gln Tyr Ile Ala Gly Arg
    850                 855                 860

Lys Asp Leu His Val Ile Gly Ile Ala Arg Gly Glu Arg Asn Leu Ile
865                 870                 875                 880

Tyr Val Ser Val Ile Asp Met Tyr Gly Arg Ile Leu Glu Gln Lys Ser
                885                 890                 895

Phe Asn Leu Val Glu Gln Val Ser Ser Gln Gly Thr Lys Arg Tyr Tyr
            900                 905                 910

Asp Tyr Lys Glu Lys Leu Gln Asn Arg Glu Glu Glu Arg Asp Lys Ala
        915                 920                 925

Arg Lys Ser Trp Lys Thr Ile Gly Lys Ile Lys Glu Leu Lys Glu Gly
    930                 935                 940

Tyr Leu Ser Ser Val Ile His Glu Ile Ala Gln Met Val Val Lys Tyr
945                 950                 955                 960

Asn Ala Ile Ile Ala Met Glu Asp Leu Asn Tyr Gly Phe Lys Arg Gly
                965                 970                 975

Arg Phe Lys Val Glu Arg Gln Val Tyr Gln Lys Phe Glu Thr Met Leu
            980                 985                 990

Ile Ser Lys Leu Asn Tyr Leu Ala Asp Lys Ser Gln Ala Val Asp Glu
        995                 1000                1005

Pro Gly Gly Ile Leu Arg Gly Tyr Gln Met Thr Tyr Val Pro Asp Asn
    1010                1015                1020

Ile Lys Asn Val Gly Arg Gln Cys Gly Ile Ile Phe Tyr Val Pro Ala
1025                1030                1035                1040

Ala Tyr Thr Ser Lys Ile Asp Pro Thr Thr Gly Phe Ile Asn Ala Phe
                1045                1050                1055

Lys Arg Asp Val Val Ser Thr Asn Asp Ala Lys Glu Asn Phe Leu Met
            1060                1065                1070

Lys Phe Asp Ser Ile Gln Tyr Asp Ile Glu Lys Gly Leu Phe Lys Phe
        1075                1080                1085

Ser Phe Asp Tyr Lys Asn Phe Ala Thr His Lys Leu Thr Leu Ala Lys
    1090                1095                1100

Thr Lys Trp Asp Val Tyr Thr Asn Gly Thr Arg Ile Gln Asn Met Lys
1105                1110                1115                1120

Val Glu Gly His Trp Leu Ser Met Glu Val Glu Leu Thr Thr Lys Met
                1125                1130                1135

Lys Glu Leu Leu Asp Asp Ser His Ile Pro Tyr Glu Glu Gly Gln Asn
            1140                1145                1150

Ile Leu Asp Asp Leu Arg Glu Met Lys Asp Ile Thr Thr Ile Val Asn
        1155                1160                1165

Gly Ile Leu Glu Ile Phe Trp Leu Thr Val Gln Leu Arg Asn Ser Arg
    1170                1175                1180

Ile Asp Asn Pro Asp Tyr Asp Arg Ile Ile Ser Pro Val Leu Asn Lys
1185                1190                1195                1200

Asn Gly Glu Phe Phe Asp Ser Asp Glu Tyr Asn Ser Tyr Ile Asp Ala
                1205                1210                1215

Gln Lys Ala Pro Leu Pro Ile Asp Ala Asp Ala Asn Gly Ala Phe Cys
            1220                1225                1230

Ile Ala Leu Lys Gly Met Tyr Thr Ala Asn Gln Ile Lys Glu Asn Trp
        1235                1240                1245

Val Glu Gly Glu Lys Leu Pro Ala Asp Cys Leu Lys Ile Glu His Ala
   1250                1255                1260

Ser Trp Leu Ala Phe Met Gln Gly Glu Arg Gly
1265                1270                1275


<210>    15
<211>    53
<212>    DNA
<213>    Artificial Sequence

<220>
<223>    CCR5 Adapter primer sequence (5'-3')


<400>    15
tcgtcggcag cgtcagatgt gtataagaga cagggtattt ctgttcagat cac          53


<210>    16
<211>    55
<212>    DNA
<213>    Artificial Sequence

<220>
<223>    CCR5 Adapter primer sequence (5'-3')


<400>    16
gtctcgtggg ctcggagatg tgtataagag acaggcccat caattataga aagcc        55


<210>    17
<211>    53
<212>    DNA
<213>    Artificial Sequence

<220>
<223>    DNMT1 Adapter primer sequence (5'-3')


<400>    17
tcgtcggcag cgtcagatgt gtataagaga cagctgcaca cagcaggcct ttg          53


<210>    18
<211>    54
<212>    DNA
<213>    Artificial Sequence

<220>
<223>    DNMT1 Adapter primer sequence (5'-3')


<400>    18
gtctcgtggg ctcggagatg tgtataagag acagcccaat aagtggcaga gtgc         54


<210>    19
<211>    28
<212>    RNA
<213>    Artificial Sequence

<220>
<223>    NbFTa14_1/2-2 crRNA sequence(PAM site)


<400>    19
tttggataat ttgtactctt gtcgatgt                                          28


<210>    20
<211>    28
<212>    RNA
<213>    Artificial Sequence

<220>
<223>    NbFTa14_1/2-4 crRNA sequence(PAM site)


<400>    20
tttagtccac aaacagctaa gcccacat                                          28


<210>    21
<211>    20
<212>    DNA
<213>    Artificial Sequence

<220>
<223>    NGS NbFTa14_1 Forward primer


<400>    21
tgagctgaag atggattatg                                                   20


<210>    22
<211>    20
<212>    DNA
<213>    Artificial Sequence

<220>
<223>    NGS NbFTa14_1 Reverse primer


<400>    22
tcatgcttaa gataaaagag                                                   20


<210>    23
<211>    20
<212>    DNA
<213>    Artificial Sequence

<220>
<223>    NGS NbFTa14_2 Forward primer


<400>    23
tcatgagctt aagatggatc                                                   20


<210>    24
<211>    20

<212>    DNA
<213>    Artificial Sequence

<220>
<223>    NGS NbFTa14_2 Reverse primer


<400>    24
gtttaagcta aaagaactac                                                    20


<210>    25
<211>    43
<212>    RNA
<213>    Artificial Sequence

<220>
<223>    LsXTb12 crRNA #1


<400>    25
aatttctact aagtgtagat tcttcatcct caattccatc acc                          43


<210>    26
<211>    43
<212>    RNA
<213>    Artificial Sequence

<220>
<223>    LsXTb12 crRNA #2


<400>    26
aatttctact aagtgtagat gcaagcctgt aactctggaa gac                          43


<210>    27
<211>    1504
<212>    DNA
<213>    Artificial Sequence

<220>
<223>    HsCCR5 Linear DNA


<400>    27
ggtggtggct gtgtttgcgt ctctcccagg aatcatcttt accagatctc aaaaagaagg        60

tcttcattac acctgcagct ctcattttcc atacagtcag tatcaattct ggaagaattt       120

ccagacatta aagatagtca tcttggggct ggtcctgccg ctgcttgtca tggtcatctg       180

ctactcggga atcctaaaaa ctctgcttcg gtgtcgaaat gagaagaaga ggcacagggc       240

tgtgaggctt atcttcacca tcatgattgt ttattttctc ttctgggctc cctacaacat       300

tgtccttctc ctgaacacct tccaggaatt ctttggcctg aataattgca gtagctctaa       360

caggttggac caagctatgc aggtgacaga gactcttggg atgacgcact gctgcatcaa       420

ccccatcatc tatgcctttg tcggggagaa gttcagaaac tacctcttag tcttcttcca       480

aaagcacatt gccaaacgct tctgcaaatg ctgttctatt ttccagcaag aggctcccga 540

gcgagcaagc tcagtttaca cccgatccac tggggagcag gaaatatctg tgggcttgtg 600

acacggactc aagtgggctg gtgacccagt cagagttgtg cacatggctt agttttcata 660

cacagcctgg gctgggggtg gggtgggaga ggtctttttt aaaaggaagt tactgttata 720

gagggtctaa gattcatcca tttatttggc atctgtttaa agtagattag atcttttaag 780

cccatcaatt atagaaagcc aaatcaaaat atgttgatga aaaatagcaa cctttttatc 840

tccccttcac atgcatcaag ttattgacaa actctccctt cactccgaaa gttccttatg 900

tatatttaaa agaaagcctc agagaattgc tgattcttga gtttagtgat ctgaacagaa 960

ataccaaaat tatttcagaa atgtacaact ttttacctag tacaaggcaa catataggtt 1020

gtaaatgtgt ttaaaacagg tctttgtctt gctatgggga gaaaagacat gaatatgatt 1080

agtaaagaaa tgacactttt catgtgtgat ttcccctcca aggtatggtt aataagtttc 1140

actgacttag aaccaggcga gagacttgtg gcctgggaga gctggggaag cttcttaaat 1200

gagaaggaat ttgagttgga tcatctattg ctggcaaaga cagaagcctc actgcaagca 1260

ctgcatgggc aagcttggct gtagaaggag acagagctgg ttgggaagac atggggagga 1320

aggacaaggc tagatcatga agaaccttga cggcattgct ccgtctaagt catgagctga 1380

gcagggagat cctggttggt gttgcagaag gtttactctg tggccaaagg agggtcagga 1440

aggatgagca tttagggcaa ggagaccacc aacagccctc aggtcagggt gaggatggcc 1500

tctg 1504

<210> 28
<211> 1119
<212> DNA
<213> Artificial Sequence

<220>
<223> HsDNMT1 Linear DNA

<400> 28
gctgctctcg aactcctggc ctcaactaat ccacctgcct tggcctccca aagtgctggg 60

attacaggcg tgagccactg ctcccagccc cacgtgtctt tgtctcaagt ctttctgaag 120

ctcttcaaag gcccagtgac ttgtggctgt ggggcgggat gatgggccag ttggagggtc 180

caaggatctt gtgctggaag ggttttgggc ccatgtgagc aggaccagaa cccttcccca 240

aggggtgcaa tgcccaggtt gtcctccatc tgagcagggg ctggcagtac acctgccccc 300

gggccttggg cctgggtgtc cacatcaggc attgcccttc tcccctcctg caggtgggca 360

atgccgtgcc accgcccctg gccaaagcca ttggcttgga gatcaagctt tgtatgttgg 420

ccaaagcccg agagagtgcc tcaggtatgg tggggtgggc caggcttcct ctggggcctg 480

actgccctct gggggtacat gtgggggcag ttgctggcca ccgttttggg ctctgggact 540

caggcgggtc acctacccac gttcgtggcc ccatctttct caaggggctg ctgtgaggat 600

tgagtgagtt gcacgtgtca agtgcttaga gcaggcgtgc tgcacacagc aggcctttgg 660

tcaggttggc tgctgggctg gccctggggc cgtttccctc actcctgctc ggtgaatttg 720

gctcagcagg cacctgcctc agctgctcac ttgagcctct gggtctagaa ccctctgggg 780

accgtttgag gagtgttcag tctccgtgaa cgttccctta gcactctgcc acttattggg 840

tcagctgtta acatcagtac gttaatgttt cctgatggtc catgtctgtt actcgcctgt 900

caagtggcgt gacaccgggc gtgttcccca gagtgacttt tccttttatt tcccttcagc 960

taaaataaag gaggaggaag ctgctaagga ctagttctgc cctcccgtca cccctgtttc 1020

tggcaccagg aatccccaac atgcactgat gttgtgtttt taacatgtca atctgtccgt 1080

tcacatgtgt ggtacatggt gtttgtggcc ttggctgac 1119

<210> 29
<211> 1460
<212> DNA
<213> Artificial Sequence

<220>
<223> HsEMX1 Linear DNA

<400> 29
gtggggacag aaggtctgga gctgcccgtg aagggcagaa tgctgccctc agacccgctt 60

cctccctgtc cttgtctgtc caaggagaat gaggtctcac tggtggattt cggactaccc 120

tgaggagctg gcacctgagg gacaaggccc cccacctgcc cagctccagc ctctgatgag 180

gggtgggaga gagctacatg aggttgctaa gaaagcctcc cctgaaggag accacacagt 240

gtgtgaggtt ggagtctcta gcagcgggtt ctgtgccccc agggatagtc tggctgtcca 300

ggcactgctc ttgatataaa caccacctcc tagttatgaa accatgccca ttctgcctct 360

ctgtatggaa aagagcatgg ggctggcccg tggggtggtg tccactttag gccctgtggg 420

agatcatggg aacccacgca gtgggtcata ggctctctca tttactactc acatccactc 480

tgtgaagaag cgattatgat ctctcctcta gaaactcgta gagtcccatg tctgccggct 540

tccagagcct gcactcctcc accttggctt ggctttgctg gggctagagg agctaggatg 600

cacagcagct ctgtgaccct ttgtttgaga ggaacaggaa aaccaccctt ctctctggcc 660

cactgtgtcc tcttcctgcc ctgccatccc cttctgtgaa tgttagaccc atgggagcag 720

ctggtcagag gggaccccgg cctggggccc ctaaccctat gtagcctcag tcttcccatc 780

aggctctcag ctcagcctga gtgttgaggc cccagtggct gctctggggg cctcctgagt 840

ttctcatctg tgcccctccc tccctggccc aggtgaaggt gtggttccag aaccggagga 900

caaagtacaa acggcagaag ctggaggagg aagggcctga gtccgagcag aagaagaagg 960

gctcccatca catcaaccgg tggcgcattg ccacgaagca ggccaatggg gaggacatcg 1020

atgtcacctc caatgactag ggtgggcaac cacaaaccca cgagggcaga gtgctgcttg 1080

ctgctggcca ggcccctgcg tgggcccaag ctggactctg gccactccct ggccaggctt 1140

tggggaggcc tggagtcatg gccccacagg gcttgaagcc cggggccgcc attgacagag 1200

ggacaagcaa tgggctggct gaggcctggg accacttggc cttctcctcg gagagcctgc 1260

ctgcctgggc gggcccgccc gccaccgcag cctcccagct gctctccgtg tctccaatct 1320

cccttttgtt ttgatgcatt tctgttttaa tttattttcc aggcaccact gtagtttagt 1380

gatccccagt gtcccccttc cctatgggaa taataaaagt ctctctctta atgacacggg 1440

catccagctc cagccccaga 1460

<210> 30
<211> 5311
<212> DNA
<213> Artificial Sequence

<220>
<223> All-in-one vector(HsCCR5)

<400> 30
cttccgcttc ctcgctcact gattcgctgc gctcggtcgt tcggctgcgg cgagcggtat 60

cagctcactc aaaggcggta atacggttat ccacagaatc aggggataac gcaggaaaga 120

acatgtgagc aaaaggccag caaaaggcca ggaaccgtaa aaaggccgcg ttgctggcgt 180

ttttccatag gctccgcccc cctgacgagc atcacaaaaa tcgacgctca agtcagaggt 240

ggcgaaaccc gacaggacta taaagatacc aggcgtttcc ccctggaagc tccctcgtgc 300

gctctcctgt tccgaccctg ccgcttaccg gatacctgtc cgcctttctc ccttcgggaa 360

gcgtggcgct ttctcatagc tcacgctgta ggtatctcag ttcggtgtag gtcgttcgct 420

ccaagctggg ctgtgtgcac gaaccccccg ttcagcccga ccgctgcgcc ttatccggta 480

actatcgtct tgaatccaac ccggtaagac acgacttatc gccactggca gcagccactg 540

gtaacaggat tagcagagcg aggtatgtag gcggtgctac agagttcttg aagtggtggc 600

ctaactacgg ctacactaga agaacagtat ttggtatctg cgctctgctg aagccagtta 660

ccttcggaaa aagagttggt agctcttgat ccggcaaaca aaccaccgct ggtagcggtg 720

gtttttttgt ttgcaagcag cagattacgc gcagaaaaaa aggatctcaa gaagatcctt 780

tgatcttttc tacggggtct gacgctcagt ggaacgaaaa ctcacgttaa gggattttgg 840

tcatgagatt atcaaaaagg atcttcacct agatcctttt aaattaaaaa tgaagtttta 900

aatcaatcta aagtatatat gagtaaactt ggtctgacag ttaccaatgc ttaatcagtg 960

aggcacctat ctcagcgatc tgtctatttc gttcatccat agttgcctgg ctccccgtcg 1020

tgtagataac tacgatacgg gagggcttac catctggccc cagtgctgca atgataccgc 1080

gagacccacg ctcaccggct ccagatttat cagcaataaa ccagccagcc ggaagggccg 1140

agcgcagaag tggtcctgca actttatccg cctccatcca gtctattaat tgttgccggg 1200

aagctagagt aagtagttcg ccagttaata gtttgcgcaa cgttgttgcc attgctacag 1260

gcatcgtggt gtcacgctcg tcgtttggta tggcttcatt cagctccggt tcccaacgat 1320

caaggcgagt tacatgatcc cccatgttgt gcaaaaaagc ggttagctcc ttcggtcctc 1380

cgatcgttgt cagaagtaag ttggccgcag tgttatcact catggttatg gcagcactgc 1440

ataattctct tactgtcatg ccatccgtaa gatgcttttc tgtgactggt gagtactcaa 1500

ccaagtcatt ctgagaatag tgtatgcggc gaccgagttg ctcttgcccg gcgtcaatac 1560

gggataatac cgcgccacat agcagaactt taaaagtgct catcattgga aaacgttctt 1620

cggggcgaaa actctcaagg atcttaccgc tgttgagatc cagttcgatg taacccactc 1680

gtgcacccaa ctgatcttca gcatctttta ctttcaccag cgtttctggg tgagcaaaaa 1740

caggaaggca aaatgccgca aaaaagggaa taagggcgac acggaaatgt tgaatactca 1800

tactcttcct ttttcaattc agaagaactc gtcaagaagg cgatagaagg cgatgcgctg 1860

cgaatcggga gcggcgatac cgtaaagcac gaggaagcgg tcagcccatt cgccgccaag 1920

ctcttcagca atatcacggg tagccaacgc tatgtcctga tagcggtccg ccacacccag 1980

ccggccacag tcgatgaatc cagaaaagcg gccattttcc accatgatat tcggcaagca 2040

ggcatcgcca tgggtcacga cgagatcctc gccgtcgggc atgctcgcct tgagcctggc 2100

gaacagttcg gctggcgcga gcccctgatg ctcttcgtcc agatcatcct gatcgacaag 2160

accggcttcc atccgagtac gtgctcgctc gatgcgatgt ttcgcttggt ggtcgaatgg 2220

gcaggtagcc ggatcaagcg tatgcagccg ccgcattgca tcagccatga tggatacttt 2280

ctcggcagga gcaaggtgag atgacaggag atcctgcccc ggcacttcgc ccaatagcag 2340

ccagtccctt cccgcttcag tgacaacgtc gagcacagct gcgcaaggaa cgcccgtcgt 2400

ggccagccac gatagccgcg ctgcctcgtc ttgcagttca ttcagggcac cggacaggtc 2460

ggtcttgaca aaaagaaccg ggcgcccctg cgctgacagc cggaacacgg cggcatcaga 2520

gcagccgatt gtctgttgtg cccagtcata gccgaatagc ctctccaccc aagcggccgg 2580

agaacctgcg tgcaatccat cttgttcaat catgcgaaac gatcctcatc ctgtctcttg 2640

atcagagctt gatcccctgc gccatcagat ccttggcggc aagaaagcca tccagtttac 2700

tttgcagggc ttcccaacct taccagaggg cgccccagct ggcaattccg gttcgcttgc 2760

tgtccataaa accgcccagt ctagctatcg ccatgtaagc ccactgcaag ctacctgctt 2820

tctctttgcg cttgcgtttt cccttgtcca gatagcccag tagctgacat tcatccgggg 2880

tcagcaccgt ttctgcggac tggctttcta cgtgaaaagg atctaggtga agatcctttt 2940

tgataatctc atgcctgaca tttatattcc ccagaacatc aggttaatgg cgtttttgat 3000

gtcattttcg cggtggctga gatcagccac ttcttccccg ataacggaga ccggcacact 3060

ggccatatcg gtggtcatca tgcgccagct ttcatccccg atatgcacca ccgggtaaag 3120

ttcacgggag actttatctg acagcagacg tgcactggcc agggggatca ccatccgtcg 3180

ccccggcgtg tcaataatat cactctgtac atccacaaac agacgataac ggctctctct 3240

tttataggtg taaaccttaa actgccgtac gtataggctg cgcaactgtt gggaagggcg 3300

atcggtgcgg gcctcttcgc tattacgcca gctggcgaaa gggggatgtg ctgcaaggcg 3360

attaagttgg gtaacgccag ggttttccca gtcacgacgt tgtaaaacga cggccagtga 3420

attgtaatac gactcactat agggcgaatt gggccctcta gatgcatgct cgagcggccg 3480

ccagtgtgat ggatatctgc agaattcgcc cttggtggtg gctgtgtttg cgtctctccc 3540

aggaatcatc tttaccagat ctcaaaaaga aggtcttcat tacacctgca gctctcattt 3600

tccatacagt cagtatcaat tctggaagaa tttccagaca ttaaagatag tcatcttggg 3660

gctggtcctg ccgctgcttg tcatggtcat ctgctactcg ggaatcctaa aaactctgct 3720

tcggtgtcga aatgagaaga agaggcacag ggctgtgagg cttatcttca ccatcatgat 3780

tgtttatttt ctcttctggg ctccctacaa cattgtcctt ctcctgaaca ccttccagga 3840

attctttggc ctgaataatt gcagtagctc taacaggttg gaccaagcta tgcaggtgac 3900

agagactctt gggatgacgc actgctgcat caaccccatc atctatgcct ttgtcgggga 3960

gaagttcaga aactacctct tagtcttctt ccaaaagcac attgccaaac gcttctgcaa 4020

atgctgttct attttccagc aagaggctcc cgagcgagca agctcagttt acacccgatc 4080

cactggggag caggaaatat ctgtgggctt gtgacacgga ctcaagtggg ctggtgaccc 4140

agtcagagtt gtgcacatgg cttagttttc atacacagcc tgggctgggg gtggggtggg 4200

agaggtcttt tttaaaagga agttactgtt atagagggtc taagattcat ccatttattt 4260

ggcatctgtt taaagtagat tagatctttt aagcccatca attatagaaa gccaaatcaa 4320

aatatgttga tgaaaaatag caaccttttt atctcccctt cacatgcatc aagttattga 4380

caaactctcc cttcactccg aaagttcctt atgtatattt aaaagaaagc ctcagagaat 4440

tgctgattct tgagtttagt gatctgaaca gaaataccaa aattatttca gaaatgtaca 4500

actttttacc tagtacaagg caacatatag gttgtaaatg tgtttaaaac aggtctttgt 4560

cttgctatgg ggagaaaaga catgaatatg attagtaaag aaatgacact tttcatgtgt 4620

gatttcccct ccaaggtatg gttaataagt ttcactgact tagaaccagg cgagagactt 4680

gtggcctggg agagctgggg aagcttctta aatgagaagg aatttgagtt ggatcatcta 4740

ttgctggcaa agacagaagc ctcactgcaa gcactgcatg ggcaagcttg gctgtagaag 4800

gagacagagc tggttgggaa gacatgggga ggaaggacaa ggctagatca tgaagaacct 4860

tgacggcatt gctccgtcta agtcatgagc tgagcaggga gatcctggtt ggtgttgcag 4920

aaggtttact ctgtggccaa aggagggtca ggaaggatga gcatttaggg caaggagacc 4980

accaacagcc ctcaggtcag ggtgaggatg gcctctgaag ggcgaattcc agcacactgg 5040

cggccgttac tagtggatcc gagctcggta ccaagcttgg cgtaatcatg gtcatagctg 5100

tttcctgtgt gaaattgtta tccgctcaca attccacaca acatacgagc cggaagcata 5160

aagtgtaaag cctggggtgc ctaatgagtg agctaactca cattaattgc gttgcgctca 5220

ctgcccgctt tccagtcggg aaacctgtcg tgccagctgc attaatgaat cggccaacgc 5280

gcggggagag gcggtttgcg tattgggcgc t 5311

<210> 31
<211> 4926
<212> DNA
<213> Artificial Sequence

<220>
<223> All-in-one vector(HsDNMT1)

<400> 31
cttccgcttc ctcgctcact gattcgctgc gctcggtcgt tcggctgcgg cgagcggtat 60

cagctcactc aaaggcggta atacggttat ccacagaatc aggggataac gcaggaaaga 120

acatgtgagc aaaaggccag caaaaggcca ggaaccgtaa aaaggccgcg ttgctggcgt 180

ttttccatag gctccgcccc cctgacgagc atcacaaaaa tcgacgctca agtcagaggt 240

ggcgaaaccc gacaggacta taaagatacc aggcgtttcc ccctggaagc tccctcgtgc 300

gctctcctgt tccgaccctg ccgcttaccg gatacctgtc cgcctttctc ccttcgggaa 360

gcgtggcgct ttctcatagc tcacgctgta ggtatctcag ttcggtgtag gtcgttcgct 420

ccaagctggg ctgtgtgcac gaaccccccg ttcagcccga ccgctgcgcc ttatccggta 480

actatcgtct tgaatccaac ccggtaagac acgacttatc gccactggca gcagccactg 540

gtaacaggat tagcagagcg aggtatgtag gcggtgctac agagttcttg aagtggtggc 600

ctaactacgg ctacactaga agaacagtat ttggtatctg cgctctgctg aagccagtta 660

ccttcggaaa aagagttggt agctcttgat ccggcaaaca aaccaccgct ggtagcggtg 720

gtttttttgt ttgcaagcag cagattacgc gcagaaaaaa aggatctcaa gaagatcctt 780

tgatcttttc tacggggtct gacgctcagt ggaacgaaaa ctcacgttaa gggattttgg 840

tcatgagatt atcaaaaagg atcttcacct agatcctttt aaattaaaaa tgaagtttta 900

aatcaatcta aagtatatat gagtaaactt ggtctgacag ttaccaatgc ttaatcagtg 960

aggcacctat ctcagcgatc tgtctatttc gttcatccat agttgcctgg ctccccgtcg 1020

tgtagataac tacgatacgg gagggcttac catctggccc cagtgctgca atgataccgc 1080

gagacccacg ctcaccggct ccagatttat cagcaataaa ccagccagcc ggaagggccg 1140

agcgcagaag tggtcctgca actttatccg cctccatcca gtctattaat tgttgccggg 1200

aagctagagt aagtagttcg ccagttaata gtttgcgcaa cgttgttgcc attgctacag 1260

gcatcgtggt gtcacgctcg tcgtttggta tggcttcatt cagctccggt tcccaacgat 1320

caaggcgagt tacatgatcc cccatgttgt gcaaaaaagc ggttagctcc ttcggtcctc 1380

cgatcgttgt cagaagtaag ttggccgcag tgttatcact catggttatg gcagcactgc 1440

ataattctct tactgtcatg ccatccgtaa gatgcttttc tgtgactggt gagtactcaa 1500

ccaagtcatt ctgagaatag tgtatgcggc gaccgagttg ctcttgcccg gcgtcaatac 1560

gggataatac cgcgccacat agcagaactt taaaagtgct catcattgga aaacgttctt 1620

cggggcgaaa actctcaagg atcttaccgc tgttgagatc cagttcgatg taacccactc 1680

gtgcacccaa ctgatcttca gcatctttta ctttcaccag cgtttctggg tgagcaaaaa 1740

caggaaggca aaatgccgca aaaaagggaa taagggcgac acggaaatgt tgaatactca 1800

tactcttcct ttttcaattc agaagaactc gtcaagaagg cgatagaagg cgatgcgctg 1860

cgaatcggga gcggcgatac cgtaaagcac gaggaagcgg tcagcccatt cgccgccaag 1920

ctcttcagca atatcacggg tagccaacgc tatgtcctga tagcggtccg ccacacccag 1980

ccggccacag tcgatgaatc cagaaaagcg gccattttcc accatgatat tcggcaagca 2040

ggcatcgcca tgggtcacga cgagatcctc gccgtcgggc atgctcgcct tgagcctggc 2100

gaacagttcg gctggcgcga gcccctgatg ctcttcgtcc agatcatcct gatcgacaag 2160

accggcttcc atccgagtac gtgctcgctc gatgcgatgt ttcgcttggt ggtcgaatgg 2220

gcaggtagcc ggatcaagcg tatgcagccg ccgcattgca tcagccatga tggatacttt 2280

ctcggcagga gcaaggtgag atgacaggag atcctgcccc ggcacttcgc ccaatagcag 2340

ccagtccctt cccgcttcag tgacaacgtc gagcacagct gcgcaaggaa cgcccgtcgt 2400

ggccagccac gatagccgcg ctgcctcgtc ttgcagttca ttcagggcac cggacaggtc 2460

ggtcttgaca aaaagaaccg ggcgcccctg cgctgacagc cggaacacgg cggcatcaga 2520

gcagccgatt gtctgttgtg cccagtcata gccgaatagc ctctccaccc aagcggccgg 2580

agaacctgcg tgcaatccat cttgttcaat catgcgaaac gatcctcatc ctgtctcttg 2640

atcagagctt gatcccctgc gccatcagat ccttggcggc aagaaagcca tccagtttac 2700

tttgcagggc ttcccaacct taccagaggg cgccccagct ggcaattccg gttcgcttgc 2760

tgtccataaa accgcccagt ctagctatcg ccatgtaagc ccactgcaag ctacctgctt 2820

tctctttgcg cttgcgtttt cccttgtcca gatagcccag tagctgacat tcatccgggg 2880

tcagcaccgt ttctgcggac tggctttcta cgtgaaaagg atctaggtga agatcctttt 2940

tgataatctc atgcctgaca tttatattcc ccagaacatc aggttaatgg cgtttttgat 3000

gtcattttcg cggtggctga gatcagccac ttcttccccg ataacggaga ccggcacact 3060

ggccatatcg gtggtcatca tgcgccagct ttcatccccg atatgcacca ccgggtaaag 3120

ttcacgggag actttatctg acagcagacg tgcactggcc agggggatca ccatccgtcg 3180

ccccggcgtg tcaataatat cactctgtac atccacaaac agacgataac ggctctctct 3240

tttataggtg taaaccttaa actgccgtac gtataggctg cgcaactgtt gggaagggcg 3300

atcggtgcgg gcctcttcgc tattacgcca gctggcgaaa gggggatgtg ctgcaaggcg 3360

attaagttgg gtaacgccag ggttttccca gtcacgacgt tgtaaaacga cggccagtga 3420

attgtaatac gattcactat agggcgaatt gggccctcta gatgcatgct cgagcggccg 3480

ccagtgtgat ggatatctgc agaattcgcc cttgctgctc tcgaactcct ggcctcaact 3540

aatccacctg ccttggcctc ccaaagtgct gggattacag gcgtgagcca ctgctcccag 3600

ccccacgtgt ctttgtctca agtctttctg aagctcttca aaggcccagt gacttgtggc 3660

tgtggggcgg gatgatgggc cagttggagg gtccaaggat cttgtgctgg aagggttttg 3720

ggcccatgtg agcaggacca gaacccttcc ccaaggggtg caatgcccag gttgtcctcc 3780

atctgagcag gggctggcag tacacctgcc cccgggcctt gggcctgggt gtccacatca 3840

ggcattgccc ttctcccctc ctgcaggtgg gcaatgccgt gccaccgccc ctggccaaag 3900

ccattggctt ggagatcaag ctttgtatgt tggccaaagc ccgagagagt gcctcaggta 3960

tggtggggtg ggccaggctt cctctggggc ctgactgccc tctgggggta catgtggggg 4020

cagttgctgg ccaccgtttt gggctctggg actcaggcgg gtcacctacc cacgttcgtg 4080

gccccatctt tctcaagggg ctgctgtgag gattgagtga gttgcacgtg tcaagtgctt 4140

agagcaggcg tgctgcacac agcaggcctt tggtcaggtt ggctgctggg ctggccctgg 4200

ggccgtttcc ctcactcctg ctcggtgaat ttggctcagc aggcacctgc ctcagctgct 4260

cacttgagcc tctgggtcta gaaccctctg ggaccgtttt gaggagtgtt cagtctccgt 4320

gaacgttccc ttagcactct gccacttatt gggtcagctg ttaacatcag tacgttaatg 4380

tttcctgatg gtccatgtct gttactcgcc tgtcaagtgg cgtgacaccg ggcgtgttcc 4440

ccagagtgac ttttcctttt atttcccttc agctaaaata aaggaggagg aagctgctaa 4500

ggactagttc tgccctcccg tcacccctgt ttctggcacc aggaatcccc aacatgcact 4560

gatgttgtgt ttttaacatg tcaatctgtc cgttcacatg tgtggtacat ggtgtttgtg 4620

gccttggctg acaaggcgaa attccagcac actggcggcc gttactagtg gatccgagct 4680

cggtaccaag cttggcgtaa tcatggtcat agctgtttcc tgtgtgaaat tgttatccgc 4740

tcacaattcc acacaacata cgagccggaa gcataaagtg taaagcctgg ggtgcctaat 4800

gagtgagcta actcacatta attgcgttgc gctcactgcc cgctttccag tcgggaaacc 4860

tgtcgtgcca gctgcattaa tgaatcggcc aacgcgcggg gagaggcggt ttgcgtattg 4920

ggcgct 4926

<210> 32
<211> 5267
<212> DNA
<213> Artificial Sequence

<220>
<223> All-in-one vector(HsEMX1)

<400> 32
cttccgcttc ctcgctcact gattcgctgc gctcggtcgt tcggctgcgg cgagcggtat 60

cagctcactc aaaggcggta atacggttat ccacagaatc aggggataac gcaggaaaga 120

acatgtgagc aaaaggccag caaaaggcca ggaaccgtaa aaaggccgcg ttgctggcgt 180

ttttccatag gctccgcccc cctgacgagc atcacaaaaa tcgacgctca agtcagaggt 240

ggcgaaaccc gacaggacta taaagatacc aggcgtttcc ccctggaagc tccctcgtgc 300

gctctcctgt tccgaccctg ccgcttaccg gatacctgtc cgcctttctc ccttcgggaa 360

gcgtggcgct ttctcatagc tcacgctgta ggtatctcag ttcggtgtag gtcgttcgct 420

ccaagctggg ctgtgtgcac gaaccccccg ttcagcccga ccgctgcgcc ttatccggta 480

actatcgtct tgaatccaac ccggtaagac acgacttatc gccactggca gcagccactg 540

gtaacaggat tagcagagcg aggtatgtag gcggtgctac agagttcttg aagtggtggc 600

ctaactacgg ctacactaga agaacagtat ttggtatctg cgctctgctg aagccagtta 660

ccttcggaaa aagagttggt agctcttgat ccggcaaaca aaccaccgct ggtagcggtg 720

gtttttttgt ttgcaagcag cagattacgc gcagaaaaaa aggatctcaa gaagatcctt 780

tgatcttttc tacggggtct gacgctcagt ggaacgaaaa ctcacgttaa gggattttgg 840

tcatgagatt atcaaaaagg atcttcacct agatcctttt aaattaaaaa tgaagtttta 900

aatcaatcta aagtatatat gagtaaactt ggtctgacag ttaccaatgc ttaatcagtg 960

aggcacctat ctcagcgatc tgtctatttc gttcatccat agttgcctgg ctccccgtcg 1020

tgtagataac tacgatacgg gagggcttac catctggccc cagtgctgca atgataccgc 1080

gagacccacg ctcaccggct ccagatttat cagcaataaa ccagccagcc ggaagggccg 1140

agcgcagaag tggtcctgca actttatccg cctccatcca gtctattaat tgttgccggg 1200

aagctagagt aagtagttcg ccagttaata gtttgcgcaa cgttgttgcc attgctacag 1260

gcatcgtggt gtcacgctcg tcgtttggta tggcttcatt cagctccggt tcccaacgat 1320

caaggcgagt tacatgatcc cccatgttgt gcaaaaaagc ggttagctcc ttcggtcctc 1380

cgatcgttgt cagaagtaag ttggccgcag tgttatcact catggttatg gcagcactgc 1440

ataattctct tactgtcatg ccatccgtaa gatgcttttc tgtgactggt gagtactcaa 1500

ccaagtcatt ctgagaatag tgtatgcggc gaccgagttg ctcttgcccg gcgtcaatac 1560

gggataatac cgcgccacat agcagaactt taaaagtgct catcattgga aaacgttctt 1620

cggggcgaaa actctcaagg atcttaccgc tgttgagatc cagttcgatg taacccactc 1680

gtgcacccaa ctgatcttca gcatctttta ctttcaccag cgtttctggg tgagcaaaaa 1740

caggaaggca aaatgccgca aaaaagggaa taagggcgac acggaaatgt tgaatactca 1800

tactcttcct ttttcaattc agaagaactc gtcaagaagg cgatagaagg cgatgcgctg 1860

cgaatcggga gcggcgatac cgtaaagcac gaggaagcgg tcagcccatt cgccgccaag 1920

ctcttcagca atatcacggg tagccaacgc tatgtcctga tagcggtccg ccacacccag 1980

ccggccacag tcgatgaatc cagaaaagcg gccattttcc accatgatat tcggcaagca 2040

ggcatcgcca tgggtcacga cgagatcctc gccgtcgggc atgctcgcct tgagcctggc 2100

gaacagttcg gctggcgcga gcccctgatg ctcttcgtcc agatcatcct gatcgacaag 2160

accggcttcc atccgagtac gtgctcgctc gatgcgatgt ttcgcttggt ggtcgaatgg 2220

gcaggtagcc ggatcaagcg tatgcagccg ccgcattgca tcagccatga tggatacttt 2280

ctcggcagga gcaaggtgag atgacaggag atcctgcccc ggcacttcgc ccaatagcag 2340

ccagtccctt cccgcttcag tgacaacgtc gagcacagct gcgcaaggaa cgcccgtcgt 2400

ggccagccac gatagccgcg ctgcctcgtc ttgcagttca ttcagggcac cggacaggtc 2460

ggtcttgaca aaaagaaccg ggcgcccctg cgctgacagc cggaacacgg cggcatcaga 2520

gcagccgatt gtctgttgtg cccagtcata gccgaatagc ctctccaccc aagcggccgg 2580

agaacctgcg tgcaatccat cttgttcaat catgcgaaac gatcctcatc ctgtctcttg 2640

atcagagctt gatcccctgc gccatcagat ccttggcggc aagaaagcca tccagtttac 2700

tttgcagggc ttcccaacct taccagaggg cgccccagct ggcaattccg gttcgcttgc 2760

tgtccataaa accgcccagt ctagctatcg ccatgtaagc ccactgcaag ctacctgctt 2820

tctctttgcg cttgcgtttt cccttgtcca gatagcccag tagctgacat tcatccgggg 2880

tcagcaccgt ttctgcggac tggctttcta cgtgaaaagg atctaggtga agatcctttt 2940

tgataatctc atgcctgaca tttatattcc ccagaacatc aggttaatgg cgtttttgat 3000

gtcattttcg cggtggctga gatcagccac ttcttccccg ataacggaga ccggcacact 3060

ggccatatcg gtggtcatca tgcgccagct ttcatccccg atatgcacca ccgggtaaag 3120

ttcacgggag actttatctg acagcagacg tgcactggcc agggggatca ccatccgtcg 3180

ccccggcgtg tcaataatat cactctgtac atccacaaac agacgataac ggctctctct 3240

tttataggtg taaaccttaa actgccgtac gtataggctg cgcaactgtt gggaagggcg 3300

atcggtgcgg gcctcttcgc tattacgcca gctggcgaaa gggggatgtg ctgcaaggcg 3360

attaagttgg gtaacgccag ggttttccca gtcacgacgt tgtaaaacga cggccagtga 3420

attgtaatac gattcactat agggcgaatt gggccctcta gatgcatgct cgagcggccg 3480

ccagtgtgat ggatatctgc agaattcgcc cttgtgggga cagaaggtct ggagctgccc 3540

gtgaagggca gaatgctgcc ctcagacccg cttcctccct gtccttgtct gtccaaggag 3600

aatgaggtct cactggtgga tttcggacta ccctgaggag ctggcacctg agggacaagg 3660

ccccccacct gcccagctcc agcctctgat gaggggtggg agagagctac atgaggttgc 3720

taagaaagcc tcccctgaag gagaccacac agtgtgtgag gttggagtct ctagcagcgg 3780

gttctgtgcc cccagggata gtctggctgt ccaggcactg ctcttgatat aaacaccacc 3840

tcctagttat gaaaccatgc ccattctgcc tctctgtatg gaaaagagca tggggctggc 3900

ccgtggggtg gtgtccactt taggccctgt gggagatcat gggaacccac gcagtgggtc 3960

ataggctctc tcatttacta ctcacatcca ctctgtgaag aagcgattat gatctctcct 4020

ctagaaactc gtagagtccc atgtctgccg gcttccagag cctgcactcc tccaccttgg 4080

cttggctttg ctggggctag aggagctagg atgcacagca gctctgtgac cctttgtttg 4140

agaggaacag gaaaaccacc cttctctctg gcccactgtg tcctcttcct gccctgccat 4200

ccccttctgt gaatgttaga cccatgggag cagctggtca gaggggaccc cggcctgggg 4260

cccctaaccc tatgtagcct cagtcttccc atcaggctct cagctcagcc tgagtgttga 4320

ggccccagtg gctgctctgg gggcctcctg agtttctcat ctgtgcccct ccctccctgg 4380

cccaggtgaa ggtgtggttc cagaaccgga ggacaaagta caaacggcag aagctggagg 4440

aggaagggcc tgagtccgag cagaagaaga agggctccca tcacatcaac cggtggcgca 4500

ttgccacgaa gcaggccaat ggggaggaca tcgatgtcac ctccaatgac tagggtgggc 4560

aaccacaaac ccacgagggc agagtgctgc ttgctgctgg ccaggcccct gcgtgggccc 4620

aagctggact ctggccactc cctggccagg ctttggggag gcctggagtc atggccccac 4680

agggcttgaa gcccggggcc gccattgaca gagggacaag caatgggctg gctgaggcct 4740

gggaccactt ggccttctcc tcggagagcc tgcctgcctg ggcgggcccg cccgccaccg 4800

cagcctccca gctgctctcc gtgtctccaa tctccctttt gttttgatgc atttctgttt 4860

taatttattt tccaggcacc actgtagttt agtgatcccc agtgtccccc ttccctatgg 4920

gaataataaa agtctctctc ttaatgacac gggcatccag ctccagcccc agaaaggcgg 4980

aattccagca cactggcggc cgttactagt ggatccgagc tcggtaccaa gcttggcgta 5040

atcatggtca tagctgtttc ctgtgtgaaa ttgttatccg ctcacaattc cacacaacat 5100

acgagccgga agcataaagt gtaaagcctg gggtgcctaa tgagtgagct aactcacatt 5160

aattgcgttg cgctcactgc ccgctttcca gtcgggaaac ctgtcgtgcc agctgcatta 5220

atgaatcggc caacgcgcgg ggagaggcgg tttgcgtatt gggcgct 5267